# EUROPEAN PATENT APPLICATION

(11) **EP 3 651 225 A1**
(43) Date of publication of application: **13.05.2020**
(21) Application number: 18205537.6
(22) Date of filing: 09.11.2018
(51) Int. Cl.: H01L 51/54, C07D 487/04, C09B 57/00

(54) **NOVEL ORGANIC COMPOUNDS AND AN ORGANIC ELECTROLUMINESCENCE DEVICE COMPRISING THE SAME**

(71) Applicant: Idemitsu Kosan Co., Ltd., Chiyoda-ku, Tokyo 100-8321 (JP)
(72) Inventor: Schäfer, Thomas, 4410 Liestal (CH); Murer, Peter, 4104 Oberwil (CH); Ogiwara, Toshinari, Sodegaura-shi, 299-0293 (JP); Chebotareva, Natalia, 68220 Hagenthal le Bas (FR)
(74) Representative: Hollah, Dorothee

(57) **Abstract**

An arylnitril carrying at least one benzimidazolo[1,2-a]benzimidazole group and at least one further donor group, a process for preparing said arylnitril carrying at least one benzimidazolo[1,2-a]benzimidazole group and at least one further donor group, an organic electroluminescence device comprising said arylnitril carrying at least one benzimidazolo[1,2-a]benzimidazole group and at least one further donor group, an electronic device comprising said organic electroluminescence device, an emitting layer comprising at least one inventive arylnitril carrying at least one benzimidazolo[1,2-a]benzimidazole group and at least one further donor group and the use of said inventive arylnitril carrying at least one benzimidazolo[1,2-a]benzimidazole group and at least one further donor group in an electronic device as a delayed-fluorescent emitter and/or as a host material.

## Description

The present invention relates to an arylnitril carrying at least one benzimidazolo[1,2-a]benzimidazole group and at least one further donor group, a process for preparing said arylnitril carrying at least one benzimidazolo[1,2-a]benzimidazole group and at least one further donor group, an organic electroluminescence device comprising said arylnitril carrying at least one benzimidazolo[1,2-a]benzimidazole group and at least one further donor group, an electronic device comprising said organic electroluminescence device, an emitting layer comprising at least one inventive arylnitril carrying at least one benzimidazolo[1,2-a]benzimidazole group and at least one further donor group and the use of said inventive arylnitril carrying at least one benzimidazolo[1,2-a]benzimidazole group and at least one further donor group in an electronic device as a delayed-fluorescent emitter and/or as a host material.

When a voltage is applied to an organic electroluminescence device (hereinafter, occasionally referred to as "organic EL device"), holes and electrons are injected into an emitting layer respectively from an anode and a cathode. The injected holes and electrons are recombined to generate excitons in the emitting layer. According to the electron spin statistics theory, singlet excitons are generated at a ratio of 25% and triplet excitons are generated at a ratio of 75%.

A fluorescent organic EL device, which uses emission caused by singlet excitons, is applied to a full color display of a mobile phone, a TV set and the like. It has been studied to further improve a performance of the fluorescent organic EL device. For instance, in order to further improve a luminous efficiency, an organic EL device with use of singlet excitons and triplet excitons has been studied.

An organic EL device using delayed fluorescence has been proposed and studied. For instance, a thermally activated delayed fluorescence (TADF) mechanism has been studied. The TADF mechanism uses such a phenomenon that inverse intersystem crossing from triplet excitons to singlet excitons thermally occurs when a material having a small energy gap (ΔST) between singlet energy level and triplet energy level is used. As for thermally activated delayed fluorescence, refer to, for instance, ADACHI, Chihaya, ed. (March 22, 2012), Yuki Hando-tai no Debaisu Bussei (Device Physics of Organic Semiconductors), Kodansha, pp. 261-262.

For instance, the following documents also disclose organic EL devices using the TADF mechanism.

Adachi et al., Nature, 2012, 492, 234-8, concerns TADF emitters based on carbazolyl dicyanobenzene (CDCB), with carbazole as a donor and dicyanobenzene as an electron acceptor.

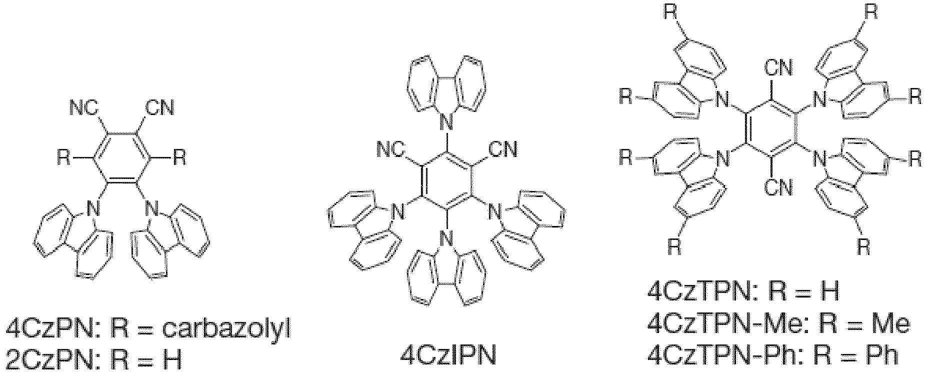

Duan et al. Mater. Horiz., 2016, 3, 145--151 concerns sterically shielded blue TADF emitter.

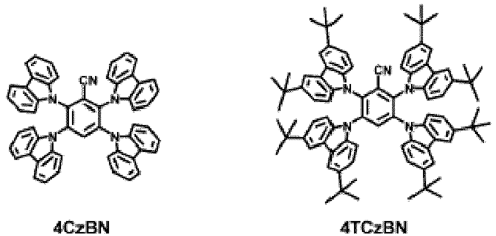

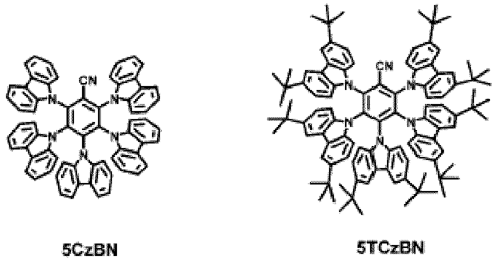

WO 2016/157113 A1 concerns benzimidazolo[1,2-a]benzimidazole carrying aryl- or heteroarylnitril groups for organic light emitting diodes. Said compounds show low singlet triplet splitting, which makes them useful as TADF (Thermally Activated Delayed Fluorescence, Adv. Mater. 2014, 26, 7931-7958) emitter or TADF host materials in combination with fluorescent emitters.

EP 2 851 408 A1 concerns a light-emitting material which emits delayed fluorescent light containing a compound represented by the following general formula (1): 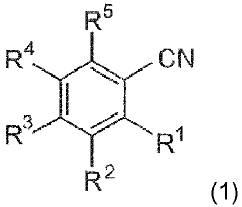 wherein in the general formula (1), at least one of R¹ to R⁵ represents a cyano group, at least one of R¹ to R⁵ represents a group represented by the following general formula (11), and the balance of R¹ to R⁵ represents a hydrogen atom or a substituent; 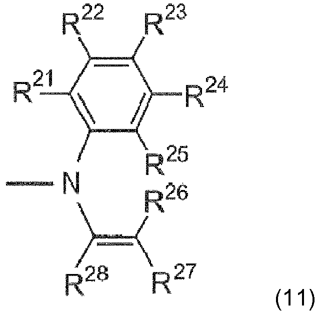 wherein in the general formula (11), R²¹ to R²⁸ each independently represents a hydrogen atom or a substituent, provided that at least one of the following requirements (A) and (B) is satisfied:
(A) R²⁵ and R²⁶ jointly form a single bond, and
(B) R²⁷ and R²⁸ jointly form an atomic group that is required for forming a substituted or unsubstituted benzene ring.

Examples are: 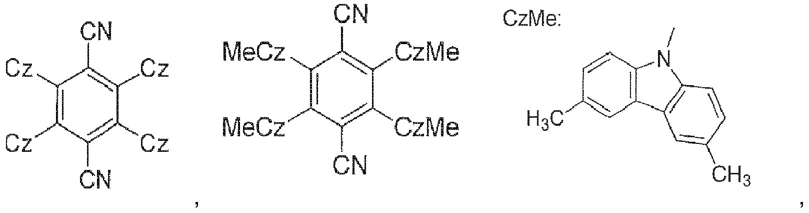 and

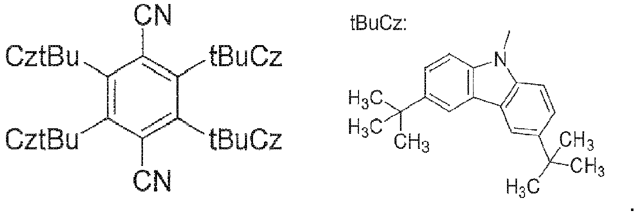

WO 2017/115596 A1 concerns a heat-activated delayed-fluorescence organic electroluminescence element that includes a luminescent layer between a positive electrode and a negative electrode that face. The organic electroluminescence element contains, in at least one luminescent layer, a heat-activated delayed-fluorescence material or a heat-activated delayed-fluorescence material and a host material. The heat-activated delayed-fluorescence material is represented by general formula (1), wherein A is an electron-withdrawing group such as a CN group and D¹ and D² are electron-donating groups that have an indole-ring structure:

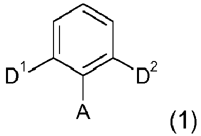

An object of the invention is to provide a compound useful in an organic electroluminescence device capable of prolonging the lifetime, improving the luminous efficiency, and inhibiting the roll-off when driven at a high current density, and an electronic device including the organic electroluminescence device. Especially, a compound should be provided useful in an organic electroluminescence device capable of improving luminous efficiency.

Said object is achieved by a compound of formula (I),
A compound of formula (I), 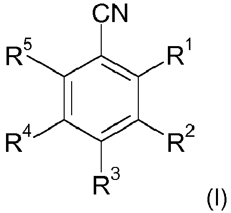 wherein
R¹, R², R³, R⁴ and R⁵ are R^{A}, D¹ or D²;
wherein
0, 1 or 2 of the residues R¹, R², R³, R⁴ and R⁵ are R^{A};
1, 2, 3 or 4 of the residues R¹, R², R³, R⁴ and R⁵ are D²; and
1, 2, 3 or 4 of the residues R¹, R², R³, R⁴ and R⁵ are D¹;
wherein the sum of the residues R^{A}, D² and D¹ is 5, and the sum of D² and D¹ is 3, 4 or 5, more preferably 3 or 4, most preferably 4;
D² is a donor group except for a group of formula (II);
D¹ is a group of formula (II) 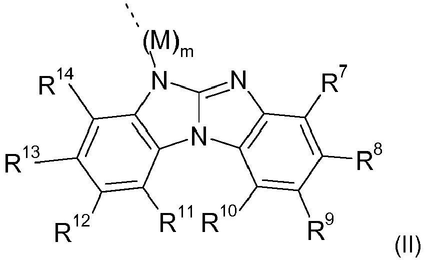 wherein
R⁷ to R¹⁴ are independently of each other H, a C₁-C₂₅alkyl group which is unsubstituted or substituted by at least one group E and/or interrupted by at least one group D, preferably a C₁-C₂₅alkyl group which is unsubstituted or substituted by at least one group E, a C₆-C₄₀aryl group which is unsubstituted or substituted by at least one group E, a C₁-C₂₄heteroaryl group which is unsubstituted or substituted by at least one group E, a C₇-C₂₅aralkyl group which is unsubstituted or substituted by at least one group E, a C₅-C₁₂cycloalkyl group which is unsubstituted or substituted by at least one group E, -OR²¹, -SR²¹, -NR¹⁷R¹⁸, -COR²⁰, -COOR¹⁹, -CONR¹⁷R¹⁸, -SiR²²R²³R²⁴, -POR²⁵R²⁷, or halogen, or
at least two of the remaining residues R⁷ to R¹⁴ if present at adjacent carbon atoms together form at least one C₆-C₁₈aryl ring or ring system which is unsubstituted or substituted by at least one group E or at least one C₁-C₂₄heteroaryl ring or ring system which is unsubstituted or substituted by at least one group E,
preferably R⁷ to R¹⁴ in the group D¹ are independently of each other H, a C₁-C₁₈alkyl group which is unsubstituted, preferably methyl or t-butyl, a C₆-C₁₈aryl group which is unsubstituted or substituted by at least one group E, preferably phenyl, or -OR²¹, preferably methoxy,
more preferably, R⁹ and R¹² are independently of each other H, a C₁-C₁₈alkyl group which is unsubstituted, preferably methyl or t-butyl, a C₆-C₁₈aryl group which is unsubstituted or substituted by at least one group E, preferably phenyl, or -OR²¹, preferably methoxy, and R⁷, R⁸, R¹⁰, R¹¹, R¹³ and R¹⁴ are H,
in a further most preferred embodiment, each pair of R⁷ and R¹⁴, R⁸ and R¹³, R⁹ and R¹² and R¹⁰ and R¹¹ independently of each other pair has the same meaning selected from the following residues: H, a C₁-C₁₈alkyl group which is unsubstituted, preferably methyl or t-butyl, a C₆-C₁₈aryl group which is unsubstituted or substituted by at least one group E, preferably phenyl, and -OR²¹, preferably methoxy,
most preferably, one pair of R⁷ and R¹⁴, R⁸ and R¹³, R⁹ and R¹² and R¹⁰ and R¹¹ has the same meaning selected from the following residues: H, a C₁-C₁₈alkyl group which is unsubstituted, preferably methyl or t-butyl, a C₆-C₁₈aryl group which is unsubstituted or substituted by at least one group E, preferably phenyl, and -OR²¹, preferably methoxy, and the remaining residues are hydrogen,
most preferably, R⁷ to R¹⁴ are H,
R^{A} is H, a C₁-C₂₅alkyl group which is unsubstituted or substituted by at least one group E and/or interrupted by at least one group D, preferably a C₁-C₂₅alkyl group which is unsubstituted or substituted by at least one group E, a C₆-C₄₀aryl group which is unsubstituted or substituted by at least one group E, a C₁-C₂₄heteroaryl group which is unsubstituted or substituted by at least one group E, a C₇-C₂₅aralkyl group which is unsubstituted or substituted by at least one group E, a C₅-C₁₂cycloalkyl group which is unsubstituted or substituted by at least one group E, -OR²¹, -SR²¹, -NR¹⁷R¹⁸, -COR²⁰, -COOR¹⁹, -CONR¹⁷R¹⁸, -SiR²²R²³R²⁴, -POR²⁵R²⁷, or halogen, or
at least two of the residues R^{A} if present at adjacent carbon atoms together form at least one C₆-C₁₈aryl ring or ring system which is unsubstituted or substituted by at least one group E or at least one C₁-C₂₄heteroaryl ring or ring system which is unsubstituted or substituted by at least one group E,
preferably, R^{A} is H,
wherein the dotted line is a bonding site to formula (I),
M is a C₆-C₄₀arylene group which is unsubstituted or substituted by at least one group E, a C₁-C₂₄heteroarylene group which is unsubstituted or substituted by at least one group E, a C₁-C₂₅alkylene group which is unsubstituted or substituted by at least one group E and/or interrupted by at least one group D, preferably a C₁-C₂₅alkylene group which is unsubstituted or substituted by at least one group E, or a C₂-C₂₅alkenylene group which is unsubstituted or substituted by at least one group E and/or interrupted by at least one group D, preferably a C₂-C₂₅alkenylene group which is unsubstituted or substituted by at least one group E,
preferably, M is a C₆-C₄₀arylene group which is unsubstituted or substituted by at least one group E, a C₁-C₂₄heteroarylene group which is unsubstituted or substituted by at least one group E, more preferably, M is a C₆-C₄₀arylene group which is unsubstituted, a C₁-C₂₄heteroarylene group which is unsubstituted, most preferably a C₆-C₁₀arylene group which is unsubstituted or a C₁-C₁₄heteroarylene group which is unsubstituted, further most preferably dibenzofuran-diyl, 1,2-phenylene, 1,3-phenylene or 1,4-phenylene, even further most preferably 1,2-phenylene,
m is 0, 1 or 2, preferably 0 or 1, more preferably 0,
D is -CO-, -COO-, -S-, -SO-, -SO₂-, -O-, -CR¹⁵=CR¹⁶-, -NR¹⁷-, -SiR²²R²³-, -POR²⁵-, -C≡C-,
preferably, D is -CO-, -COO-, -S-, -SO-, -SO₂-, -O-, -NR¹⁷-, more preferably, D is O,
E is -OR²¹, -SR²¹, -NR¹⁷R¹⁸, -COR²⁰, -COOR¹⁹, -CONR¹⁷R¹⁸, -CN, -SiR²²R²³R²⁴, -POR²⁵R²⁷, halogen, preferably F,
a C₆-C₄₀aryl group which is unsubstituted or is substituted by at least one group selected from a C₁-C₁₈alkyl group, a C₁-C₁₈alkoxy group, a C₆-C₁₈aryl group, which is in turn unsubstituted or substituted by at least one C₁-C₁₈alkyl group or at least one C₁-C₁₈alkoxy group, a C₅-C₁₂cycloalkyl group, which is in turn unsubstituted or substituted by at least one C₁-C₁₈alkyl group or at least one C₁-C₁₈alkoxy group, a C₁-C₁₈heteroaryl group, which is in turn unsubstituted or substituted by at least one C₁-C₁₈alkyl group or at least one C₁-C₁₈alkoxy group, -F, -CF₃, -CF₂CF₃, -CF₂CF₂CF₃, -CF(CF₃)₂, -(CF₂)₃CF₃ and -C(CF₃)₃,
a C₁-C₁₈alkyl group, or
a C₁-C₂₄heteroaryl group which is unsubstituted or is substituted by at least one group selected from a C₁-C₁₈alkyl group, a C₁-C₁₈alkoxy group, a C₆-C₁₈aryl group, which is in turn unsubstituted or substituted by at least one C₁-C₁₈alkyl group or at least one C₁-C₁₈alkoxy group, a C₅-C₁₂cycloalkyl group, which is in turn unsubstituted or substituted by at least one C₁-C₁₈alkyl group or at least one C₁-C₁₈alkoxy group, a C₁-C₁₈heteroaryl group, which is in turn unsubstituted or substituted by at least one C₁-C₁₈alkyl group or at least one C₁-C₁₈alkoxy group, -F, -CF₃, -CF₂CF₃, -CF₂CF₂CF₃, -CF(CF₃)₂, -(CF₂)₃CF₃ and -C(CF₃)₃,
preferably, E is CN, F, CF₃,
a C₆-C₁₈aryl group which is unsubstituted or is substituted by at least one group selected from a C₁-C₁₈alkyl group, a C₁-C₁₈alkoxy group, an unsubstituted C₆-C₁₈aryl group,
a C₁-C₁₈ alkyl group,
a C₁-C₂₄heteroaryl group which is unsubstituted or substituted by at least one group selected from a C₁-C₁₈alkyl group, a C₁-C₁₈alkoxy group, an unsubstituted C₆-C₁₈aryl group,
R¹⁵ and R¹⁶ are independently of each other H, a C₆-C₁₈aryl group which is unsubstituted or substituted by at least one C₁-C₁₈alkyl group or at least one C₁-C₁₈alkoxy group, a C₁-C₁₈alkyl group or a C₁-C₁₈alkoxy group,
R¹⁷ and R¹⁸ are independently of each other H , a C₆-C₁₈aryl group which is unsubstituted or substituted by at least one C₁-C₁₈alkyl group or at least one C₁-C₁₈alkoxy group, a C₁-C₁₈alkyl group or a C₁-C₁₈alkoxy group, or
R¹⁷ and R¹⁸ together form a five or six membered aliphatic, aromatic or heteroaromatic ring, which is unsubstituted or substituted by at least one group E,
R¹⁹ is H, a C₆-C₁₈aryl group which is unsubstituted or substituted by at least one C₁-C₁₈alkyl group or at least one C₁-C₁₈alkoxy group, a C₁-C₁₈alkyl group or a C₁-C₁₈alkoxy group,
R²⁰ is H or a C₆-C₁₈aryl group which is unsubstituted or substituted by at least one C₁-C₁₈alkyl group or at least one C₁-C₁₈alkoxy group, a C₁-C₁₈alkyl group or a C₁-C₁₈alkoxy group,
R²¹ is H, a C₆-C₁₈aryl group which is unsubstituted or substituted by at least one C₁-C₁₈alkyl group or at least one C₁-C₁₈alkoxy group or a C₁-C₁₈alkyl group,
R^{22,} R²³ and R²⁴ are independently of each other H, a C₁-C₁₈alkyl group or a C₆-C₁₈aryl group which is unsubstituted or substituted by at least one C₁-C₁₈alkyl group, and
R²⁵ and R²⁷ are independently of each other H, a C₁-C₁₈alkyl group, a C₆-C₁₈aryl group which is unsubstituted or substituted by at least one C₁-C₁₈alkyl group, a C₇-C₂₅aralkyl which is unsubstituted or substituted by at least one group E, a C₅-C₁₂cycloalkyl group which is unsubstituted or substituted by at least one group E.

According to the present invention, compounds suitable for use in an organic electroluminescence device capable of prolonging a lifetime, improving the luminous efficiency, and/or inhibiting a roll-off when driven at a high current density, an organic electroluminescence device, and an electronic device including the organic electroluminescence device are provided. Especially, the compounds are useful in an organic electroluminescence device capable of improving the luminous efficiency, especially at high current.

One key finding of the inventors of the present invention is the relevance of the presence of a benzimidazolo[1,2-a]benzimidazole group and a further donor group in an arylnitril compound comprising at least three donor groups. Therefore, the compounds of the present invention are characterized by a good suitability as TADF hosts or emitters in a TADF emitter system.

The compounds of the present invention may be used for electrophotographic photoreceptors, photoelectric converters, organic solar cells (organic photovoltaics), switching elements, such as organic transistors, for example, organic FETs and organic TFTs, organic light emitting field effect transistors (OLEFETs), image sensors, dye lasers and electroluminescence devices, such as, for example, organic light-emitting diodes (OLEDs).

Accordingly, a further subject of the present invention is directed to an electronic device, comprising a compound according to the present invention. The electronic device is preferably an electroluminescence device, such as an organic light-emitting diode (OLED).

The compounds of formula (I) are preferably used as TADF emitter materials, or TADF host materials in combination with at least one fluorescent emitter.

A further subject of the present invention is directed to an emitting layer, comprising a compound of formula (I) according to the present invention. In said embodiment a compound of formula (I) is preferably used as TADF emitter material, or TADF host material in combination with at least one fluorescent emitter.

The term of "XX to YY carbon atoms" referred to by "a substituted or unsubstituted group ZZ having XX to YY carbon atoms" used herein is the number of carbon atoms of the unsubstituted group ZZ and does not include any carbon atom in the substituent of the substituted group ZZ. "YY" is larger than "XX" and each of "XX" and "YY" represents an integer of 1 or more.

The term of "XX to YY atoms" referred to by "a substituted or unsubstituted group ZZ having XX to YY atoms" used herein is the number of atoms of the unsubstituted group ZZ and does not include any atom in the substituent of the substituted group ZZ. "YY" is larger than "XX" and each of "XX" and "YY" represents an integer of 1 or more.

The term "C_{ZZ}-C_{YY}-" referred to by "C_{ZZ}-C_{YY}-ZZ group which is unsubstituted or substituted" used herein is the number of carbon atoms of the unsubstituted group ZZ and does not include any heteroatom or any atom in the substituent of the substituted group ZZ. "YY" is larger than "XX" and each of "XX" and "YY" represents an integer of 1 or more.

The term of "unsubstituted group ZZ" referred to by "a substituted or unsubstituted group ZZ" used herein means the group ZZ wherein no hydrogen atom is substituted by a substituent.

The number of "ring carbon atoms" ("a ring formed of carbon atoms") referred to herein means the number of the carbon atoms included in the atoms which are members forming the ring itself of a compound in which a series of atoms is bonded to form the ring (for example, a monocyclic compound, a fused ring compound, a cross-linked compound, a carbocyclic compound, and a heterocyclic compound). If the ring has a substituent, the carbon atom in the substituent is not included in the ring carbon atom. The same applies to the number of "ring carbon atom" described below, unless otherwise noted. For example, a benzene ring has 6 ring carbon atoms, a naphthalene ring has 10 ring carbon atoms, a pyridinyl group has 5 ring carbon atoms, and a furanyl group has 4 ring carbon atoms. If a benzene ring or a naphthalene ring has, for example, an alkyl substituent, the carbon atom in the alkyl substituent is not counted as the ring carbon atom of the benzene or naphthalene ring. In case of a fluorene ring to which a fluorene substituent is bonded (inclusive of a spirofluorene ring), the carbon atom in the fluorene substituent is not counted as the ring carbon atom of the fluorene ring.

The number of "ring atom" ("a ring formed of atoms") referred to herein means the number of the atoms which are members forming the ring itself (for example, a monocyclic ring, a fused ring, and a ring assembly) of a compound in which a series of atoms is bonded to form the ring (for example, a monocyclic compound, a fused ring compound, a cross-linked compound, a carbocyclic compound, and a heterocyclic compound). The atom not forming the ring (for example, hydrogen atom(s) for saturating the valence of the atom which forms the ring) and the atom in a substituent, if the ring is substituted, are not counted as the ring atom. The same applies to the number of "ring atoms" described below, unless otherwise noted. For example, a pyridine ring has 6 ring atoms, a quinazoline ring has 10 ring atoms, and a furan ring has 5 ring atoms. The hydrogen atom on the ring carbon atom of a pyridine ring or a quinazoline ring and the atom in a substituent are not counted as the ring atom. In case of a fluorene ring to which a fluorene substituent is bonded (inclusive of a spirofluorene ring), the atom in the fluorene substituent is not counted as the ring atom of the fluorene ring.

The definition of "hydrogen atom" used herein includes isotopes different in the neutron numbers, i.e., light hydrogen (protium), heavy hydrogen (deuterium), and tritium.

The residues mentioned in the specification of the present application generally have the following preferred meanings, if said residues are not further specified in specific embodiments mentioned below:
Halogen is fluorine, chlorine, bromine and iodine.

C₁-C₂₅alkyl (C₁-C₁₈alkyl) is typically linear or branched, where possible. Examples are methyl, ethyl, n-propyl, isopropyl, n-butyl, sec.-butyl, isobutyl, tert.-butyl, n-pentyl, 2-pentyl, 3-pentyl, 2,2-dimethylpropyl, 1,1,3,3-tetramethylpentyl, n-hexyl, 1-methylhexyl, 1,1,3,3,5,5-hexamethylhexyl, n-heptyl, isoheptyl, 1,1,3,3-tetramethylbutyl, 1-methylheptyl, 3-methylheptyl, n-octyl, 1,1,3,3-tetramethylbutyl and 2-ethylhexyl, n-nonyl, decyl, undecyl, dodecyl, tridecyl, tetradecyl, pentadecyl, hexadecyl, heptadecyl, or octadecyl. C₁-C₈alkyl is typically methyl, ethyl, n-propyl, isopropyl, n-butyl, sec.-butyl, isobutyl, tert.-butyl, n-pentyl, 2-pentyl, 3-pentyl, 2,2-dimethyl-propyl, n-hexyl, n-heptyl, n-octyl, 1,1,3,3-tetramethylbutyl and 2-ethylhexyl. Preferred alkyl groups are C₁-C₄alkyl groups. C₁-C₄alkyl is typically methyl, ethyl, n-propyl, isopropyl, n-butyl, sec.-butyl, isobutyl, tert.-butyl. More preferred alkyl groups are methyl, isopropyl and tert.-butyl.

The alkyl groups may be substituted by one or more halogens and form halogenated alkyl groups (haloalkyl groups). Specific examples of the above halogenated alkyl groups are a fluoromethyl group, difluoromethyl group, trifluoromethyl group, fluoroethyl group, trifluoromethylmethyl group, trifluoroethyl group and pentafluoroethyl group.

C₁-C₂₅alkoxy groups (C₁-C₁₈alkoxy groups) are straight-chain or branched alkoxy groups, e.g. methoxy, ethoxy, n-propoxy, isopropoxy, n-butoxy, sec-butoxy, tert-butoxy, amyloxy, isoamyloxy or tert-amyloxy, heptyloxy, octyloxy, isooctyloxy, nonyloxy, decyloxy, undecyloxy, dodecyloxy, tetradecyloxy, pentadecyloxy, hexadecyloxy, heptadecyloxy and octadecyloxy. Examples of C₁-C₈alkoxy are methoxy, ethoxy, n-propoxy, isopropoxy, n-butoxy, sec.-butoxy, isobutoxy, tert.-butoxy, n-pentyloxy, 2-pentyloxy, 3-pentyloxy, 2,2-dimethylpropoxy, n-hexyloxy, n-heptyloxy, n-octyloxy, 1,1,3,3-tetramethylbutoxy and 2-ethylhexyloxy, preferably C₁-C₄alkoxy such as typically methoxy, ethoxy, n-propoxy, isopropoxy, n-butoxy, sec.-butoxy, isobutoxy, tert.-butoxy.

The term "cycloalkyl group" is typically C₅-C₁₂cycloalkyl, such as cyclopentyl, cyclohexyl, cycloheptyl, cyclooctyl, cyclononyl, cyclodecyl, cycloundecyl, cyclododecyl, preferably cyclopentyl, cyclohexyl, cycloheptyl, or cyclooctyl, which may be unsubstituted or substituted. More preferred cycloalkyl groups are cyclopentyl and cyclohexyl, which may be unsubstituted or substituted.

C₆-C₄₀aryl (preferably C₆-C₂₄aryl, more preferably C₆-C₁₈aryl), which optionally can be substituted, is typically phenyl, 4-methylphenyl, 4-methoxyphenyl, naphthyl, especially 1-naphthyl, or 2-naphthyl, biphenylyl, terphenylyl, pyrenyl, 2- or 9-fluorenyl, phenanthryl, or anthryl, which may be unsubstituted or substituted. More preferred aryl groups are C₆-C₁₀aryl groups which are unsubstituted or substituted. Phenyl, 1-naphthyl and 2-naphthyl are examples of a C₆-C₁₀aryl group. Most preferred is a phenyl group which is unsubstituted or substituted. Further most preferred is an unsubstituted phenyl group.

C₆-C₂₄aryloxy, which optionally can be substituted, is typically C₆-C₁₀aryloxy, which optionally can be substituted by one, or more C₁-C₈alkyl and/or C₁-C₈alkoxy groups, such as, for example, phenoxy, 1-naphthoxy, or 2-naphthoxy.

C₇-C₂₅aralkyl is typically benzyl, 2-benzyl-2-propyl, β-phenyl-ethyl, α,α-dimethylbenzyl, ω-phenyl-butyl, ω,ω-dimethyl-ω-phenyl-butyl, ω-phenyl-dodecyl, ω-phenyl-octadecyl, ω-phenyl-eicosyl or ω-phenyl-docosyl, preferably C₇-C₁₈aralkyl such as benzyl, 2-benzyl-2-propyl, β-phenyl-ethyl, α,α-dimethylbenzyl, ω-phenyl-butyl, ω,ω-dimethyl-ω-phenyl-butyl, ω-phenyl-dodecyl or ω-phenyl-octadecyl, and particularly preferred C₇-C₁₂aralkyl such as benzyl, 2-benzyl-2-propyl, β-phenyl-ethyl, α,α-dimethylbenzyl, ω-phenyl-butyl, or ω,ω-dimethyl-ω-phenyl-butyl, in which both the aliphatic hydrocarbon group and aromatic hydrocarbon group may be unsubstituted or substituted. Preferred examples are benzyl, 2-phenylethyl, 3-phenylpropyl, naphthylethyl, naphthylmethyl, and cumyl.

C₁-C₃₀heteroaryl (preferably C₂-C₁₃ heteroaryl) represents a ring with five to seven ring atoms or a condensed ring system, wherein nitrogen, oxygen or sulfur are the possible hetero atoms, and is typically a heterocyclic group with five to 30 atoms having at least six conjugated π-electrons such as thienyl, benzothiophenyl, dibenzothiophenyl, thianthrenyl, furyl, furfuryl, 2H-pyranyl, benzofuranyl, isobenzofuranyl, dibenzofuranyl, phenoxythienyl, tetrazol, pyrrolyl, imidazolyl, pyrazolyl, pyridyl, bipyridyl, triazinyl, pyrimidinyl, pyrazinyl, pyridazinyl, indolizinyl, isoindolyl, indolyl, indazolyl, purinyl, quinolizinyl, chinolyl, isochinolyl, phthalazinyl, naphthyridinyl, chinoxalinyl, chinazolinyl, cinnolinyl, pteridinyl, carbazolyl, carbolinyl, benzotriazolyl, benzoxazolyl, phenanthridinyl, acridinyl, pyrimidinyl, phenanthrolinyl, phenazinyl, isothiazolyl, phenothiazinyl, isoxazolyl, furazanyl, 4-imidazo[1,2-a]benzimidazolyl, 5-benzimidazo[1,2-a]benzimidazolyl, benzimidazolo[2,1-b][1,3]benzothiazolyl, carbazolyl, or phenoxazinyl, which can be unsubstituted or substituted. Benzimidazo[1,2-a]benzimidazo-5-yl, benzimidazo[1,2-a]benzimidazo-2-yl, carbazolyl and dibenzofuranyl are examples of a C₂-C₁₃heteroaryl group.

A C₂-C₁₃heteroaryl group is for example, benzimidazo[1,2-a]benzimidazo-5-yl ( 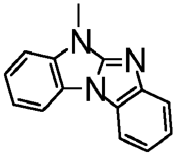 ), benzimidazo[1,2-a]benzimidazo-2-yl ( 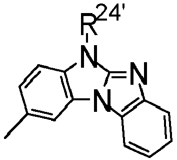 ), benzimidazolo[2,1-b][1,3]benzothiazolyl, benzimidazolo[2,1-b][1,3]benzoxazole, carbazolyl, dibenzofuranyl, or dibenzotihophenyl, which can be unsubstituted or substituted, especially by C₆-C₁₀aryl, or C₆-C₁₀aryl, which is substituted by C₁-C₄alkyl; or C₂-C₁₃heteroaryl.

C₂-C₃₀heteroaryl (preferably C₂-C₁₃ heteroarylaryl) means that the heteroaryl residue comprises at least 2 carbon atoms and at most 30 carbon atoms in the base skeleton (without substituents). The further atoms in the heteroaryl base skeleton are heteroatoms (N, O and/or S).

R^{24'} is in each case independently C₁-C₁₈alkyl, such as methyl, ethyl, n-propyl, iso-propyl, n-butyl, isobutyl, sec-butyl, hexyl, octyl, or 2-ethyl-hexyl, or C₆-C₁₄aryl, such as phenyl, tolyl, naphthyl, phenanthronyl, triphenylenyl, fluoranthenyl or biphenylyl

C₆-C₂₄arylene groups are typically phenylene, 4-methylphenylene, 4-methoxyphenylene, naphthylene, especially 1-naphthylene, or 2-naphthylene, biphenylylene, terphenylylene, pyrenylene, 2- or 9-fluorenylene, phenanthrylene, or anthrylene, which may be unsubstituted or substituted. Preferred C₆-C₂₄arylene groups are 1,4-phenylene, 1,3-phenylene, 1,2-phenylene, 3,3'-biphenylylene, 3,3'-m-terphenylene, 2- or 9-fluorenylene, phenanthrylene, or 4-t-butyl-phenylene, which may be unsubstituted or substituted. More preferred arylene groups are 1,4-phenylene, 1,3-phenylene, 1,2-phenylene groups and 4-t-butyl-phenylene.

C₂-C₃₀heteroarylene groups, which optionally can be substituted, represent a ring with five to seven ring atoms or a condensed ring system comprising at least one heteroatom. Nitrogen, oxygen or sulfur are the possible hetero atoms. Typical heteroarylene groups are heterocyclic groups with five to 30 atoms having at least six conjugated -electrons such as thienylene, benzothiophenylene, dibenzothiophenylene, thianthrenylene, furylene, furfurylene, 2H-pyranylene, benzofuranylene, isobenzofuranylene, dibenzofuranylene, phenoxythienylene, pyrrolylene, imidazolylene, pyrazolylene, pyridylene, bipyridylene, triazinylene, pyrimidinylene, pyrazinylene, pyridazinylene, indolizinylene, isoindolylene, indolylene, indazolylene, purinylene, quinolizinylene, chinolylene, isochinolylene, phthalazinylene, naphthyridinylene, chinoxalinylene, chinazolinylene, cinnolinylene, pteridinylene, carbolinylene, benzotriazolylene, benzoxazolylene, phenanthridinylene, acridinylene, pyrimidinylene, phenanthrolinylene, phenazinylene, isothiazolylene, phenothiazinylene, isoxazolylene, furazanylene, carbazolylene, benzimidazo[1,2-a]benzimidazo-2,5-ylene, or phenoxazinylene, which can be unsubstituted or substituted. Preferred C₂-C₃₀heteroarylene groups are pyridylene, triazinylene, pyrimidinylene, carbazolylene, dibenzofuranylene and benzimidazo[1,2-a]benzimidazo-2,5-ylene ( 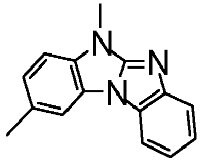 ), which can be unsubstituted or substituted, especially by C₆-C₁₀aryl, C₆-C₁₀aryl which is substituted by C₁-C₄alkyl; or C₂₋C₁₄heteroaryl.

Suitable substituents of the abovementioned groups are the groups E mentioned above and below.

If a substituent occurs more than one time in a group, it can be different in each occurrence.

The wording "substituted by E" means that one or more, especially one, two or three substituents E might be present.

As described above, some of the aforementioned groups may be substituted by E and/or, if desired, interrupted by D. Interruptions are of course possible only in the case of groups containing at least 2 carbon atoms connected to one another by single bonds; C₆-C₁₈aryl is not interrupted; interrupted arylalkyl contains the unit D in the alkyl moiety. "a C₁-C₁₈alkyl substituted by one or more E and/or interrupted by one or more units D" or "a C₁-C₁₈alkyl group, which is interrupted by at least one O" is for example, (CH₂CH₂O)₁₋₉-R^{x}, where R^{x} is H or C₁-C₁₀alkyl or C₂-C₁₀alkanoyl (e.g. CO-CH(C₂H₅)C₄H₉), CH₂-CH(OR^{y'})-CH₂-O-R^{y}, where R^{y} is C₁-C₁₈alkyl, C₅-C₁₂cycloalkyl, phenyl, C₇-C₁₅phenylalkyl, and R^{y'} embraces the same definitions as R^{y} or is H; C₁-C₈alkylene-COO-R^{z}, e.g. CH₂COOR_{z}, CH(CH₃)COOR^{z}, C(CH₃)₂COOR^{z}, where R^{z} is H, C₁-C₁₈alkyl, (CH₂CH₂O)₁₋₉-R^{x}, and R^{x} embraces the definitions indicated above; CH₂CH₂-O-CO-CH=CH₂; CH₂CH(OH)CH₂-O-CO-C(CH₃)=CH₂.

D is -CO-, -COO-, -S-, -SO-, -SO₂-, -O-, -CR¹⁵=CR¹⁶-, -NR¹⁷-, -SiR²²R²³-, -POR²⁵- or -C≡C-. Preferably, D is -CO-, -COO-, -S-, -SO-, -SO₂-, -O-, -NR¹⁷-, wherein R¹⁷ is C₁-C₁₈alkyl, such as methyl, ethyl, n-propyl, iso-propyl, n-butyl, isobutyl, or sec-butyl, or C₆-C₁₄aryl, such as phenyl, tolyl, naphthyl, or biphenylyl, or C₂-C₃₀heteroaryl, such as, for example, benzimidazo[1,2-a]benzimidazo-2-yl ( 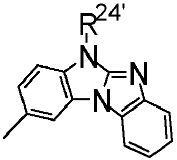 ), carbazolyl, dibenzofuranyl, dibenzothiophenyl, dialkyl fluranthenyl (dimethyl) which can be unsubstituted or substituted by E, especially by C₆-C₁₀aryl, which may in turn be unsubstituted or substituted by C₁-C₄alkyl; or C₂-C₁₃heteroaryl, which can be unsubstituted or substituted by E, which is preferably unsubstituted. More preferably, D is O.

E is -OR²¹, -SR²¹, -NR¹⁷R¹⁸, -COR²⁰, -COOR¹⁹, -CONR¹⁷R¹⁸, -CN, -SiR²²R²³R²⁴, -POR²⁵R²⁷, halogen, preferably F,
a C₆-C₄₀aryl group which is unsubstituted or is substituted by at least one group selected from a C₁-C₁₈alkyl group, a C₁-C₁₈alkoxy group, a C₁-C₁₈alkyl group, which is interrupted by at least one O, a C₆-C₁₈aryl group, which is in turn unsubstituted or substituted by at least one C₁-C₁₈alkyl group or at least one C₁-C₁₈alkoxy group, a C₅-C₁₂cycloalkyl group, which is in turn unsubstituted or substituted by at least one C₁-C₁₈alkyl group or at least one C₁-C₁₈alkoxy group, a C₁-C₁₈heteroaryl group, which is in turn unsubstituted or substituted by at least one C₁-C₁₈alkyl group or at least one C₁-C₁₈alkoxy group, -F, -CF₃, -CF₂CF₃, -CF₂CF₂CF₃, -CF(CF₃)₂, -(CF₂)₃CF₃
and -C(CF₃)₃,
a C₁-C₁₈ alkyl group,
a C₁-C₁₈ alkoxy group,
a C₁-C₂₄heteroaryl group which is unsubstituted or substituted by at least one group selected from a C₁-C₁₈alkyl group, a C₁-C₁₈alkoxy group, a C₁-C₁₈alkyl group, which is interrupted by at least one O, a C₆-C₁₈aryl group, which is in turn unsubstituted or substituted by at least one C₁-C₁₈alkyl group or at least one C₁-C₁₈alkoxy group, a C₅-C₁₂cycloalkyl group, which is in turn unsubstituted or substituted by at least one C₁-C₁₈alkyl group or at least one C₁-C₁₈alkoxy group, a C₁-C₁₈heteroaryl group, which is in turn unsubstituted or substituted by at least one C₁-C₁₈alkyl group or at least one C₁-C₁₈alkoxy group, -F, -CF₃, -CF₂CF₃, -CF₂CF₂CF₃, -CF(CF₃)₂, -(CF₂)₃CF₃ and -C(CF₃)₃.

Preferably, E is CN, F, CF₃,
a C₆-C₁₈aryl group which is unsubstituted or is substituted by at least one group selected from a C₁-C₁₈alkyl group, a C₁-C₁₈alkoxy group, an unsubstituted C₆-C₁₈aryl group,
a C₁-C₁₈alkyl group,
a C₁-C₂₄heteroaryl group which is unsubstituted or substituted by at least one group selected from a C₁-C₁₈alkyl group, a C₁-C₁₈alkoxy group, an unsubstituted C₆-C₁₈aryl group.

R¹⁵ and R¹⁶ are independently of each other H, a C₆-C₁₈aryl group which is unsubstituted or substituted by at least one C₁-C₁₈alkyl group or at least one C₁-C₁₈alkoxy group, preferably H, a C₆-C₁₀aryl group which is unsubstituted or substituted by at least one C₁-C₈alkyl group or at least one C₁-C₈alkoxy group, more preferably H.

R¹⁷ and R¹⁸ are independently of each other H, a C₆-C₁₈aryl group which is unsubstituted or substituted by at least one C₁-C₁₈alkyl group or at least one C₁-C₁₈alkoxy group, a C₁-C₁₈alkyl group or a C₁-C₁₈alkyl group, which is interrupted by at least one O, or
R¹⁷ and R¹⁸ together form a five or six membered aliphatic, aromatic or heteroaromatic ring, which is unsubstituted or substituted by at least one group E,

Preferably, R¹⁷ and R¹⁸ are independently of each other a C₆-C₁₀aryl group which is unsubstituted or substituted by at least one C₁-C₈alkyl group or at least one C₁-C₈alkoxy group, a C₁-C₈alkyl group or a C₁-C₈alkoxy group, more preferably a C₆-C₁₀aryl group which is unsubstituted.

R¹⁹ is H, a C₆-C₁₈aryl group which is unsubstituted or substituted by at least one C₁-C₁₈alkyl group or at least one C₁-C₁₈alkoxy group, a C₁-C₁₈alkyl group or a C₁-C₁₈alkyl group, which is interrupted by at least one O, preferably a C₆-C₁₀aryl group which is unsubstituted or substituted by at least one C₁-C₈alkyl group or at least one C₁-C₈alkoxy group or a C₁-C₈alkyl group, more preferably a C₆-C₁₀aryl group which is unsubstituted or a C₁-C₈alkyl group.

R²⁰ is H or a C₆-C₁₈aryl group which is unsubstituted or substituted by at least one C₁-C₁₈alkyl group or at least one C₁-C₁₈alkoxy group, a C₁-C₁₈ alkyl group or a C₁-C₁₈alkyl group, which is interrupted by at least one O, preferably a C₆-C₁₀aryl group which is unsubstituted or substituted by at least one C₁-C₈alkyl group or at least one C₁-C₈alkoxy group or a C₁-C₈alkyl group, more preferably a C₆-C₁₀aryl group which is unsubstituted or a C₁-C₈alkyl group.

R²¹ is independently of each other H, a C₆-C₁₈aryl group which is unsubstituted or substituted by at least one C₁-C₁₈alkyl group or at least one C₁-C₁₈alkoxy group or a C₁-C₁₈alkyl group, preferably a C₆-C₁₀aryl group which is unsubstituted or substituted by at least one C₁-C₈alkyl group or at least one C₁-C₈alkoxy group or a C₁-C₈alkyl group, more preferably a C₆-C₁₀aryl group which is unsubstituted or a C₁-C₈alkyl group.

R^{22,} R²³ and R²⁴ are independently of each other H, a C₁-C₁₈alkyl group, a C₆-C₁₈aryl group which is unsubstituted or substituted by at least one C₁-C₁₈alkyl group, preferably a C₆-C₁₀aryl group which is unsubstituted or substituted by at least one C₁-C₈alkyl group or at least one C₁-C₈alkoxy group or a C₁-C₈alkyl group, more preferably a C₆-C₁₀aryl group which is unsubstituted or a C₁-Csalkyl group.

R²⁵ and R²⁷ are independently of each other H, a C₁-C₁₈alkyl group, a C₆-C₁₈aryl group which is unsubstituted or substituted by at least one C₁-C₁₈alkyl group, a C₇-C₂₅aralkyl which is unsubstituted or substituted by at least one group E, a C₅-C₁₂cycloalkyl group which is unsubstituted or substituted by at least one group E, preferably a C₆-C₁₀aryl group which is unsubstituted or substituted by at least one C₁-C₈alkyl group or at least one C₁-C₈alkoxy group or a C₁-C₈alkyl group, more preferably a C₆-C₁₀aryl group which is unsubstituted or a C₁-C₈alkyl group.

### The donor group D²

D² is a donor group except for a group of formula (II).

Preferably, the donor group D² is selected from the groups consisting of (i), (ii) and (iii)
(i) a group of formula (III), 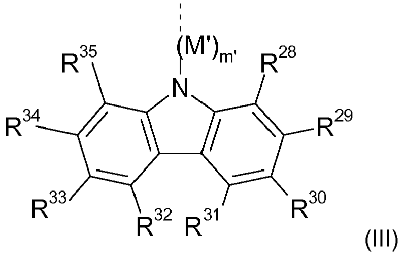 wherein
   the dotted line is a bonding site to formula (I),
   the residues R²⁸ to R³⁵ are independently of each other H, a C₁-C₂₅alkyl group which is unsubstituted or substituted by at least one group E and/or interrupted by at least one group D, preferably a C₁-C₂₅alkyl group which is unsubstituted or substituted by at least one group E, a C₆-C₄₀aryl group which is unsubstituted or substituted by at least one group E, a C₁-C₂₄heteroaryl group which is unsubstituted or substituted by at least one group E, a C₇-C₂₅aralkyl group which is unsubstituted or substituted by at least one group E, a C₅-C₁₂cycloalkyl group which is unsubstituted or substituted by at least one group E, -OR²¹, -SR²¹, -NR¹⁷R¹⁸, -COR²⁰, -COOR¹⁹, -CONR¹⁷R¹⁸, -SiR²²R²³R²⁴, -POR²⁵R²⁷, or halogen, or two adjacent residues R28, R²⁹, R³⁰, R³¹, R³², R³³, R³⁴ and R³⁵ together may form a ring of formula (IV) 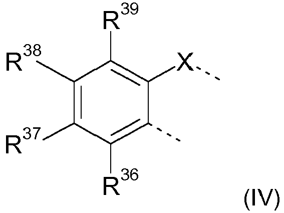 wherein
   X is O, S, NR^{17'}, CR^{18'}R^{19'}, SiR^{18'}R^{19'} or PO(OR^{18'}); preferably O, S, NR^{17'} or CR^{18'}R^{19'},
   wherein
   the dotted lines are bonding sites to the group of formula (III), wherein
   R³⁶ to R³⁹ are independently of each other H, a C₁-C₂₅alkyl group which is unsubstituted or substituted by at least one group E and/or interrupted by at least one group D, preferably a C₁-C₂₅alkyl group which is unsubstituted or substituted by at least one group E, a C₆-C₄₀aryl group which is unsubstituted or substituted by at least one group E, a C₁-C₂₄heteroaryl group which is unsubstituted or substituted by at least one group E, a C₇-C₂₅aralkyl group which is unsubstituted or substituted by at least one group E, a C₅-C₁₂cycloalkyl group which is unsubstituted or substituted by at least one group E, -OR²¹, -SR21, -NR¹⁷R¹⁸, -COR²⁰, -COOR¹⁹, -CONR¹⁷R¹⁸, -SiR²²R²³R²⁴, -POR²⁵R²⁷, or halogen, preferably, R³⁶ to R³⁹ are independently of each other H, a C₁-C₁₈alkyl group which is unsubstituted, a C₆-C₁₈aryl group which is unsubstituted or substituted by at least one group E or -OR²¹, or
   at least two of the residues R³⁶ to R³⁹ if present at adjacent carbon atoms together may form at least one C₆-C₁₈aryl ring or ring system, which is unsubstituted or substituted by at least one group E or at least one C₁-C₂₄heteroaryl ring or ring system, which is unsubstituted or substituted by at least one group E,
   preferably, the residues R²⁸ to R³⁵ are independently of each other H, a C₁-C₁₈alkyl group which is unsubstituted, a C₆-C₁₈aryl group which is unsubstituted or -OR²¹,
   more preferably, R³⁰ and R³³ are independently of each other H, a C₁-C₁₈alkyl group which is unsubstituted, a C₆-C₁₈aryl group which is unsubstituted or -OR²¹, and R²⁸, R²⁹, R³¹, R³², R³⁴ and R³⁵ are H,
   R^{17'} is a C₆-C₄₀aryl group which is unsubstituted or substituted by at least one group E or a C₁-C₂₄heteroaryl group which is unsubstituted or substituted by at least one group E,
   R^{18'} and R^{19'} are each independently hydrogen, a C₆-C₄₀aryl group which is unsubstituted or substituted by at least one group E or a C₁-C₂₄heteroaryl group which is unsubstituted or substituted by at least one group E,
   M' is a C₆-C₄₀arylene group which is unsubstituted or substituted by at least one group E, a C₁-C₂₄heteroarylene group which is unsubstituted or substituted by at least one group E, a C₁-C₂₅alkylene group which is unsubstituted or substituted by at least one group E and/or interrupted by at least one group D, preferably a C₁-C₂₅alkylene group which is unsubstituted or substituted by at least one group E, or a C₂-C₂₅alkenylene group which is unsubstituted or substituted by at least one group E and/or interrupted by at least one group D, preferably a C₂-C₂₅alkenylene group which is unsubstituted or substituted by at least one group E,
   preferably, M' is a C₆-C₄₀arylene group which is unsubstituted or substituted by at least one group E, a C₁-C₂₄heteroarylene group which is unsubstituted or substituted by at least one group E, more preferably, M' is a C₆-C₄₀arylene group which is unsubstituted, a C₁-C₂₄heteroarylene group which is unsubstituted, most preferably a C₆-C₁₀arylene group which is unsubstituted or a C₁-C₁₄heteroarylene group which is unsubstituted, further most preferably dibenzofuran-diyl, 1,2-phenylene, 1,3-phenylene or 1,4-phenylene, even further most preferably 1,2-phenylene,
   m' is 0, 1 or 2, preferably 0 or 1, more preferably 0;
(ii) a group of formula (V),

   ---(M")_{m"}-NAr₁Ar₂ (V)

   wherein
   Ar₁ and Ar₂ are each independently a C₆-C₄₀aryl group which is unsubstituted or substituted by at least one group E or a C₁-C₂₄heteroaryl group which is unsubstituted or substituted by at least one group E; or
   Ar₁ and Ar₂ may be connected together to form a ring,
   preferably, Ar₁ and Ar₂ are each independently a C₆-C₁₆aryl group which is unsubstituted or substituted by at least one group E or a C₂-C₁₃heteroaryl group which is unsubstituted or substituted by at least one group E;
   M" is a C₆-C₄₀arylene group which is unsubstituted or substituted by at least one group E, a C₁-C₂₄heteroarylene group which is unsubstituted or substituted by at least one group E, a C₁-C₂₅alkylene group which is unsubstituted or substituted by at least one group E and/or interrupted by at least one group D, preferably a C₁-C₂₅alkylene group which is unsubstituted or substituted by at least one group E, or a C₂-C₂₅alkenylene group which is unsubstituted or substituted by at least one group E and/or interrupted by at least one group D, preferably a C₂-C₂₅alkenylene group which is unsubstituted or substituted by at least one group E,
   preferably, M" is a C₆-C₄₀arylene group which is unsubstituted or substituted by at least one group E, a C₁-C₂₄heteroarylene group which is unsubstituted or substituted by at least one group E, more preferably, M" is a C₆-C₄₀arylene group which is unsubstituted, a C₁-C₂₄heteroarylene group which is unsubstituted, most preferably a C₆-C₁₀arylene group which is unsubstituted or a C₁-C₁₄heteroarylene group which is unsubstituted, further most preferably dibenzofuran-diyl, 1,2-phenylene, 1,3-phenylene or 1,4-phenylene, even further most preferably 1,2-phenylene,
   m" is 0, 1 or 2, preferably 0 or 1, more preferably 0,
   wherein the dotted line is a bonding site to formula (I);
   and
(iii) a group of formula (VI), 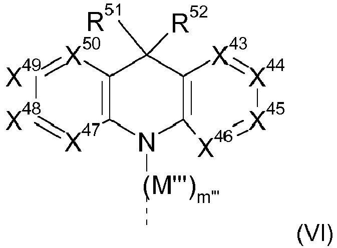 wherein
   X⁴³ is CR⁴³ or N;
   X⁴⁴ is CR⁴⁴ or N;
   X⁴⁵ is CR⁴⁵ or N;
   X⁴⁶ is CR⁴⁶ or N;
   X⁴⁷ is CR⁴⁷ or N;
   X⁴⁸ is CR⁴⁸ or N;
   X⁴⁹ is CR⁴⁹ or N;
   X⁵⁰ is CR⁵⁰ or N;
   wherein
   0, 1 or 2, preferably 0 or 1, more preferably 0 of the groups X⁴³, X⁴⁴, X⁴⁵, X⁴⁶, X⁴⁷, X⁴⁸, X⁴⁹ and X⁵⁰ are N;
   R⁴³ to R⁵⁰ are independently of each other H, -CN, a C₁-C₂₅alkyl group which is unsubstituted or substituted by at least one group E and/or interrupted by at least one group D, preferably a C₁-C₂₅alkyl group which is unsubstituted or substituted by at least one group E, a C₆-C₄₀aryl group which is unsubstituted or substituted by at least one group E, a C₁-C₂₄heteroaryl group which is unsubstituted or substituted by at least one group E, a C₇-C₂₅aralkyl group which is unsubstituted or substituted by at least one group E, a C₅-C₁₂cycloalkyl group which is unsubstituted or substituted by at least one group E, -OR²¹, -SR²¹, -NR¹⁷R¹⁸, -COR²⁰, -COOR¹⁹, -CONR¹⁷R¹⁸,-SiR²²R²³R²⁴, -POR²⁵R²⁷, or halogen, or
   at least two of the residues R⁴³ to R⁵⁰ if present at adjacent carbon atoms together may form at least one C₆-C₁₈aryl ring, which is unsubstituted or substituted by at least one group E or at least one C₁-C₂₄heteroaryl ring, which is unsubstituted or substituted by at least one group E,
   preferably, R⁴³ to R⁵⁰ are independently of each other H, a C₁-C₁₈alkyl group which is unsubstituted, a C₆-C₁₈aryl group which is unsubstituted or substituted by at least one group E or -OR²¹, more preferably, R⁴³ to R⁵⁰ are H,
   M''' is a C₆-C₄₀arylene group which is unsubstituted or substituted by at least one group E, a C₁-C₂₄heteroarylene group which is unsubstituted or substituted by at least one group E, a C₁-C₂₅alkylene group which is unsubstituted or substituted by at least one group E and/or interrupted by at least one group D, preferably a C₁-C₂₅alkylene group which is unsubstituted or substituted by at least one group E, or a C₂-C₂₅alkenylene group which is unsubstituted or substituted by at least one group E and/or interrupted by at least one group D, preferably a C₂-C₂₅alkenylene group which is unsubstituted or substituted by at least one group E,
   preferably, M''' is a C₆-C₄₀arylene group which is unsubstituted or substituted by at least one group E, a C₁-C₂₄heteroarylene group which is unsubstituted or substituted by at least one group E, more preferably, M''' is a C₆-C₄₀arylene group which is unsubstituted, a C₁-C₂₄heteroarylene group which is unsubstituted, most preferably a C₆-C₁₀arylene group which is unsubstituted or a C₁-C₁₄heteroarylene group which is unsubstituted, further most preferably dibenzofuran-diyl, 1,2-phenylene, 1,3-phenylene or 1,4-phenylene, even further most preferably 1,2-phenylene,
   m''' is 0, 1 or 2, preferably 0 or 1, more preferably 0,
   wherein the dotted line is a bonding site to formula (I);
   R⁵¹ and R⁵² are each independently hydrogen, a C₁-C₂₅alkyl group which is unsubstituted or substituted by at least one group E and/or interrupted by at least one group D, preferably a C₁-C₂₅alkyl group which is unsubstituted or substituted by at least one group E, a C₆-C₄₀aryl group which is unsubstituted or substituted by at least one group E or a C₁-C₂₄heteroaryl group which is unsubstituted or substituted by at least one group E,
   or
   R⁵¹ and R⁵² may together form a ring, which is unsubstituted or substituted by at least one group E.

Most preferably, the donor group D² in the compounds of formula (I) is a group of formula (III).

The present invention therefore preferably relates to a compound of formula (I), 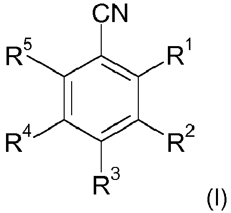 wherein
R¹, R², R³, R⁴ and R⁵ are R^{A}, D¹ or D²;
wherein
0, 1 or 2 of the residues R¹, R², R³, R⁴ and R⁵ are R^{A};
1, 2, 3 or 4 of the residues R¹, R², R³, R⁴ and R⁵ are D²; and
1, 2, 3 or 4 of the residues R¹, R², R³, R⁴ and R⁵ are D¹;
wherein the sum of the residues R^{A}, D² and D¹ is 5, and preferably, the sum of D² and D¹ is 3, 4 or 5, more preferably 3 or 4, most preferably 4;
D² is a donor group of formula (III) 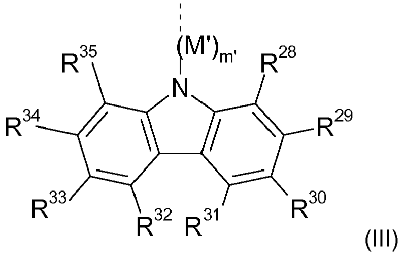 wherein
the dotted line is a bonding site to formula (I),
the residues R²⁸ to R³⁵ are independently of each other H, a C₁-C₂₅alkyl group which is unsubstituted or substituted by at least one group E and/or interrupted by at least one group D, preferably a C₁-C₂₅alkyl group which is unsubstituted or substituted by at least one group E, a C₆-C₄₀aryl group which is unsubstituted or substituted by at least one group E, a C₁-C₂₄heteroaryl group which is unsubstituted or substituted by at least one group E, a C₇-C₂₅aralkyl group which is unsubstituted or substituted by at least one group E, a C₅-C₁₂cycloalkyl group which is unsubstituted or substituted by at least one group E, -OR²¹, -SR²¹, -NR¹⁷R¹⁸, -COR²⁰, -COOR¹⁹, -CONR¹⁷R¹⁸,-SiR²²R²³R²⁴, -POR²⁵R²⁷, or halogen, or
two adjacent residues R²⁸, R²⁹, R³⁰, R³¹, R³², R³³, R³⁴ and R³⁵ together may form a ring of formula (IV) 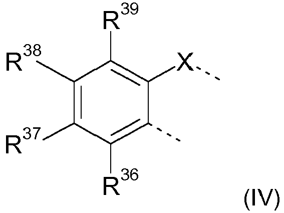 wherein
X is O, S, NR^{17'}, CR^{18'}R^{19'}, SiR^{18'}R^{19'} or PO(OR^{18'}); preferably O, S, NR^{17'} or CR^{18'}R^{19'},
wherein
the dotted lines are bonding sites to the group of formula (III), wherein
R³⁶ to R³⁹ are independently of each other H, a C₁-C₂₅alkyl group which is unsubstituted or substituted by at least one group E and/or interrupted by at least one group D, preferably a C₁-C₂₅alkyl group which is unsubstituted or substituted by at least one group E, a C₆-C₄₀aryl group which is unsubstituted or substituted by at least one group E, a C₁-C₂₄heteroaryl group which is unsubstituted or substituted by at least one group E, a C₇-C₂₅aralkyl group which is unsubstituted or substituted by at least one group E, a C₅-C₁₂cycloalkyl group which is unsubstituted or substituted by at least one group E, -OR²¹, -SR²¹, -NR¹⁷R¹⁸, -COR²⁰, -COOR¹⁹, -CONR¹⁷R¹⁸, -SiR²²R²³R²⁴,-POR²⁵R²⁷, or halogen, preferably, R³⁶ to R³⁹ are independently of each other H, a C₁-C₁₈alkyl group which is unsubstituted, a C₆-C₁₈aryl group which is unsubstituted or substituted by at least one group E or -OR²¹, or
at least two of the residues R³⁶ to R³⁹ if present at adjacent carbon atoms together may form at least one C₆-C₁₈aryl ring or ring system, which is unsubstituted or substituted by at least one group E or at least one C₁-C₂₄heteroaryl ring or ring system, which is unsubstituted or substituted by at least one group E,
preferably, the residues R²⁸ to R³⁵ are independently of each other H, a C₁-C₁₈alkyl group which is unsubstituted, a C₆-C₁₈aryl group which is unsubstituted or -OR²¹,
more preferably, R³⁰ and R³³ are independently of each other H, a C₁-C₁₈alkyl group which is unsubstituted, a C₆-C₁₈aryl group which is unsubstituted or -OR²¹, and R²⁸, R²⁹, R³¹, R³², R³⁴ and R³⁵ are H,
R^{17'} is a C₆-C₄₀aryl group which is unsubstituted or substituted by at least one group E or a C₁-C₂₄heteroaryl group which is unsubstituted or substituted by at least one group E,
R^{18'} and R^{19'} are each independently hydrogen, a C₆-C₄₀aryl group which is unsubstituted or substituted by at least one group E or a C₁-C₂₄heteroaryl group which is unsubstituted or substituted by at least one group E,
M' is a C₆-C₄₀arylene group which is unsubstituted or substituted by at least one group E, a C₁-C₂₄heteroarylene group which is unsubstituted or substituted by at least one group E, a C₁-C₂₅alkylene group which is unsubstituted or substituted by at least one group E and/or interrupted by at least one group D, preferably a C₁-C₂₅alkylene group which is unsubstituted or substituted by at least one group E, or a C₂-C₂₅alkenylene group which is unsubstituted or substituted by at least one group E and/or interrupted by at least one group D, preferably a C₂-C₂₅alkenylene group which is unsubstituted or substituted by at least one group E,
preferably, M' is a C₆-C₄₀arylene group which is unsubstituted or substituted by at least one group E, a C₁-C₂₄heteroarylene group which is unsubstituted or substituted by at least one group E, more preferably, M' is a C₆-C₄₀arylene group which is unsubstituted, a C₁-C₂₄heteroarylene group which is unsubstituted, most preferably a C₆-C₁₀arylene group which is unsubstituted or a C₁-C₁₄heteroarylene group which is unsubstituted, further most preferably dibenzofuran-diyl, 1,2-phenylene, 1,3-phenylene or 1,4-phenylene, even further most preferably1,2-phenylene,
m' is 0, 1 or 2, preferably 0 or 1, more preferably 0;
D¹ is a group of formula (II) 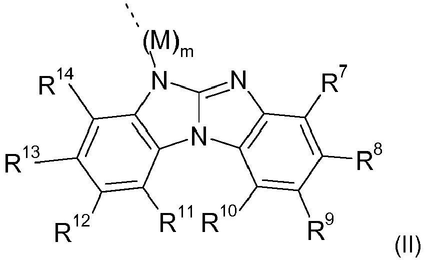 wherein
R⁷ to R¹⁴ are independently of each other H, a C₁-C₂₅alkyl group which is unsubstituted or substituted by at least one group E and/or interrupted by at least one group D, preferably a C₁-C₂₅alkyl group which is unsubstituted or substituted by at least one group E, a C₆-C₄₀aryl group which is unsubstituted or substituted by at least one group E, a C₁-C₂₄heteroaryl group which is unsubstituted or substituted by at least one group E, a C₇-C₂₅aralkyl group which is unsubstituted or substituted by at least one group E, a C₅-C₁₂cycloalkyl group which is unsubstituted or substituted by at least one group E, -OR²¹, -SR²¹, -NR¹⁷R¹⁸, -COR²⁰, -COOR¹⁹, -CONR¹⁷R¹⁸, -SiR²²R²³R²⁴, -POR²⁵R²⁷, or halogen, or
at least two of the remaining residues R⁷ to R¹⁴ if present at adjacent carbon atoms together form at least one C₆-C₁₈aryl ring or ring system which is unsubstituted or substituted by at least one group E or at least one C₁-C₂₄heteroaryl ring or ring system which is unsubstituted or substituted by at least one group E,
preferably, R⁷ to R¹⁴ are H,
R^{A} is H, a C₁-C₂₅alkyl group which is unsubstituted or substituted by at least one group E and/or interrupted by at least one group D, preferably a C₁-C₂₅alkyl group which is unsubstituted or substituted by at least one group E, a C₆-C₄₀aryl group which is unsubstituted or substituted by at least one group E, a C₁-C₂₄heteroaryl group which is unsubstituted or substituted by at least one group E, a C₇-C₂₅aralkyl group which is unsubstituted or substituted by at least one group E, a C₅-C₁₂cycloalkyl group which is unsubstituted or substituted by at least one group E, -OR²¹, -SR²¹, - NR¹⁷R¹⁸, -COR²⁰, -COOR¹⁹, -CONR¹⁷R¹⁸, -SiR²²R²³R²⁴, -POR²⁵R²⁷, or halogen, or
at least two of the residues R^{A} if present at adjacent carbon atoms together form at least one C₆-C₁₈aryl ring or ring system which is unsubstituted or substituted by at least one group E or at least one C₁-C₂₄heteroaryl ring or ring system which is unsubstituted or substituted by at least one group E,
preferably, R^{A} is H,
wherein the dotted line is a bonding site to formula (I),
M is a C₆-C₄₀arylene group which is unsubstituted or substituted by at least one group E, a C₁-C₂₄heteroarylene group which is unsubstituted or substituted by at least one group E, a C₁-C₂₅alkylene group which is unsubstituted or substituted by at least one group E and/or interrupted by at least one group D, preferably a C₁-C₂₅alkylene group which is unsubstituted or substituted by at least one group E, or a C₂-C₂₅alkenylene group which is unsubstituted or substituted by at least one group E and/or interrupted by at least one group D, preferably a C₂-C₂₅alkenylene group which is unsubstituted or substituted by at least one group E,
preferably, M is a C₆-C₄₀arylene group which is unsubstituted or substituted by at least one group E, a C₁-C₂₄heteroarylene group which is unsubstituted or substituted by at least one group E, more preferably, M is a C₆-C₄₀arylene group which is unsubstituted, a C₁-C₂₄heteroarylene group which is unsubstituted, most preferably a C₆-C₁₀arylene group which is unsubstituted or a C₁-C₁₄heteroarylene group which is unsubstituted, further most preferably dibenzofuran-diyl, 1,2-phenylene, 1,3-phenylene or 1,4-phenylene, even further most preferably 1,2-phenylene,
m is 0, 1 or 2, preferably 0 or 1, more preferably 0,
D is -CO-, -COO-, -S-, -SO-, -SO₂-, -O-, -CR¹⁵=CR¹⁶-, -NR¹⁷-, -SiR²²R²³-, -POR²⁵-, -C≡C-,
preferably, D is -CO-, -COO-, -S-, -SO-, -SO₂-, -O-, -NR¹⁷-, more preferably, D is O,
E is -OR²¹, -SR²¹, -NR¹⁷R¹⁸, -COR²⁰, -COOR¹⁹, -CONR¹⁷R¹⁸, -CN, -SiR²²R²³R²⁴, -POR²⁵R²⁷, halogen, preferably F,
a C₆-C₄₀aryl group which is unsubstituted or is substituted by at least one group selected from a C₁-C₁₈alkyl group, a C₁-C₁₈alkoxy group, a C₆-C₁₈aryl group, which is in turn unsubstituted or substituted by at least one C₁-C₁₈alkyl group or at least one C₁-C₁₈alkoxy group, a C₅-C₁₂cycloalkyl group, which is in turn unsubstituted or substituted by at least one C₁-C₁₈alkyl group or at least one C₁-C₁₈alkoxy group, a C₁-C₁₈heteroaryl group, which is in turn unsubstituted or substituted by at least one C₁-C₁₈alkyl group or at least one C₁-C₁₈alkoxy group, -F, -CF₃, -CF₂CF₃, -CF₂CF₂CF₃, -CF(CF₃)₂, -(CF₂)₃CF₃ and -C(CF₃)₃,
a C₁-C₁₈ alkyl group, or
a C₁-C₂₄heteroaryl group which is unsubstituted or is substituted by at least one group selected from a C₁-C₁₈alkyl group, a C₁-C₁₈alkoxy group, a C₆-C₁₈aryl group, which is in turn unsubstituted or substituted by at least one C₁-C₁₈alkyl group or at least one C₁-C₁₈alkoxy group, a C₅-C₁₂cycloalkyl group, which is in turn unsubstituted or substituted by at least one C₁-C₁₈alkyl group or at least one C₁-C₁₈alkoxy group, a C₁-C₁₈heteroaryl group, which is in turn unsubstituted or substituted by at least one C₁-C₁₈alkyl group or at least one C₁-C₁₈alkoxy group, -F, -CF₃, -CF₂CF₃, -CF₂CF₂CF₃, -CF(CF₃)₂, -(CF₂)₃CF₃ and -C(CF₃)₃,
preferably, E is CN, F, CF₃,
a C₆-C₁₈aryl group which is unsubstituted or is substituted by at least one group selected from a C₁-C₁₈alkyl group, a C₁-C₁₈alkoxy group, an unsubstituted C₆-C₁₈aryl group,
a C₁-C₁₈ alkyl group,
a C₁-C₂₄heteroaryl group which is unsubstituted or substituted by at least one group selected from a C₁-C₁₈alkyl group, a C₁-C₁₈alkoxy group, an unsubstituted C₆-C₁₈aryl group,
R¹⁵ and R¹⁶ are independently of each other H, a C₆-C₁₈aryl group which is unsubstituted or substituted by at least one C₁-C₁₈alkyl group or at least one C₁-C₁₈alkoxy group, a C₁-C₁₈alkyl group or a C₁-C₁₈alkoxy group,
R¹⁷ and R¹⁸ are independently of each other H , a C₆-C₁₈aryl group which is unsubstituted or substituted by at least one C₁-C₁₈alkyl group or at least one C₁-C₁₈alkoxy group, a C₁-C₁₈alkyl group or a C₁-C₁₈alkoxy group, or
R¹⁷ and R¹⁸ together form a five or six membered aliphatic, aromatic or heteroaromatic ring, which is unsubstituted or substituted by at least one group E,
R¹⁹ is H, a C₆-C₁₈aryl group which is unsubstituted or substituted by at least one C₁-C₁₈alkyl group or at least one C₁-C₁₈alkoxy group, a C₁-C₁₈alkyl group or a C₁-C₁₈alkoxy group,
R²⁰ is H or a C₆-C₁₈aryl group which is unsubstituted or substituted by at least one C₁-C₁₈alkyl group or at least one C₁-C₁₈alkoxy group, a C₁-C₁₈alkyl group or a C₁-C₁₈alkoxy group,
R²¹ is H, a C₆-C₁₈aryl group which is unsubstituted or substituted by at least one C₁-C₁₈alkyl group or at least one C₁-C₁₈alkoxy group or a C₁-C₁₈alkyl group,
R²², R²³ and R²⁴ are independently of each other H, a C₁-C₁₈alkyl group or a C₆-C₁₈aryl group which is unsubstituted or substituted by at least one C₁-C₁₈alkyl group, and
R²⁵ and R²⁷ are independently of each other H, a C₁-C₁₈alkyl group, a C₆-C₁₈aryl group which is unsubstituted or substituted by at least one C₁-C₁₈alkyl group, a C₇-C₂₅aralkyl which is unsubstituted or substituted by at least one group E, a C₅-C₁₂cycloalkyl group which is unsubstituted or substituted by at least one group E.

### (i) D², the donor group of formula (III)

The donor group D² of formula (III) is described above.

Preferably, the residues R²⁸ to R³⁵ in the donor group of formula (III) are independently of each other H, a C₁-C₁₈alkyl group which is unsubstituted, a C₆-C₁₈aryl group which is unsubstituted or - OR21,
more preferably, R³⁰ and R³³ are independently of each other H, a C₁-C₁₈alkyl group which is unsubstituted, a C₆-C₁₈aryl group which is unsubstituted or -OR²¹, and R²⁸, R²⁹, R³¹, R³², R³⁴ and R³⁵ are H,

M' is a C₆-C₄₀arylene group which is unsubstituted or substituted by at least one group E, a C₁-C₂₄heteroarylene group which is unsubstituted or substituted by at least one group E, preferably, M' is a C₆-C₄₀arylene group which is unsubstituted, a C₁-C₂₄heteroarylene group which is unsubstituted, more preferably a C₆-C₁₀arylene group which is unsubstituted or a C₁-C₁₄heteroarylene group which is unsubstituted, most preferably dibenzofuran-diyl, 1,2-phenylene, 1,3-phenylene or 1,4-phenylene, further most preferably1,2-phenylene.
m' is 0 or 1, more preferably 0.
R²¹ is H, a C₆-C₁₈aryl group which is unsubstituted or substituted by at least one C₁-C₁₈alkyl group or at least one C₁-C₁₈alkoxy group or a C₁-C₁₈alkyl group.

Preferably, D² is 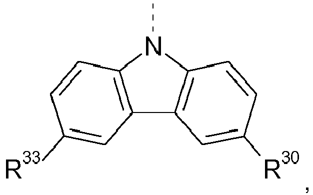 wherein
R³⁰ and R³³ are independently of each other H, a C₁-C₁₈alkyl group which is unsubstituted, a C₆-C₁₈aryl group which is unsubstituted or -OR²¹, and
R²¹ is H, a C₆-C₁₈aryl group which is unsubstituted or substituted by at least one C₁-C₁₈alkyl group or at least one C₁-C₁₈alkoxy group or a C₁-C₁₈alkyl group.

More preferred groups D² are: 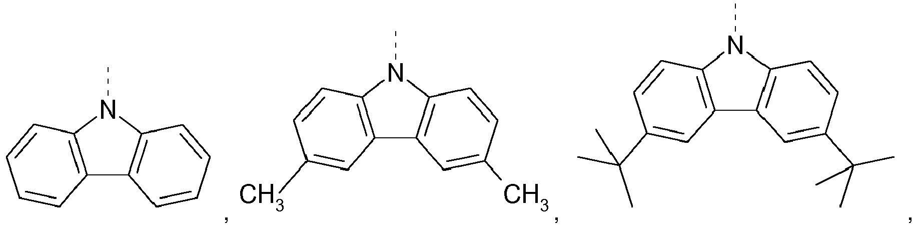 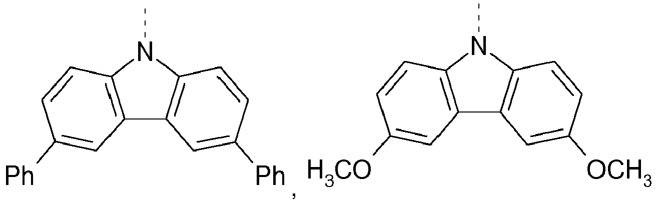 wherein the dotted line is a bonding site to the compound of formula (I).

### (ii) D², the donor group of formula (V)

The donor group D² of formula (V) is described above.

Preferably, Ar₁ and Ar₂ are each independently a C₆-C₁₀aryl group which is unsubstituted or substituted by at least one group E or a C₂-C₁₃heteroaryl group which is unsubstituted or substituted by at least one group E, more preferably a C₆-C₁₀aryl group which is unsubstituted or a C₂-C₁₃heteroaryl group which is unsubstituted, most preferably phenyl.

Most preferred groups of formula (V) are the following groups:

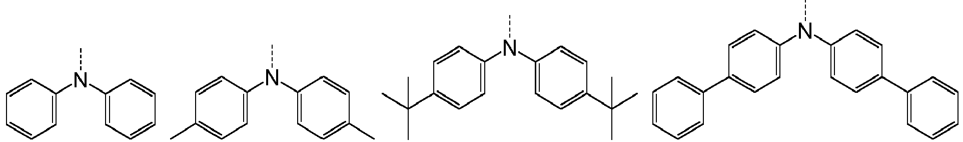

### (iii) D², the donor group of formula (VI)

The donor group D² of formula (VI) is described above.

Preferably, R⁴³ to R⁵⁰ are independently of each other H, a C₁-C₈alkyl group which is unsubstituted or substituted by at least one group E and/or interrupted by at least one group D, a C₆-C₁₀aryl group which is unsubstituted or substituted by at least one group E or a C₂-C₁₃heteroaryl group which is unsubstituted or substituted by at least one group E, more preferably H, a C₁-C₈alkyl group which is unsubstituted or a C₆-C₁₀aryl group which is unsubstituted, most preferably H.

Preferably, R⁵¹ and R⁵² are each independently a C₁-C₈alkyl group which is unsubstituted or substituted by at least one group E and/or interrupted by at least one group D, a C₆-C₁₀aryl group which is unsubstituted or substituted by at least one group E or a C₂-C₁₃heteroaryl group which is unsubstituted or substituted by at least one group E,
or
R⁵¹ and R⁵² may togetherform a five or six membered ring.

Most preferably, R⁵¹ and R⁵² are each independently a C₁-C₈alkyl group which is unsubstituted or a C₆-C₁₀aryl group which is unsubstituted.

Most preferred groups of formula (VI) are represented by formula (VIA) 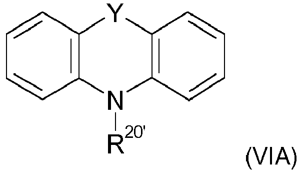 wherein
Y is CR⁵¹R⁵², O or S,
R⁵¹ and R⁵² are each independently a C₁-C₈alkyl group which is unsubstituted or substituted by at least one group E and/or interrupted by at least one group D, a C₆-C₁₀aryl group which is unsubstituted or substituted by at least one group E or a C₂-C₁₃heteroaryl group which is unsubstituted or substituted by at least one group E,
or
R⁵¹ and R⁵² may togetherform a five or six membered ring;
Preferably, R⁵¹ and R⁵² are each independently a C₁-C₈alkyl group which is unsubstituted or a C₆-C₁₀aryl group which is unsubstituted,
R^{20'} is a group -(M)ₘ---, preferably a bonding site, i.e. m is 0,
wherein the dotted line is a bonding site to formula (I).

Most preferred groups of formula (VI) are the following groups:

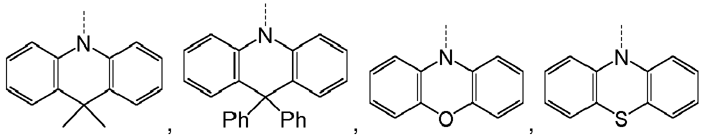

The groups and residues D, E, M, m, R¹⁵ to R²⁵ and R²⁷ in the groups mentioned under (i), (ii) and (iii) are defined above.

### D¹, the group of formula (II)

The group D¹ of formula (II) is described above.

Preferably R⁷ to R¹⁴ in the group D¹ are independently of each other H, a C₁-C₁₈alkyl group which is unsubstituted, a C₆-C₁₈aryl group which is unsubstituted or substituted by at least one group E or -OR²¹,
more preferably, R⁹ and R¹² are independently of each other H, a C₁-C₁₈alkyl group which is unsubstituted, a C₆-C₁₈aryl group which is unsubstituted or -OR²¹, and R⁷, R⁸, R¹⁰, R¹¹, R¹³ and R¹⁴ are H,
most preferably, R⁷ to R¹⁴ in the group D¹ are H.

M' is a C₆-C₄₀arylene group which is unsubstituted or substituted by at least one group E, a C₁-C₂₄heteroarylene group which is unsubstituted or substituted by at least one group E, more preferably, M' is a C₆-C₄₀arylene group which is unsubstituted, a C₁-C₂₄heteroarylene group which is unsubstituted, most preferably a C₆-C₁₀arylene group which is unsubstituted or a C₁-C₁₄heteroarylene group which is unsubstituted, further most preferably dibenzofuran-diyl, 1,2-phenylene, 1,3-phenylene or 1,4-phenylene, even further most preferably 1,2-phenylene.

m' is 0 or 1, more preferably 0.
R²¹ is H, a C₆-C₁₈aryl group which is unsubstituted or substituted by at least one C₁-C₁₈alkyl group or at least one C₁-C₁₈alkoxy group or a C₁-C₁₈alkyl group.

Most preferred group D¹ are the following groups:

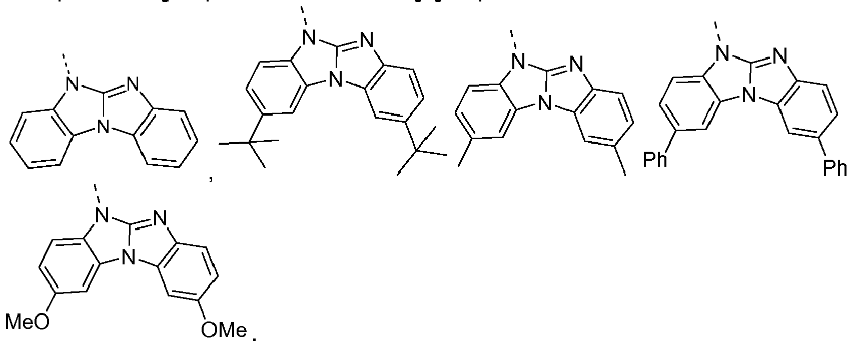

The present invention therefore more preferably relates to a compound of formula (I), 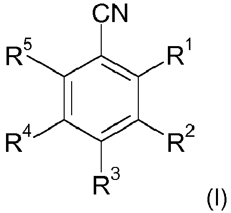 wherein
R¹, R², R³, R⁴ and R⁵ are R^{A}, D¹ or D²;
wherein
0, 1 or 2 of the residues R¹, R², R³, R⁴ and R⁵ are R^{A};
1, 2, 3 or 4 of the residues R¹, R², R³, R⁴ and R⁵ are D²; and
1, 2, 3 or 4 of the residues R¹, R², R³, R⁴ and R⁵ are D¹;
wherein the sum of the residues R^{A}, D² and D¹ is 5, and the sum of D² and D¹ is 3, 4 or 5, more preferably 3 or 4, most preferably 4;
D² is a donor group selected from the group consisting of 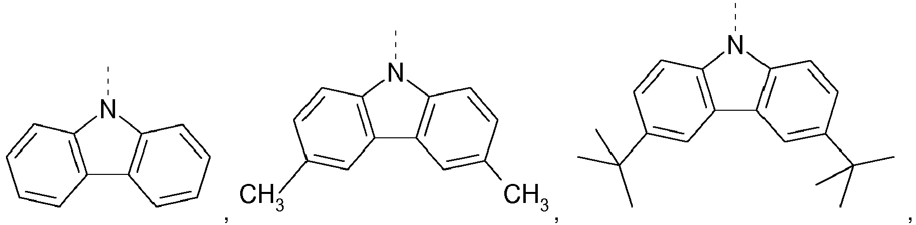 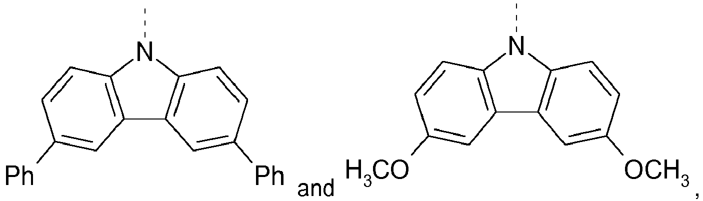 wherein
the dotted line is a bonding site to formula (I),
D¹ is a group of the following formula 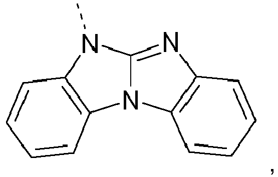 wherein
the dotted line is a bonding site to formula (I),
R^{A} is H, a C₁-C₂₅alkyl group which is unsubstituted or substituted by at least one group E and/or interrupted by at least one group D, preferably a C₁-C₂₅alkyl group which is unsubstituted or substituted by at least one group E, a C₆-C₄₀aryl group which is unsubstituted or substituted by at least one group E, a C₁-C₂₄heteroaryl group which is unsubstituted or substituted by at least one group E, a C₇-C₂₅aralkyl group which is unsubstituted or substituted by at least one group E, a C₅-C₁₂cycloalkyl group which is unsubstituted or substituted by at least one group E, -OR²¹, -SR²¹,-NR¹⁷R¹⁸, -COR²⁰, -COOR¹⁹, -CONR¹⁷R¹⁸, -SiR²²R²³R²⁴, -POR²⁵R²⁷, or halogen, or
at least two of the residues R^{A} if present at adjacent carbon atoms together form at least one C₆-C₁₈aryl ring or ring system which is unsubstituted or substituted by at least one group E or at least one C₁-C₂₄heteroaryl ring or ring system which is unsubstituted or substituted by at least one group E,
preferably, R^{A} is H.

In the compounds of formula (I), R¹, R², R³, R⁴ and R⁵ are R^{A}, D¹ or D²;
wherein
0, 1 or 2 of the residues R¹, R², R³, R⁴ and R⁵ are R^{A};
1, 2, 3 or 4 of the residues R¹, R², R³, R⁴ and R⁵ are D²; and
1, 2, 3 or 4 of the residues R¹, R², R³, R⁴ and R⁵ are D¹;
wherein the sum of the residues R^{A}, D² and D¹ is 5, and the sum of D² and D¹ is 3, 4 or 5, more preferably 3 or 4, most preferably 4.

Therefore, the compounds of formula (I) comprise at least 3 groups D¹ and D².

Most preferably, the compounds of formula (I) comprise 4 groups D¹ and D² and one residue R^{A}. Said residue R^{A} may be present in any position R¹, R², R³, R⁴ or R⁵. Preferably, the residue R³ is R^{A}. Preferred groups D¹ and D² and preferred residues R^{A} are mentioned above. Most preferably, R^{A} is H.

Most preferably, the compound of formula (I) is therefore represented by a compound of formula (la) 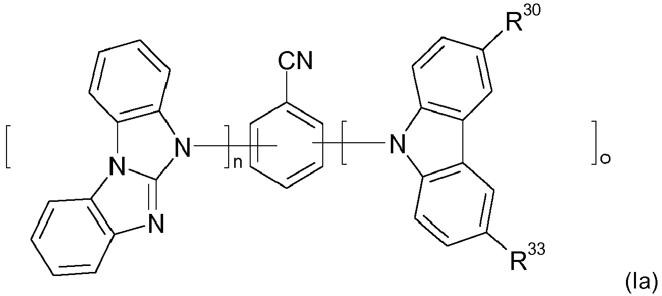 wherein
R³⁰ and R³³ are independently of each other H, a C₁-C₁₈alkyl group which is unsubstituted, a C₆-C₁₈aryl group which is unsubstituted or -OR²¹,
R²¹ is H, a C₆-C₁₈aryl group which is unsubstituted or substituted by at least one C₁-C₁₈alkyl group or at least one C₁-C₁₈alkoxy group or a C₁-C₁₈alkyl group,
n is 1, 2, 3 or 4, and
o is 1, 2, 3 or 4,
wherein the sum of n and o is 3, 4 or 5, preferably 4.

Preferred examples of the compounds of formula (I) are shown in the following table:

| Nr | R1 | R2 | R3 | R4 | R5 |
|---|---|---|---|---|---|
| 1 | 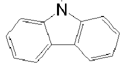 | 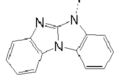 | H | 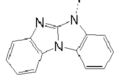 | 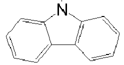 |
| 2 | 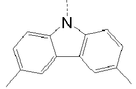 | 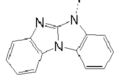 | H | 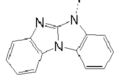 | 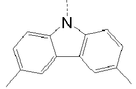 |
| 3 | 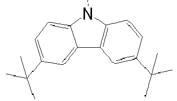 | 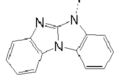 | H | 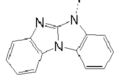 | 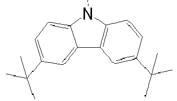 |
| 4 | 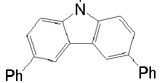 | 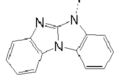 | H | 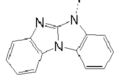 | 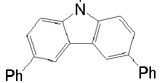 |
| 5 | 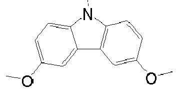 | 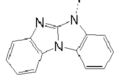 | H | 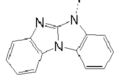 | 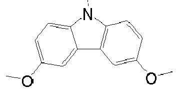 |
| 6 | 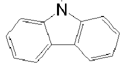 | 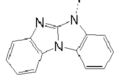 | H | 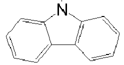 | 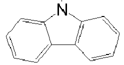 |
| 7 | 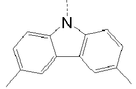 | 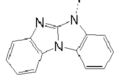 | H | 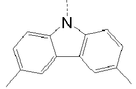 | 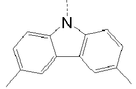 |
| 8 | 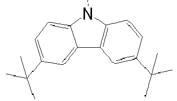 | 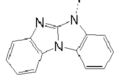 | H | 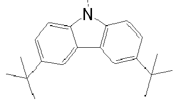 | 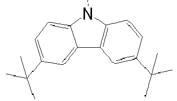 |
| 9 | 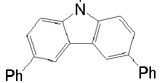 | 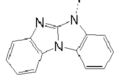 | H | 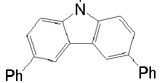 | 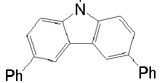 |
| 10 | 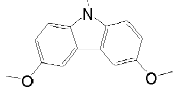 | 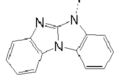 | H | 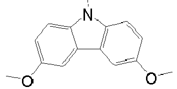 | 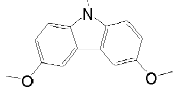 |
| 11 | 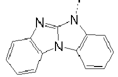 | 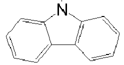 | H | 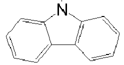 | 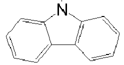 |
| 12 | 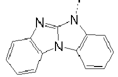 | 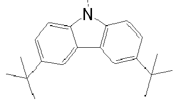 | H | 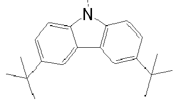 | 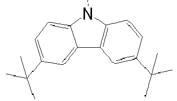 |
| 13 | 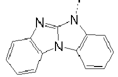 | 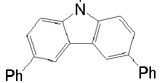 | H | 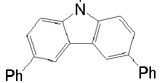 | 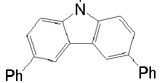 |
| 14 | 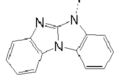 | 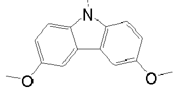 | H | 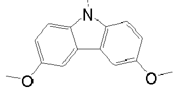 | 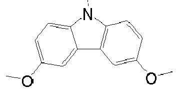 |
| 15 | 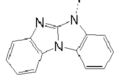 | 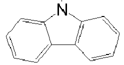 | H | H | 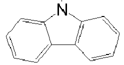 |
| 16 | 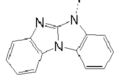 | 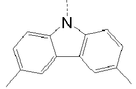 | H | H | 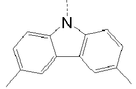 |
| 17 | 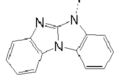 | 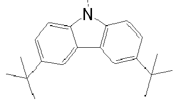 | H | H | 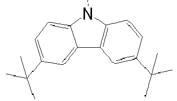 |
| 18 | 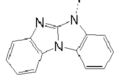 | 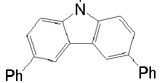 | H | H | 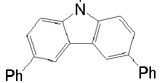 |
| 19 | 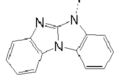 | 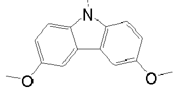 | H | H | 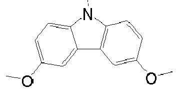 |
| 20 | 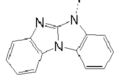 | 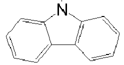 | 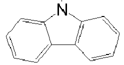 | H | 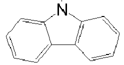 |
| 21 | 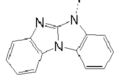 | 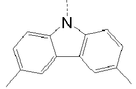 | 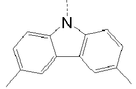 | | 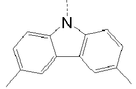 |
| 22 | 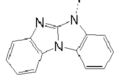 | 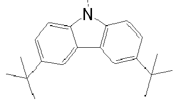 | 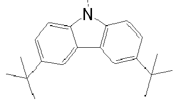 | H | 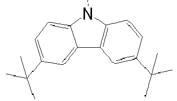 |
| 23 | 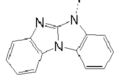 | 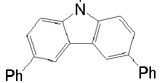 | 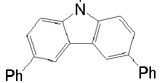 | H | 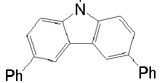 |
| 24 | 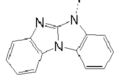 | 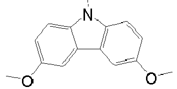 | 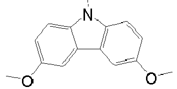 | H | 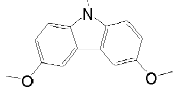 |
| 25 | 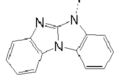 | 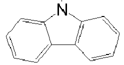 | H | 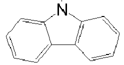 | 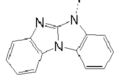 |
| 26 | 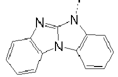 | 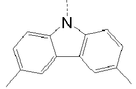 | H | 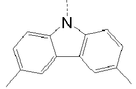 | 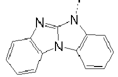 |
| 27 | 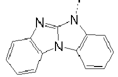 | 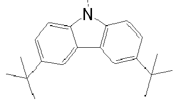 | H | 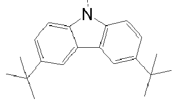 | 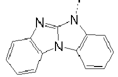 |
| 28 | 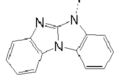 | 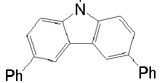 | H | 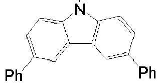 | 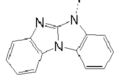 |
| 29 | 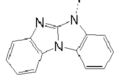 | 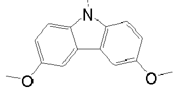 | H | 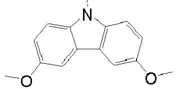 | 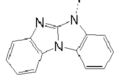 |
| 30 | 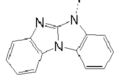 | 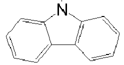 | H | H | 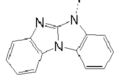 |
| 31 | 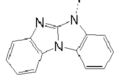 | 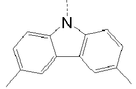 | H | H | 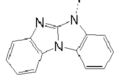 |
| 32 | 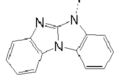 | 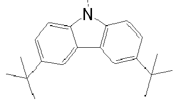 | H | H | 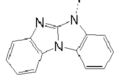 |
| 33 | 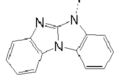 | 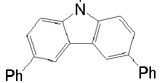 | H | H | 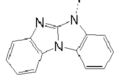 |
| 34 | 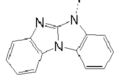 | 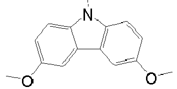 | H | H | 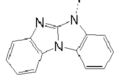 |
| 35 | 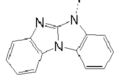 | 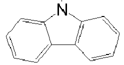 | 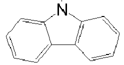 | H | H |
| 36 | 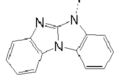 | 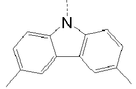 | 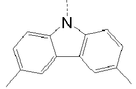 | H | H |
| 37 | 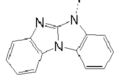 | 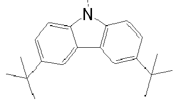 | 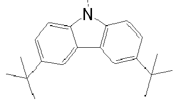 | H | H |
| 38 | 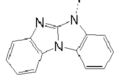 | 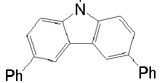 | 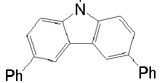 | H | H |
| 39 | 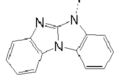 | 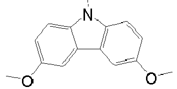 | 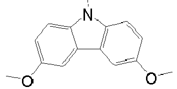 | H | H |
| 40 | 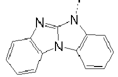 | 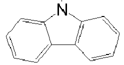 | 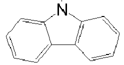 | 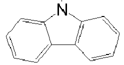 | 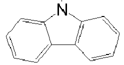 |
| 41 | 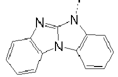 | 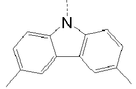 | 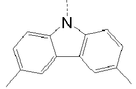 | 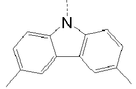 | 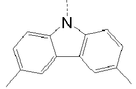 |
| 42 | 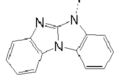 | 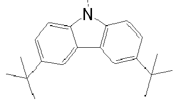 | 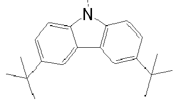 | 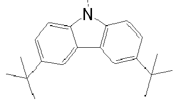 | 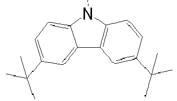 |
| 43 | 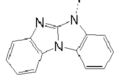 | 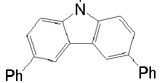 | 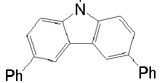 | 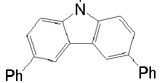 | 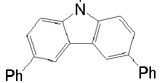 |
| 44 | 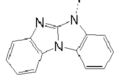 | 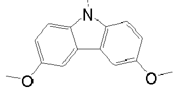 | 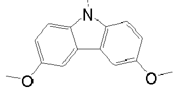 | 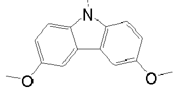 | 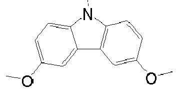 |
| 45 | 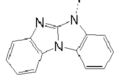 | 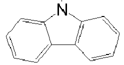 | H | 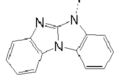 | 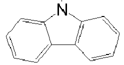 |
| 46 | 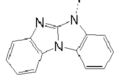 | 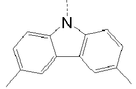 | H | 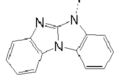 | 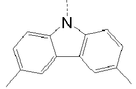 |
| 47 | 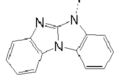 | 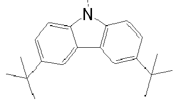 | H | 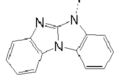 | 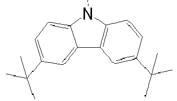 |
| 48 | 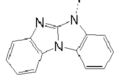 | 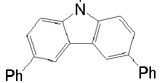 | H | 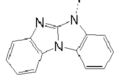 | 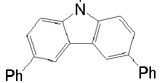 |
| 49 | 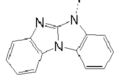 | 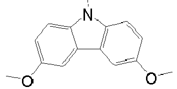 | H | 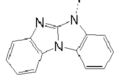 | 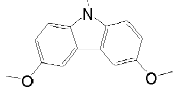 |

### Preparation of the compounds of formula (I)

The compounds of formula (I) can for example be prepared by reacting intermediates (IIa) or (IIIa), (Va) or (VIa) 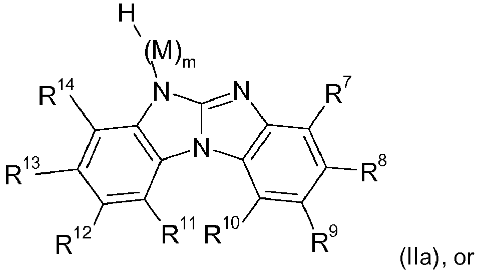 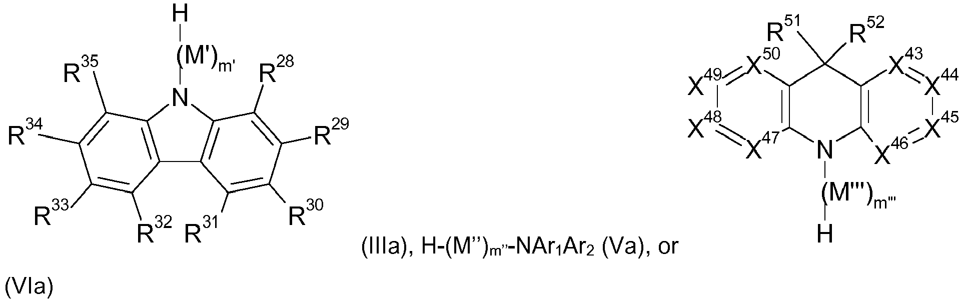 with a compound of formula 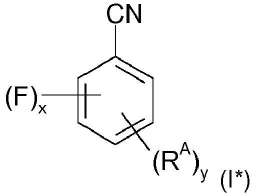 wherein
y is 0, 1 or 2, and
x is 2, 3, 4 or 5,
wherein the sum of the x and y is 5,
in the presence of a base, preferably K₃PO₄, K₂CO₃, Cs₂CO₃, NaOtBu or NaH,
wherein
the groups and residues R⁷ to R¹⁴, R^{A}, M, m, D, E, R¹⁵ to R²⁵ and R²⁷, R²⁸ to R³⁵ and R³⁶ to R³⁹, R^{17'} R^{18'}, R^{19'}, M' m', Ar₁, Ar₂, M", m"

X⁴³ to X⁵⁰, R⁴³ to R⁵⁰, M"', m"', R⁵¹ and R⁵² are defined above.

The reaction may be carried out in a solvent. Suitable solvents are dimethylsulfoxide (DMSO), dimethylformamide (DMF), dimethylacetamide (DMA) and N-methyl-2-pyrrolidone (NMP).

Suitable reaction conditions are described in the examples.

### Device

TADF (thermally activated delayed fluorescence) generally involves an emitter system of emitter and host and optionally a third material. The compounds of formula (I) are useful for organic electroluminescence devices (organic EL devices), preferably as TADF host or as TADF emitter in a TADF emitter system. In case of a TADF emitter, one or more host materials, usually one host material, is used and the TADF emitter emits light. In case of a TADF host, the TADF host material transfers excitons to one or more, usually one, fluorescence emitter. Preferably, a third material is used for diluting the TADF host material.

The present invention therefore relates to an organic electroluminescence device comprising at least one compound of formula (I) according to the present application.

The compound of formula (I) is capable of prolonging a lifetime, improving a luminous efficiency, and/or inhibiting a roll-off when driven at a high current density of organic electroluminescence devices comprising the same.

Usually, the inventive organic electroluminescence device comprises a cathode, an anode, and a plurality of organic layers provided between the cathode and the anode, wherein at least one of the plurality of organic layers is an emitting layer, wherein at least one organic layer comprises at least one compound of formula (I) according to the present application.

Preferably, the emitting layer of the organic electroluminescence device comprises the at least one compound of formula (I) as a delayed-fluorescent emitter (TADF emitter) and/or as a host material (TADF host).

In addition to the emitting layer, the inventive organic electroluminescence device may further comprise a hole transporting layer between the anode and the emitting layer and/or an electron transporting layer between the cathode and the emitting layer. Examples of organic EL devices comprising additional layers are described below.

The compound of formula (I) is preferably used in the organic electroluminescence device, more preferably in the emitting layer, as TADF emitter materials, or TADF host materials in combination with at least one fluorescent emitter.

A further subject of the present invention is directed to an emitting layer, comprising a compound of formula (I) according to the present invention. In said embodiment a compound of formula (I) is preferably used as TADF emitter material, or TADF host material in combination with at least one fluorescent emitter.

Arrangement(s) of an organic EL device of the invention will be described below.

The organic EL device includes an anode, a cathode and an organic layer disposed therebetween. The organic layer includes one or more layers formed of an organic compound. The organic layer may further contain an inorganic compound. The organic layer of the organic EL device of the exemplary embodiment includes at least one emitting layer. For instance, the organic layer may consist of the emitting layer, or may include one of layers usable in a typical organic EL device, such as a hole injecting layer, a hole transporting layer, an electron injecting layer, an electron transporting layer, a hole blocking layer and an electron blocking layer.

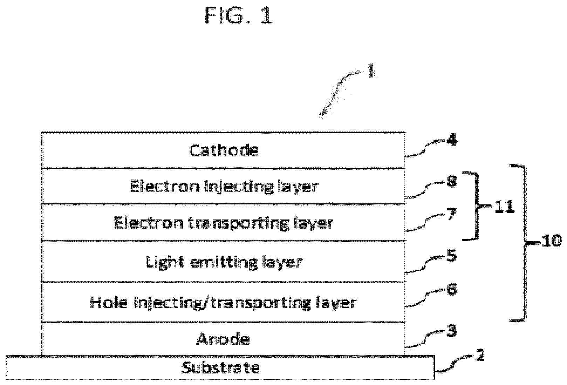

**FIG. 1** shows a schematic configuration of one example of the organic EL device of the invention. The organic EL device 1 comprises a substrate 2, an anode 3, a cathode 4 and an emitting unit 10 provided between the anode 3 and the cathode 4. The emitting unit 10 comprises an emitting layer 5 preferably comprising a host material and a dopant. A hole injecting and transporting layer 6 or the like may be provided between the emitting layer 5 and the anode 3 and an electroninjecting layer 8 and an electron transporting layer 7 or the like (electron injecting and transporting unit 11) may be provided between the emitting layer 5 and the cathode 4. An electron-barrier layer may be provided on the anode 3 side of the emitting layer 5 and a hole-barrier layer may be provided on the cathode 4 side of the emitting layer 5. Due to such configuration, electrons or holes can be confined in the emitting layer 5, whereby possibility of generation of excitons in the emitting layer 5 can be improved.

### Emitting Layer

The emitting layer of the organic EL device according to the present invention comprises a first compound and a second compound, wherein at least one of the first and the second compound is a compound of formula (I) according to the present invention. The first compound is a host compound and the second compound is an emitter compound.

In the following exemplary embodiments of suitable devices according to the present invention are disclosed. It should be noted that the invention is not limited to the embodiments mentioned below. The invention may include any modification and improvement compatible with the invention.

### First Embodiment

### First Compound

The first compound in the first embodiment is a delayed fluorescent compound (TADF host).

The first compound is represented by a compound of formula (I).

### Delayed Fluorescence

Delayed fluorescence (thermally activated delayed fluorescence) is explained in " ADACHI, Chi-haya, ed., "Yuki Hando-tai no Debaisu Bussei (Device Physics of Organic Semiconductors)", Ko-dansha, pp. 261-268 ." According to this literature, when an energy gap ΔE₁₃ (ΔE_{S1T1}) between the singlet state and the triplet state of a fluorescent material can be reduced, inverse energy transfer from the triplet state to the singlet state, which usually occurs at a low transition probability, occurs at a high transition probability to cause thermally activated delayed fluorescence (TADF). Further, Fig. 10.38 in this literature illustrates an occurrence mechanism of the delayed fluorescence. The first compound of the exemplary embodiment is a compound exhibiting thermally activated delayed fluorescence caused by this mechanism. Occurrence of delayed fluorescence emission can be determined by transient PL (Photo Luminescence) measurement.

The behavior of delayed fluorescence can be analyzed based on the decay curve obtained by the transient PL measurement. The transient PL measurement is a process where a sample is irradiated with a pulse laser to be excited, and a decay behavior (transient characteristics) of PL emission after the irradiation is stopped is measured. PL emission using a TADF material is divided into an emission component from singlet excitons generated by the first PL excitation and an emission component from singlet excitons generated via triplet excitons. The lifetime of the singlet excitons generated by the first PL excitation is in a nano-second order and considerably short. Emission from these singlet excitons thus decays immediately after the irradiation with the pulse laser.

In contrast, delayed fluorescence, which is emission from the singlet excitons generated via long-life triplet excitons, decays slowly. There is thus a large difference in time between emission from the singlet excitons generated by the first PL excitation and emission from the singlet excitons generated via triplet excitons. Therefore, a luminous intensity resulting from the delayed fluorescence can be obtained.

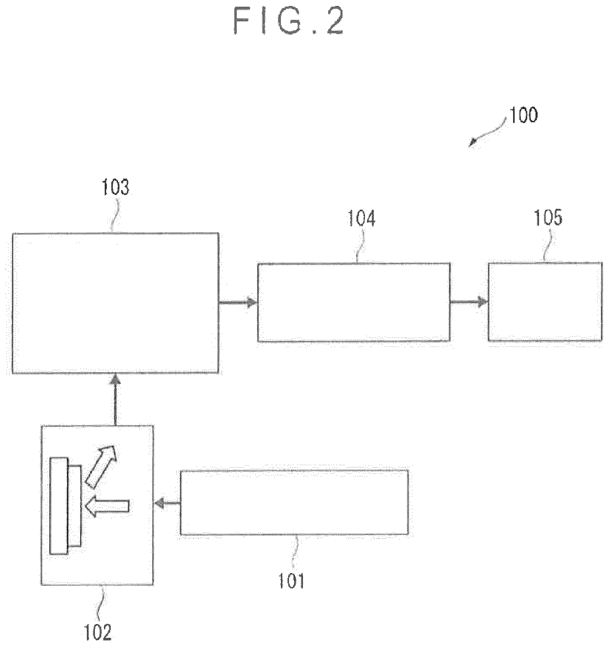

**Fig. 2** schematically shows an exemplary device for measuring transient PL.

A transient PL measuring device 100 of the exemplary embodiment includes: a pulse laser 101 capable of emitting light with a predetermined wavelength; a sample chamber 102 configured to house a measurement sample; a spectrometer 103 configured to disperse light emitted from the measurement sample; a streak camera 104 configured to form a two-dimensional image; and a personal computer 105 configured to analyze the two-dimensional image imported thereinto. It should be noted that transient PL may be measured by a device different from one described in the exemplary embodiment.

The sample to be housed in the sample chamber 102 is prepared by forming a thin film, which is made of a matrix material doped with a doping material at a concentration of 12 mass%, on a quartz substrate.

The thus-obtained thin film sample is housed in the sample chamber 102, and is irradiated with a pulse laser emitted from the pulse laser 101 to excite the doping material. The emitted excitation light is taken in a 90-degree direction with respect to the irradiation direction of the excitation light, and is dispersed by the spectrometer 103. A two-dimensional image of the light is formed through the streak camera 104. In the thus-obtained two-dimensional image, an ordinate axis corresponds to time, an abscissa axis corresponds to wavelength, and a bright spot corresponds to luminous intensity. The two-dimensional image is taken at a predetermined time axis, thereby obtaining an emission spectrum with an ordinate axis representing luminous intensity and an abscissa axis representing wavelength. Further, the two-dimensional image is taken at a wavelength axis, thereby obtaining a decay curve (transient PL) with an ordinate axis representing the logarithm of luminous intensity and an abscissa axis representing time.

For instance, a thin film sample A was prepared using a reference compound M1 below as a matrix material and a reference compound DP1 below as a doping material, and transient PL was measured.

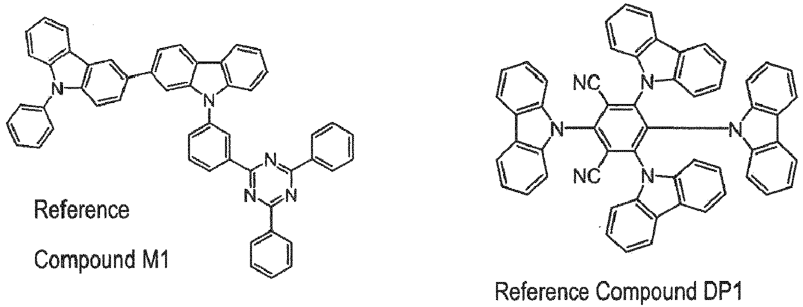

Respective decay curves of the thin film sample A and a thin film sample B were analyzed. The thin film sample B was prepared in the same manner as described above using a reference compound M2 below as a matrix material and the reference compound DP1 as a doping material.

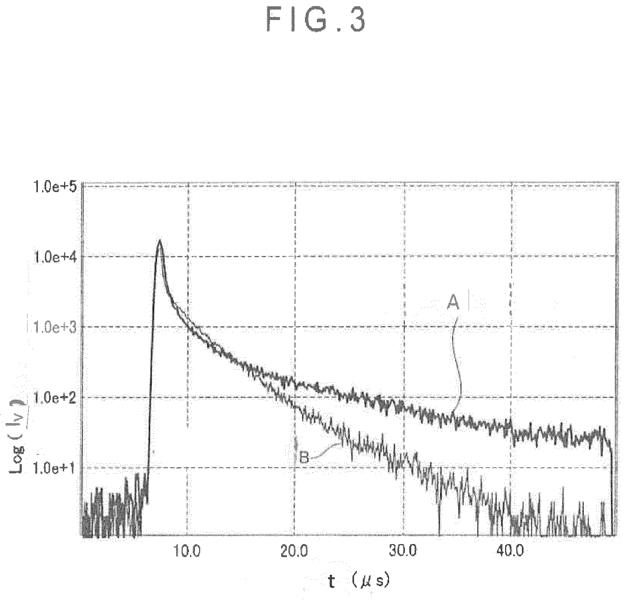

**Fig. 3** shows a decay curve obtained from transient PL measured using each of the thin film samples A and B.

### In Fig. 3:

A is thin film sample A
B is thin film sample B
t is the time in (µs)
I_{V} is the luminous intensity in (cd) (logarithmic scale)

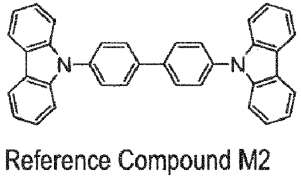

As described above, an emission decay curve with an ordinate axis representing luminous intensity and an abscissa axis representing time can be obtained by the transient PL measurement. Based on the emission decay curve, a fluorescence intensity ratio between fluorescence emitted from a singlet state generated by photo-excitation and delayed fluorescence emitted from a singlet state generated by inverse energy transfer via a triplet state can be estimated. In a delayed fluorescent material, a ratio of the intensity of the slowly decaying delayed fluorescence to the intensity of the promptly decaying fluorescence is relatively large.

In the first embodiment, the luminescence amount of the delayed fluorescence can be obtained using the device shown in **Fig. 2****.** Emission from the first compound includes: Prompt emission observed immediately when the excited state is achieved by exciting the first compound with a pulse beam (i.e., a beam emitted from a pulse laser) having an absorbable wavelength; and Delay emission observed not immediately when but after the excited state is achieved. In the exemplary embodiment, the amount of Delay emission is preferably 5% or more relative to the amount of Prompt emission.

The amount of Prompt emission and the amount of Delay emission can be obtained in the same method as a method described in "Nature 492, 234-238, 2012". The amount of Prompt emission and the amount of Delay emission may be calculated using a device different from one described in the above Literature.

For instance, a sample usable for measuring the delayed fluorescence may be prepared by codepositing the first compound and a compound TH-2 below on a quartz substrate at a ratio of the first compound being 12 mass% to form a 100-nm-thick thin film.

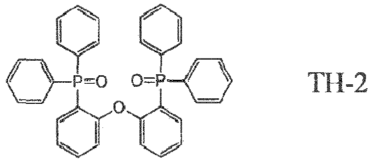

### Second Compound

The second compound in the first embodiment is a fluorescent compound (emitter).

The second compound preferably emits fluorescence having a main peak wavelength (occasionally referred to as an emission peak wavelength) of 430 to 780 nm (blue, green, red). The main peak wavelength means a peak wavelength of luminescence spectrum exhibiting a maximum luminous intensity among luminous spectra measured using a toluene solution where the second compound is dissolved at a concentration from 10⁻⁶ mol/l to 10⁻⁵ mol/l.

The second compound preferably emits a red, green or blue fluorescence. The second compound is preferably a material exhibiting a high emission quantum efficiency.

Suitable fluorescent emitters emitting a red, green or blue fluorescence are known to a person skilled in the art.

Examples of the fluorescent emitter include a bisarylamino naphthalene derivative, an aryl-substituted naphthalene derivative, a bisarylamino anthracene derivative, an aryl-substituted anthracene derivative, a bisarylamino pyrene derivative, an aryl-substituted pyrene derivative, a bisarylamino chrysene derivative, an aryl-substituted chrysene derivative, a bisarylamino fluoranthene derivative, an aryl-substituted fluoranthene derivative, an indenoperylene derivative, a pyrromethene boron complex compound, a compound having a pyrromethene skeleton or a metal complex thereof, a diketopyrrolopyrrole derivative, and a perylene derivative.

Further suitable fluorescence emitters are for example described in US 2016/0172601 A1, especially the compounds of formula (2) and preferred compounds described in US 2016/0172601 A1

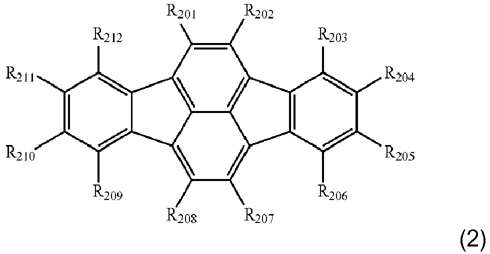

In the formula (2), R₂₀₁ to R₂₁₂ are each independently a hydrogen atom or a substituent.

When R₂₀₁ to R₂₁₂ are a substituent, the substituent is selected from the group consisting of a substituted or unsubstituted alkyl group having 1 to 30 carbon atoms, a substituted or unsubstituted aryl group having 6 to 30 ring carbon atoms, a substituted or unsubstituted heterocyclic group having 5 to 30 ring atoms, a substituted or unsubstituted alkoxy group having 1 to 30 carbon atoms, a substituted or unsubstituted aryloxy group having 6 to 30 ring carbon atoms, a substituted or unsubstituted aralkyl group having 6 to 30 ring carbon atoms, a substituted amino group, a cyano group and a halogen atom. The substituents of R₂₀₁ to R₂₁₂ may be mutually bonded to form a cyclic structure.

Further suitable fluorescence emitters are for example described in US 2016/0190469 A1, especially the compounds of formula (2) and preferred compounds described in US 2016/0190469 A1

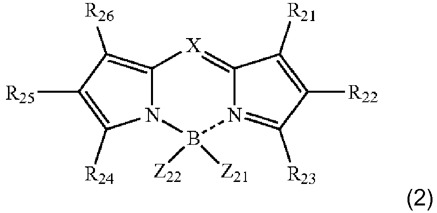

In the formula (2): X is a nitrogen atom or a carbon atom to be bonded to Y; Y is a hydrogen atom or a substituent; R₂₁ to R₂₆ are each independently a hydrogen atom or a substituent; when Y and R₂₁ to R₂₆ are substituents, the substituents are selected from the group consisting of a substituted or unsubstituted alkyl group, a substituted or unsubstituted cycloalkyl group, a substituted or unsubstituted aromatic hydrocarbon group, a substituted or unsubstituted alkoxy group, a substituted or unsubstituted alkylthio group, a substituted or unsubstituted aryloxy group, a substituted or unsubstituted arylthio group, a substituted or unsubstituted alkenyl group, a substituted or unsubstituted aralkyl group, a substituted or unsubstituted heterocyclic group, a halogen atom, a haloalkyl group, a carboxy group, an ester group, a carbamoyl group, an amino group, a nitro group, a cyano group, a silyl group, and a siloxanyl group; Z₂₁ and Z₂₂ are each independently selected from the group consisting of a halogen atom, a substituted or unsubstituted aromatic hydrocarbon group, a substituted or unsubstituted alkoxy group, and a substituted or un substituted aryloxy group.

Two or more of R₂₁ to R₂₆ in the formula (2) may be bonded to each other to form a cyclic structure.

Further suitable fluorescence emitters are for example described in WO 2017/146397 and US 20170141322, especially the compounds of formula (4),

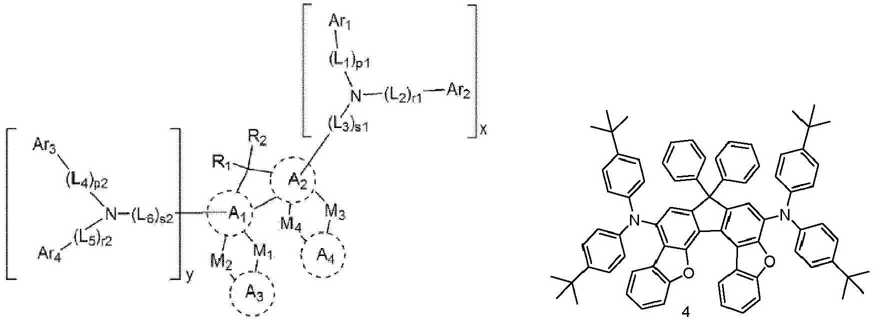

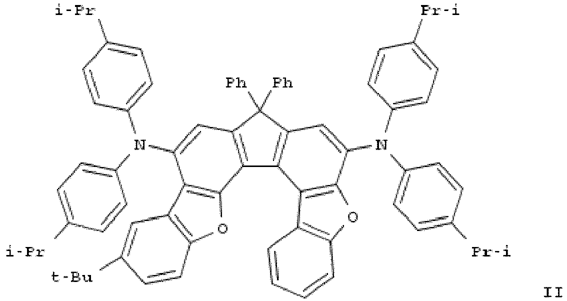

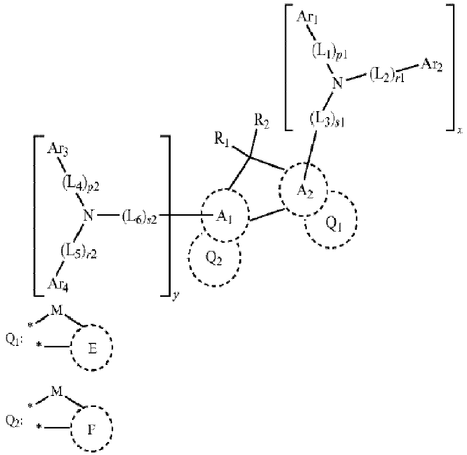

Further suitable fluorescence emitters are for example described in EP 3 109 253 A1, Advanced Materials (Weinheim, Germany), 28(14), 2777-2781; 2016, especially the compounds of formula (3)

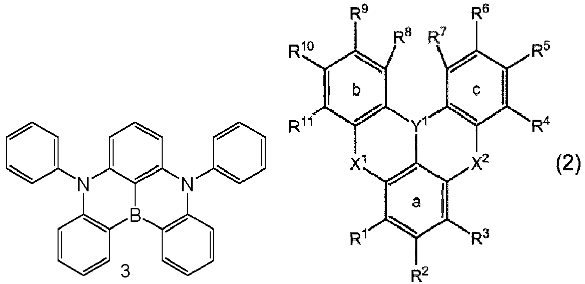

### Combination of first compound and second compound

The organic EL device in the first embodiment is capable of prolonging a lifetime, improving a luminous efficiency, and inhibiting a roll-off when driven at a high current density. The roll-off refers to a phenomenon in which a luminous efficiency is decreased when the organic EL device is driven at a high current density.

The first compound contained in the emitting layer in the exemplary embodiment is a delayed fluorescent compound, a compound of formula (I). Since the delayed fluorescent compound has a donor (electron-donating) moiety and an acceptor (electron accepting) moiety in a molecular structure, electric charges exist in a localized state in a molecule of the delayed fluorescent compound when the delayed fluorescent compound is excited. Since such a localized state of the electric charges possibly causes various side reactions, the delayed fluorescent compound having a long excited lifetime tends to exhibit a low stability especially in the excited state. Moreover, a half bandwidth of the luminescence spectrum of the delayed fluorescent compound is increased. Accordingly, it is difficult to use the delayed fluorescent compound as a luminescent material for a display unit requiring a high definition display in a large-sized TV set and the like.

Accordingly, an organic EL device including an emitting layer containing a delayed fluorescent compound and a fluorescent compound has been studied. In the organic EL device with the above arrangement, the delayed fluorescent compound is used as a sensitizer in the emitting layer and the fluorescent compound is used as the luminescent material. According to the organic EL device with the above arrangement, since the fluorescent compound emits light, it is expected that the half bandwidth of the luminescence spectrum is decreased. Moreover, since emission from the delayed fluorescent compound having a low excitation stability can be avoided, it is expected that a lifetime of the organic EL device is prolonged. Further, according to the organic EL device with this arrangement, it is theoretically expected that the same luminous efficiency as that of an organic EL device including an emitting layer containing a delayed fluorescent compound as a luminescent material is obtained.

The inventors have found that a combination of the delayed fluorescent compound that is the first compound and the fluorescent compound that is the second compound, wherein the first compound (delayed fluorescent compound (TADF host)) is a compound of formula (I) according to the present invention, can provide advantages of prolonging a lifetime, improving a luminous efficiency, and inhibiting a roll-off in a high current density region.

The organic EL device in the first embodiment preferably emits light exhibiting a peak in a wavelength range of 430 to 780 nm. In other words, the main peak wavelength of the light emitted from the organic EL device preferably falls within a range of 430 to 780 nm. When the organic EL device in the exemplary embodiment emits light, it is preferable that the second compound mainly emits light in the emitting layer (5). The wavelength range of the light emitted by the organic EL device in the first embodiment is more preferably from 430 nm to 570 nm.

It is therefore preferable that the organic EL device in the first embodiment emits blue or green fluorescence.

### TADF Mechanism

In the organic EL device of the first embodiment, the first compound is preferably a compound having a small ΔST(M1) so that inverse intersystem crossing from the triplet energy level of the first compound to the singlet energy level thereof is easily caused by a heat energy given from the outside. An energy state conversion mechanism to perform spin exchange from the triplet state of electrically excited excitons within the organic EL device to the singlet state by inverse intersystem crossing is referred to as a TADF mechanism.

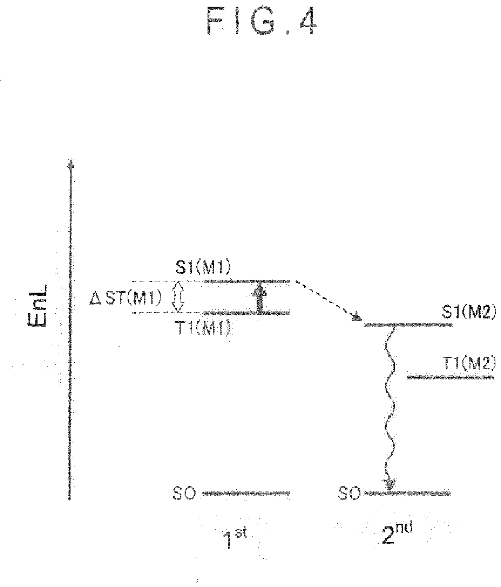

**Fig. 4** exemplarily shows a relationship in energy levels (EnL in Fig. 4 is the energy level) between the first compound (1^{st}) and the second compound (2^{nd}) in the emitting layer. In Fig. 4, S0 represents a ground state, S1(M1) represents a lowest singlet state of the first compound, T1 (M1) represents a lowest triplet state of the first compound, S1 (M2) represents a lowest singlet state of the second compound, and T1(M2) represents a lowest triplet state of the second compound. A dashed arrow directed from S1 (M1) to S1 (M2) in Fig. 4 represents Forster energy transfer from the lowest singlet state of the first compound to the lowest singlet state of the second compound. A difference between the lowest singlet state S1 and the lowest triplet state T1 is defined as ΔST.

As shown in Fig. 4, when a compound having a small ΔST(M1) is used as the first compound, inverse intersystem crossing from the lowest triplet state T1(M1) to the lowest singlet state S1(M1) can be caused by a heat energy. Accordingly, Forster energy transfer from the lowest singlet state S1(M1) of the first compound to the lowest singlet state S1(M2) of the second compound is caused. As a result, fluorescence from the lowest singlet state S1(M2) of the second compound can be observed. It is considered that the internal quantum efficiency can be theoretically raised up to 100% also by using delayed fluorescence by the TADF mechanism.

In the first embodiment, a singlet energy S(M1) of the first compound is preferably larger than a singlet energy S(M2) of the second compound.

In the first embodiment, an energy gap T77K(M1) at 77[K] of the first compound is preferably larger than an energy gap T77K(M2) at 77[K] of the second compound. T77K(M1) is preferably 2.0 eV or more, more preferably 2.2 eV or more.

### Relationship between Triplet Energy and Energy Gap at 77 [K]

Description will be made on a relationship between a triplet energy and an energy gap at 77 [K]. In the exemplary embodiment, the energy gap at 77 [K] is different from a typical triplet energy in some aspects.

The triplet energy is measured as follows. Firstly, a target compound for measurement is deposited on a quartz substrate to prepare a sample. Alternatively, the target compound is dissolved in an appropriate solvent to prepare a solution and the solution is encapsulated in a quartz glass pipe to prepare a sample. A phosphorescent spectrum (ordinate axis: phosphorescent luminous intensity, abscissa axis: wavelength) of the sample is measured at a low temperature (77K). A tangent is drawn to the rise of the phosphorescent spectrum on the short-wavelength side. The triplet energy is calculated by a predetermined conversion equation based on a wavelength value at an intersection of the tangent and the abscissa axis.

The first compound usable in the exemplary embodiment is preferably a compound having a small ΔST. When ΔST is small, intersystem crossing and inverse intersystem crossing are likely to occur even at a low temperature (77K), so that the singlet state and the triplet state coexist. As a result, the spectrum to be measured in the same manner as the above includes emission from both the singlet state and the triplet state. Although it is difficult to distinguish the emission from the singlet state from the emission from the triplet state, the value of the triplet energy is basically considered dominant.

Accordingly, in the first embodiment, the triplet energy is measured by the same method as a typical triplet energy T, but a value measured in the following manner is referred to as an energy gap T_{77K} in order to differentiate the measured energy from the typical triplet energy in a strict meaning. In the measurement using a thin film, the target compound for the measurement is deposited at a film thickness of 100 nm on a quartz substrate to form a sample. A phosphorescent spectrum (ordinate axis: phosphorescent luminous intensity, abscissa axis: wavelength) of the sample is measured at a low temperature (77K). A tangent is drawn to the rise of the phosphorescent spectrum on the short-wavelength side. An energy amount is calculated by the following conversion equation 1 based on a wavelength value λ_{edge}[nm] at an intersection of the tangent and the abscissa axis and defined as an energy gap T_{77K}. ${T}_{77 K} \left[eV\right] = 1239.85 / {λ}_{dge}$

The tangent to the rise of the phosphorescence spectrum on the short-wavelength side is drawn as follows. While moving on a curve of the phosphorescence spectrum from the short-wavelength side to the maximum spectral value closest to the short-wavelength side among the maximum spectral values, a tangent is checked at each point on the curve toward the long-wavelength of the phosphorescence spectrum. An inclination of the tangent is increased as the curve rises (i.e., a value of the ordinate axis is increased). A tangent drawn at a point of the maximum inclination (i.e., a tangent at an inflection point) was defined as the tangent to the rise of the phosphorescence spectrum on the short-wavelength side.

The maximum with peak intensity being 15% or less of the maximum peak intensity of the spectrum is not included in the above-mentioned maximum closest to the short-wavelength side of the spectrum. The tangent drawn at a point of the maximum spectral value being closest to the short-wavelength side and having the maximum inclination is defined as a tangent to the rise of the phosphorescence spectrum on the short-wavelength side.

For phosphorescence measurement, a spectrophotofluorometer body F-4500 (manufactured by Hitachi High-Technologies Corporation) is usable. The measurement instrument is not limited to this arrangement. A combination of a cooling unit, a low temperature container, an excitation light source and a light-receiving unit may be used for measurement.

### Singlet Energy S

The singlet energy S is measured as follows. 10 µmol/L of a toluene solution containing the target compound for the measurement was prepared and put in a quartz cell to prepare a sample. An absorption spectrum (ordinate axis: luminous intensity, abscissa axis: wavelength) of the sample was measured at a normal temperature (300K). A tangent was drawn to the fall of the absorption spectrum on the long-wavelength side, and a wavelength value λ_{edge}[nm] at an intersection of the tangent and the abscissa axis was substituted in the following conversion equation to calculate a singlet energy. $S \left[eV\right] = 1239.85 / {λ}_{edge}$

In Example, the absorption spectrum was measured using a spectrophotometer manufactured by Hitachi, Ltd. (device name: U3310). It should be noted that the absorption spectrum measuring device may be different from the above device.

The tangent to the fall of the absorption spectrum on the long-wavelength side is drawn as follows. While moving on a curve of the absorption spectrum from the maximum spectral value closest to the long-wavelength side in a long-wavelength direction, a tangent at each point on the curve is checked. An inclination of the tangent is decreased and increased in a repeated manner as the curve falls (i.e., a value of the ordinate axis is decreased). A tangent drawn at a point of the minimum inclination closest to the long-wavelength side (except when absorbance is 0.1 or less) is defined as the tangent to the fall of the absorption spectrum on the long-wavelength side.

The maximum absorbance of 0.2 or less is not included in the above-mentioned maximum absorbance on the long-wavelength side.

### Film Thickness of Emitting Layer

A film thickness of the emitting layer (5) of the organic EL device (1) of the exemplary embodiment is preferably in a range from 5 nm to 100 nm, more preferably in a range from 7 nm to 100 nm, and most preferably in a range from 10 nm to 100 nm. The thickness of less than 5 nm may cause difficulty in forming the emitting layer (5) and in controlling chromaticity, while the thickness of more than 100 nm may raise drive voltage.

### Content Ratio of Compounds in Emitting Layer

In the emitting layer (5) of the organic EL device (1) according to the first embodiment, a content ratio of the first compound is preferably 5 mass% or more, more preferably in a range from 10 mass% to 80 mass%, further preferably in a range from 40 mass% to 60 mass%. A content ratio of the second compound is preferably in a range from 1 mass% to 10 mass%. An upper limit of the total of the respective content ratios of the first and second compounds in the emitting layer 5 is 100 mass%. It should be noted that the emitting layer 5 of the exemplary embodiment may further contain another material in addition to the first and second compounds.

### Substrate

A substrate (2) is used as a support for the organic EL device (1). For instance, glass, quartz, plastics and the like are usable as the substrate (2). A flexible substrate is also usable. The flexible substrate is a bendable substrate. The flexible substrate is exemplified by a plastic substrate formed of polycarbonate, polyarylate, polyethersulfone, polypropylene, polyester, polyvinyl fluoride, polyvinyl chloride, polyimide, polyethylene naphthalate or the like. Moreover, an inorganic vapor deposition film is also usable as the substrate (2).

### Anode

Metal, alloy, an electrically conductive compound, a mixture thereof and the like, which have a large work function (specifically, of 4.0 eV or more) is preferably usable as the anode (3) formed on the substrate (2). Specific examples of the material for the anode include indium tin oxide (ITO), indium tin oxide containing silicon or silicon oxide, indium zinc oxide, tungsten oxide, indium oxide containing zinc oxide and graphene. In addition, gold (Au), platinum (Pt), nickel (Ni), tungsten (W), chrome (Cr), molybdenum (Mo), iron (Fe), cobalt (Co), copper (Cu), palladium (Pd), titanium (Ti), or nitrides of a metal material (e.g., titanium nitride) are usable.

The above materials are typically deposited as a film by sputtering. For instance, indium zinc oxide can be deposited as a film by sputtering using a target that is obtained by adding zinc oxide in a range from 1 mass% to 10 mass% to indium oxide. Moreover, for instance, indium oxide containing tungsten oxide and zinc oxide can be deposited as a film by sputtering using a target that is obtained by adding tungsten oxide in a range from 0.5 mass% to 5 mass% and zinc oxide in a range from 0.1 mass% to 1 mass% to indium oxide. In addition, vapor deposition, coating, ink jet printing, spin coating and the like may be used for forming the anode (3).

Among the organic layers formed on the anode (3), a hole injecting layer (6) formed adjacent to the anode (3) is formed of a composite material that facilitates injection of holes irrespective of the work function of the anode (3). Accordingly, a material usable as an electrode material (e.g., metal, alloy, an electrically conductive compound, a mixture thereof, and elements belonging to Groups 1 and 2 of the periodic table of the elements) is usable as the material for the anode (3).

The elements belonging to Groups 1 and 2 of the periodic table of the elements, which are materials having a small work function, namely, an alkali metal such as lithium (Li) and cesium (Cs) and an alkaline earth metal such as magnesium (Mg), calcium (Ca) and strontium (Sr), alloy thereof (e.g., MgAg, AILi), a rare earth metal such as europium (Eu) and ytterbium (Yb), and alloy thereof are also usable as the material for the anode. When the anode (3) is formed of the alkali metal, alkaline earth metal and alloy thereof, vapor deposition and sputtering are usable. Further, when the anode (3) is formed of silver paste and the like, coating, ink jet printing and the like are usable.

### Hole Injecting Layer

A hole injecting layer (6) is a layer containing a highly hole-injectable substance. Examples of the highly hole-injectable substance include molybdenum oxide, titanium oxide, vanadium oxide, rhenium oxide, ruthenium oxide, chromium oxide, zirconium oxide, hafnium oxide, tantalum oxide, silver oxide, tungsten oxide, and manganese oxide.

In addition, the examples of the highly hole-injectable substance further include: an aromatic amine compound, which is a low-molecule compound, such that 4,4',4"-tris(N,N-diphenylamino)triphenylamine (abbreviation: TDATA), 4,4',4"-tris[N-(3-methylphenyl)-N-phenylamino]triphenylamine (abbreviation: MTDATA), 4,4'-bis[N-(4-diphenylaminophenyl)-N-phenylamino]biphenyl(abbreviation: DPAB), 4,4'-bis(N-{4-[N'-(3-methylphenyl)-N'-phenylamino]phenyl}-N-phenylamino)biphenyl (abbreviation: DNTPD), 1,3,5-tris[N-(4-diphenylaminophenyl)-N-phenylamino]benzene (abbreviation: DPA3B), 3-[N-(9-phenylcarbazole-3-yl)-N-phenylamino]-9-phenylcarbazole (abbreviation: PCzPCA1), 3,6-bis[N-(9-phenylcarbazole-3-yl)-N-phenylamino]-9-phenylcarbazole (abbreviation: PCzPCA2), and 3-[N-(1-naphthyl)-N-(9-phenylcarbazole-3-yl)amino]-9-phenylcarbazole (abbreviation: PCzPCNI); and dipyrazino[2,3-f:20,30-h]quinoxaline-2,3,6,7,10,11-hexacarbonitrile (HAT-CN).

Moreover, a high-molecule compound (e.g., an oligomer, dendrimer and polymer) is also usable as the highly hole-injectable substance. Examples of the high-molecule compound include poly(N-vinylcarbazole) (abbreviation: PVK), poly(4-vinyltriphenylamine) (abbreviation: PVTPA), poly[N-(4-{N'-[4-(4-diphenylamino)phenyl]phenyl-N' -phenylamino}phenyl)methacrylamido] (abbreviation: PTPDMA), and poly[N,N'-bis(4-butylphenyl)-N,N'-bis(phenyl)benzidine] (abbreviation: Poly-TPD). Furthermore, the examples of the high-molecule compound include a high-molecule compound added with an acid such as poly(3,4-ethylene dioxythiophene)/poly(styrene sulfonic acid) (PEDOT/PSS), and polyaniline/poly(styrene sulfonic acid) (PAni/PSS).

### Hole Transporting Layer

A hole transporting layer (7) is a layer containing a highly hole-transportable substance. An aromatic amine compound, carbazole derivative, anthracene derivative and the like are usable for the hole transporting layer (7). Specific examples of a material for the hole transporting layer 7 include 4,4'-bis[N-(1-naphthyl)-N-phenylamino]biphenyl (abbreviation: NPB), N,N'-bis(3-methylphenyl)-N,N'-diphenyl-[1,1'-biphenyl]-4,4'-diamine (abbreviation: TPD), 4-phenyl-4'-(9-phenylfluorene-9-yl)triphenylamine (abbreviation: BAFLP), 4,4'-bis[N-(9,9-dimethylfluorene-2-yl)-N-phenylamino]biphenyl (abbreviation: DFLDPBi), 4,4',4"-tris(N,N-diphenylamino)triphenylamine (abbreviation: TDATA), 4,4',4"-tris[N-(3-methylphenyl)-N-phenylamino]triphenylamine (abbreviation: MTDATA), and 4,4'-bis[N-(spiro-9,9'-bifluorene-2-yl)-N-phenylamino]biphenyl (abbreviation: BSPB). The above-described substances mostly have a hole mobility of 10⁻⁶ cm² /(V•S) or more.

A carbazole derivative such as CBP, 9-[4-(N-carbazolyl)]phenyl-10-phenyl anthracene (CzPA) and 9-phenyl-3-[4-(10-phenyl-9-anthryl)phenyl]-9H-carbazole (PCzPA); an anthracene derivative such as t-BuDNA, DNA, and DPAnth; and a high-molecular weight compound such as poly(N-vinylcarbazole)(abbreviation: PVK) and poly(4-vinyltriphenylamine)(abbreviation: PVTPA) are usable for the hole transporting layer (7).

However, any substance having a hole transporting performance higher than an electron transporting performance may be used in addition to the above substances. A highly hole-transportable substance may be provided in the form of a single layer or a laminated layer of two or more layers of the above substance.

When the hole transporting layer includes two or more layers, one of the layers with a larger energy gap is preferably provided closer to the emitting layer (5).

In the first embodiment, the hole transporting layer (7) preferably has a function of preventing triplet excitons generated in the emitting layer (5) from diffusing into the hole transporting layer to trap the triplet excitons within the emitting layer (5).

### Electron Transporting Layer

An electron transporting layer (8) is a layer containing a highly electron-transportable substance. As the electron transporting layer, 1) a metal complex such as an aluminum complex, beryllium complex and zinc complex, 2) heteroaromatic compound such as an imidazole derivative, benzimidazole derivative, azine derivative, carbazole derivative, and phenanthroline derivative, and 3) a high-molecule compound are usable. Specifically, as a low-molecule organic compound, a metal complex such as Alq, tris(4-methyl-8-quinolinato)aluminum (abbreviation: Almq₃), bis(10-hydroxybenzo[h]quinolinato)beryllium (abbreviation: BeBq₂), BAlq, Znq, ZnPBO and ZnBTZ are usable. In addition to the metal complex, a heteroaromatic compound such as 2-(4-biphenyl)-5-(4-tert-butylphenyl)-1,3,4-oxadiazole (abbreviation: PBD), 1,3-bis[5-(ptert-butylphenyl)-1,3,4-oxadiazole-2-yl]benzene (abbreviation: OXD-7), 3-(4-tert-butylphenyl)-4-phenyl-5-(4-biphenyl)-1,2,4-triazole (abbreviation: TAZ), 3-(4-tert-butylphenyl)-4-(4-ethylphenyl)-5-(4-biphenyl)-1,2,4-triazole (abbreviation: p-EtTAZ), bathophenanthroline (abbreviation: BPhen), bathocuproine (abbreviation: BCP), and 4,4'-bis(5-methylbenzoxazole-2-yl)stilbene (abbreviation: BzOs) are usable. In the first embodiment, a benzimidazole compound is suitably usable. The above-described substances mostly have an electron mobility of 10⁻⁶ cm² /(V•s) or more. However, any substance having an electron transporting performance higher than a hole transporting performance may be used for the electron transporting layer (8) in addition to the above substances. The electron transporting layer (8) may be provided in the form of a single layer or a laminated layer of two or more layers of the above substance(s).

Moreover, a high-molecule compound is also usable for the electron transporting layer (8). For instance, poly[(9,9-dihexylfluorene-2,7-diyl)-co-(pyridine-3,5-diyl)] (abbreviation: PF-Py), poly[(9,9-dioctylfluorene-2,7-diyl)-co-(2,2'-bipyridine-6,6'-diyl)](abbreviation: PF-BPy) and the like are usable.

In the first embodiment, the hole transporting layer (8) preferably has a function of preventing triplet excitons generated in the emitting layer (5) from diffusing into the hole transporting layer (8) and the electron injecting layer (9) to trap the triplet excitons within the emitting layer (5).

### Electron Injecting Layer

An electron injecting layer (9) is a layer containing a highly electron-injectable substance. Examples of a material for the electron injecting layer include an alkali metal, alkaline earth metal and a compound thereof, examples of which include lithium (Li), cesium (Cs), calcium (Ca), lithium fluoride (LiF), cesium fluoride (CsF), calcium fluoride (CaF2), and lithium oxide (LiOx). In addition, a substance obtained by containing an alkali metal, alkaline earth metal or a compound thereof in the electron transportable substance, specifically, for instance, a substance obtained by containing magnesium (Mg) in Alq may be used. With this substance, electrons can be more efficiently injected from the cathode (4).

Alternatively, a composite material provided by mixing an organic compound with an electron donor may be used for the electron injecting layer (9). The composite material exhibits excellent electron injecting performance and electron transporting performance since the electron donor generates electron in the organic compound. In this arrangement, the organic compound is preferably a material exhibiting an excellent transforming performance of the generated electrons. Specifically, for instance, the above-described substance for the electron transporting layer (8) (e.g., the metal complex and heteroaromatic compound) is usable. The electron donor may be any substance exhibiting an electron donating performance to the organic compound. Specifically, an alkali metal, alkaline earth metal and a rare earth metal are preferable, examples of which include lithium, cesium, magnesium, calcium, erbium and ytterbium. Moreover, an alkali metal oxide and alkaline earth metal oxide are preferable, examples of which include lithium oxide, calcium oxide, and barium oxide. Further, Lewis base such as magnesium oxide is also usable. Furthermore, tetrathiafulvalene (abbreviation: TTF) is also usable.

### Cathode

Metal, alloy, an electrically conductive compound, a mixture thereof and the like, which have a small work function, specifically, of 3.8 eV or less, is preferably usable as a material for the cathode (4). Specific examples of the material for the cathode 4 include: the elements belonging to Groups 1 and 2 of the periodic table of the elements, namely, an alkali metal such as litium (Li) and cesium (Cs) and an alkaline earth metal such as magnesium (Mg), calcium (Ca) and strontium (Sr); alloy thereof (e.g., MgAg, AILi); a rare earth metal such as europium (Eu) and ytterbium (Yb); and alloy thereof.

When the cathode (4) is formed of the alkali metal, alkaline earth metal and alloy thereof, vapor deposition and sputtering are usable. Moreover, when the cathode (4) is formed of silver paste and the like, coating, ink jet printing and the like are usable.

By providing the electron injecting layer (9), various conductive materials such as Al, Ag, ITO, graphene and indium tin oxide containing silicon or silicon oxide are usable for forming the cathode (4) irrespective of the magnitude of the work function. The conductive materials can be deposited as a film by sputtering, ink jet printing, spin coating and the like.

### Layer Formation Method(s)

There is no restriction except for the above particular description for a method for forming each layer of the organic EL device (1) in the exemplary embodiment. Known methods such as dry film-forming and wet film-forming are applicable. Examples of the dry film-forming include vacuum deposition, sputtering, plasma and ion plating. Examples of the wet film-forming include spin coating, dipping, flow coating and ink-jet printing.

### Film Thickness

There is no restriction except for the above particular description for a film thickness of each of the organic layers of the organic EL device (1) in the exemplary embodiment. The film thickness is typically preferably in a range of several nanometers to 1 µm because an excessively-thinned film is likely to cause defects such as a pin hole while an excessively-thickened film requires high voltage to be applied and deteriorates efficiency.

### Electronic Device

The organic EL device (1) according to the first embodiment of the invention is usable in an electronic device such as a display unit and a light-emitting unit. Examples of the display unit include display components such as an organic EL panel module, TV, mobile phone, tablet, and personal computer. Examples of the light-emitting unit include an illuminator and a vehicle light.

The present invention therefore further relates to an electronic device comprising the organic electroluminescence device according to the present application and the use of the compound of formula (I) according to the present invention in an electronic device.

### Second Embodiment

Arrangement(s) of an organic EL device according to a second embodiment will be described below. In the description of the second embodiment, the same components as those in the first embodiment are denoted by the same reference signs and names to simplify or omit an explanation of the components. In the second embodiment, the same materials and compounds as described in the first embodiment are usable for a material and a compound which are not particularly described.

The organic EL device according to the second embodiment is different from the organic EL device according to the first embodiment in that the emitting layer comprises a first compound and a second compound, wherein the second compound is a delayed fluorescent compound (TADF emitter). The first compound is - in the second embodiment - not a delayed fluorescent compound.

### Second compound

The second compound in the third embodiment is represented by a compound of formula (I).

The inventors have found that a combination of the delayed fluorescent compound that is the second compound and a host that is the first compound, wherein the second compound is a delayed fluorescent compound (TADF emitter) is a compound of formula (I) according to the present invention, can provide advantages of prolonging a lifetime, improving a luminous efficiency, and inhibiting a roll-off in a high current density region.

The organic EL device in the second embodiment preferably emits light exhibiting a peak in a wavelength range of 430 to 780 nm. In other words, the main peak wavelength of the light emitted from the organic EL device preferably falls within a range of 430 to 780 nm. When the organic EL device in the exemplary embodiment emits light, it is preferable that the second compound mainly emits light in the emitting layer (5). The wavelength range of the light emitted by the organic EL device in the first embodiment is more preferably from 430 nm to 570 nm.

It is therefore preferable that the organic EL device in the first embodiment emits blue or green fluorescence.

### First compound

Host materials for TADF emitters are for example described in Chem. Soc. Rev 2017, 46, 915 (start page 974), Adv. Mater. 2017, 1605444, Volume 29, Issue 22, June 13, 2017 1605444 (Start page 39), J. Mater. Chem. C, 2016, 4, 11355-11381 (start page 11355), J. Mater. Chem. C, 2017,5, 8622-8653.

The first compound is a host compound, whereby suitable host compounds are known to a person skilled in the art.

Examples for suitable hosts are described in the following:

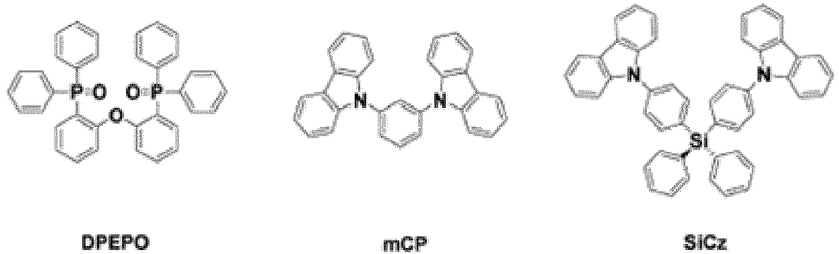

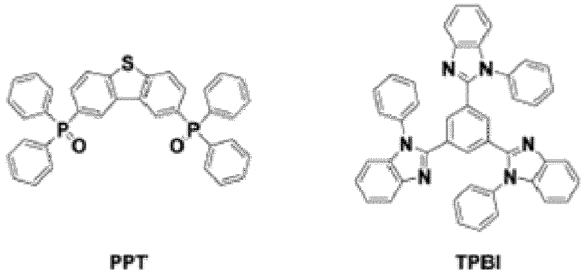

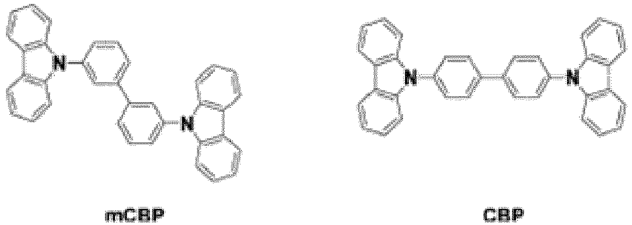

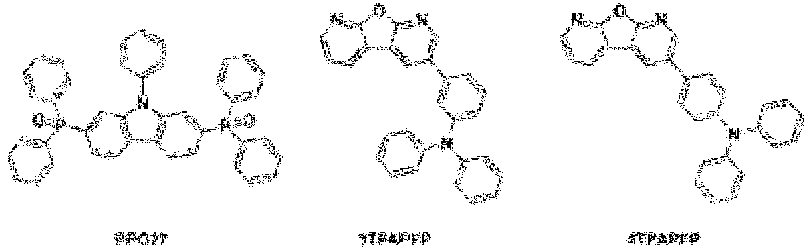

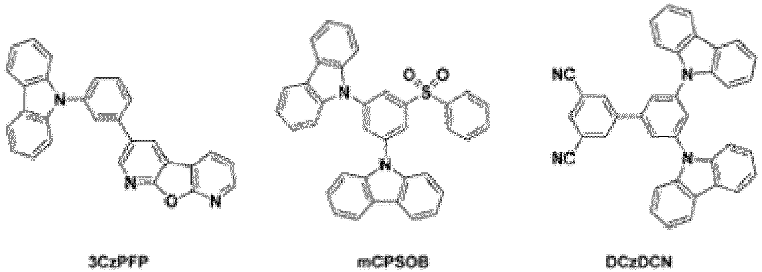

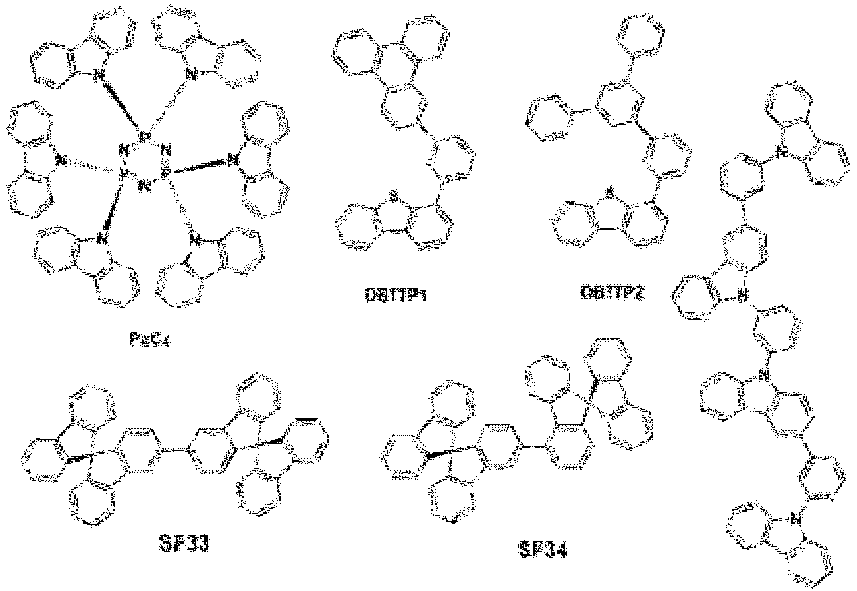

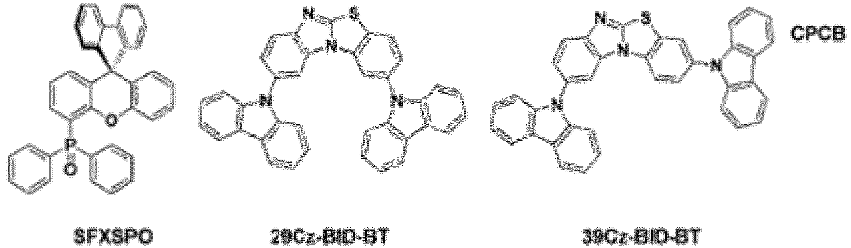

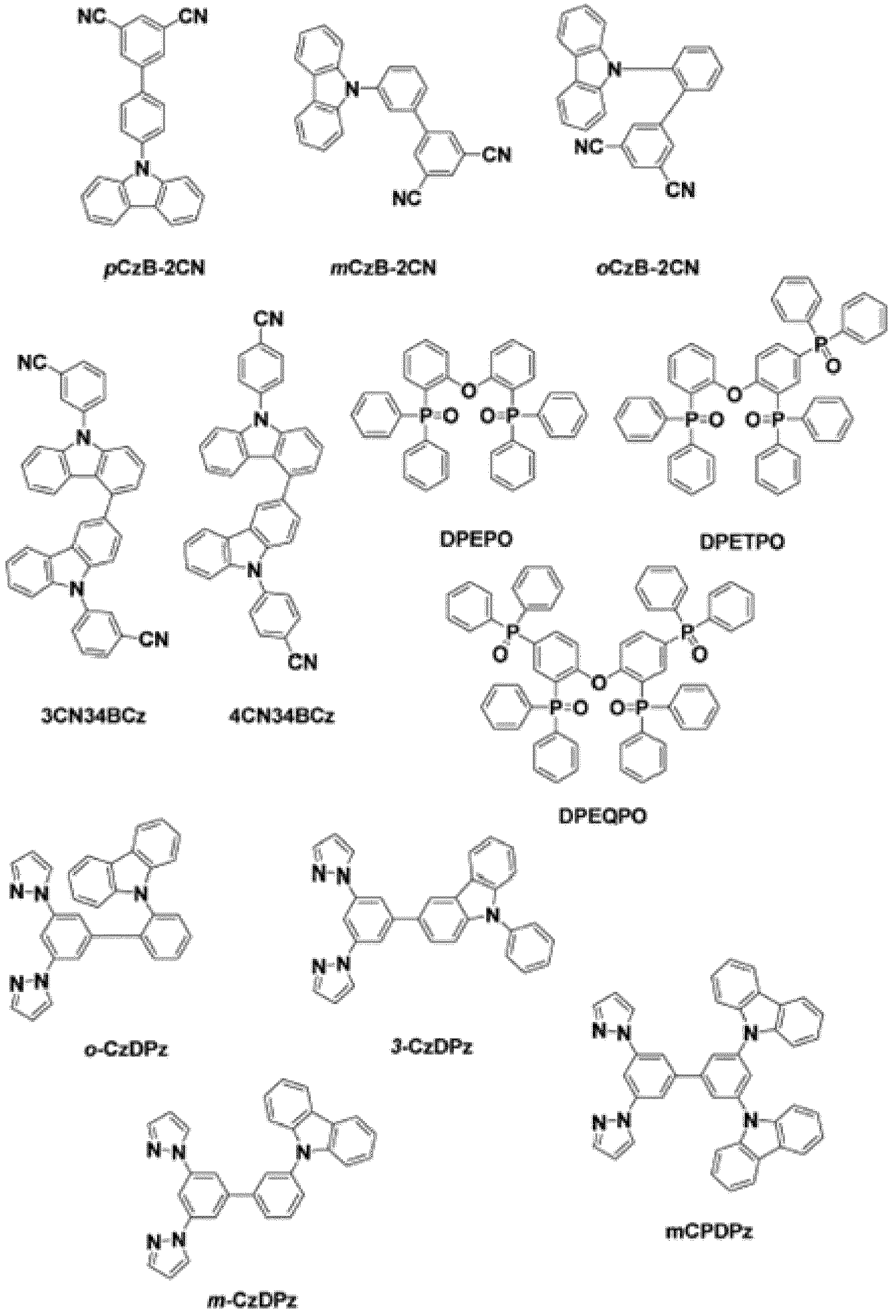

### Content Ratio of Compounds in Emitting Layer

In the emitting layer (5) of the organic EL device (1) according to the second embodiment, a content ratio of the first compound (host) is preferably 5 mass% or more, more preferably in a range from 10 mass% to 80 mass%, further preferably in a range from 40 mass% to 60 mass%. A content ratio of the second compound is preferably in a range from 1 mass% to 50 mass%. An upper limit of the total of the respective content ratios of the first and second compounds in the emitting layer (5) is 100 mass%. It should be noted that the emitting layer (5) of the exemplary embodiment may further contain another material in addition to the first and second compounds.

### Third Embodiment

Arrangement(s) of an organic EL device according to a third embodiment will be described below. In the description of the third embodiment, the same components as those in the first embodiment are denoted by the same reference signs and names to simplify or omit an explanation of the components. In the third embodiment, the same materials and compounds as described in the first embodiment are usable for a material and a compound which are not particularly described.

The organic EL device according to the third embodiment is different from the organic EL device according to the first embodiment in that the emitting layer further includes a third compound. Other components are the same as those in the first embodiment. As in the first embodiment, at least one compound of formula (I) is present as first (TADF host) compound.

### Third Compound

The singlet energy of the third compound is larger than the singlet energy of the first compound.

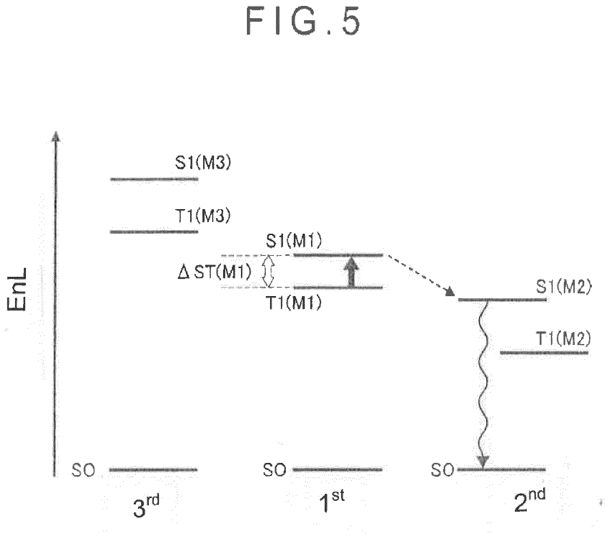

**Fig. 5** exemplarily shows a relationship in energy levels (EnL in Fig. 4 is the enery level) between the first compound (1^{st}), the second compound (2^{nd}) and the third compound (3^{rd}) in the emitting layer. In Fig. 5 , S0 represents a ground state, S1(M1) represents the lowest singlet state of the first compound, T1 (M1) represents the lowest triplet state of the first compound, S1(M2) represents the lowest singlet state of the second compound, T1(M2) represents the lowest triplet state of the second compound, S1(M3) represents a lowest singlet state of the third compound, and T1(M3) represents a lowest triplet state of the third compound. A dashed arrow directed from S1(M1) to S1(M2) in Fig. 5 represents Forster energy transfer from the lowest singlet state of the first compound to the lowest singlet state of the second compound.

As shown in Fig. 5, when a compound having a small ΔST(M1) is used as the first compound, inverse intersystem crossing from the lowest triplet state T1(M1) to the lowest singlet state S1(M1) can be caused by a heat energy. Accordingly, Forster energy transfer from the lowest singlet state S1(M1) of the first compound to the lowest singlet state S1(M2) of the second compound is caused. As a result, fluorescence from the lowest singlet state S1(M2) of the second compound can be observed. It is considered that the internal quantum efficiency can be theoretically raised up to 100% also by using delayed fluorescence by the TADF mechanism.

### Ratio of Three Components

In the emitting layer of the organic EL device of the third embodiment, it is preferable that the content ratio of the first compound is in a range from 10 mass% to 80 mass%, the content ratio of the second compound is in a range from 1 mass% to 10 mass%, and the content ratio of the third compound is in a range from 10 mass% to 80 mass%. The content ratio of the first compound is more preferably in a range from 20 mass% to 80 mass%, further preferably in a range from 40 mass% to 60 mass%. An upper limit of the total of the respective content ratios of the first to third compounds in the emitting layer is 100 mass%. It should be noted that the emitting layer of the exemplary embodiment may further contain another material in addition to the first to third compounds.

Although the third compound is not particularly limited, the third compound is preferably a compound other than an amine compound. For instance, carbazole derivatives, dibenzofuran derivatives, Si containing derivatives, phosphorous oxide derivatives and dibenzothiophene derivatives are usable as the third compound. However, the third compound is not limited thereto.

The third compound preferably has at least one of a partial structure represented by a formula (31) below and a partial structure represented by a formula (32) below in one molecule.

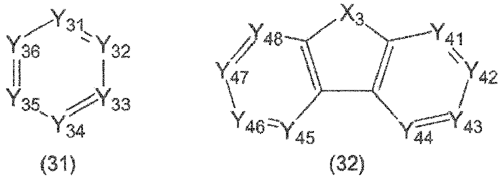

In the formula (31): Y₃₁ to Y₃₆ each independently represent a nitrogen atom or a carbon atom bonded to another atom in the molecule of the third compound.

However, at least one of Y₃₁ to Y₃₆ is a carbon atom bonded to another atom in the molecule of the third compound.

In the formula (32): Y₄₁ to Y₄₈ each independently represent a nitrogen atom or a carbon atom bonded to another atom in the molecule of the third compound.

However, at least one of Y₄₁ to Y₄₈ is a carbon atom bonded to another atom in the molecule of the third compound.

X₃ represents a nitrogen atom, an oxygen atom or a sulfur atom.

In the formula (32), it is also preferable that at least two of Y₄₁ to Y₄₈ are carbon atoms bonded to another atom in the molecule of the third compound and a ring structure is formed including the carbon atoms.

Specific partial structures represented by the formula (32) and by the formula (31) are described in EP 3 255 693 A1, especially in paragraphs [0189] to [0212].

The third compound is also preferably an aromatic hydrocarbon compound or an aromatic heterocyclic compound. It is also preferable that the third compound has no fused aromatic hydrocarbon ring in a molecule.

Examples of substituents of the third compound are mentioned in EP 3 255 693 A1, especially in paragraphs [0214] to [0226].

Further suitable third compounds are

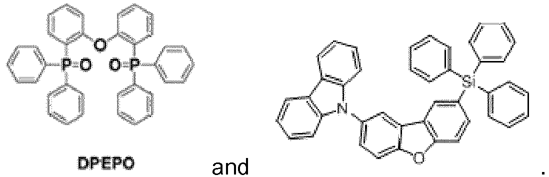

The third compound may be prepared by a method described in WO 2012/153780, WO 2013/038650 and EP 3 255 693.

The organic EL device in the third embodiment is capable of prolonging a lifetime, improving a luminous efficiency, and inhibiting a roll-off when driven at a high current density.

The emitting layer of the organic EL device according to the third embodiment includes the delayed fluorescent first compound, the fluorescent second compound, and the third compound having a larger singlet energy than the first compound, whereby the luminous efficiency of the organic EL device is further improved. It is deduced that the luminous efficiency is improved because a carrier balance in the emitting layer is improved by containing the third compound.

### Modification of Embodiments

It should be noted that the invention is not limited to the embodiments mentioned above. The invention may include any modification and improvement compatible with the invention.

For instance, the emitting layer is not limited to a single layer, but may be provided by laminating a plurality of emitting layers. When the organic EL device has the plurality of emitting layers, it is only required that at least one of the emitting layers contains the first and second compounds.

For instance, the rest of the emitting layers may be a fluorescent emitting layer or a phosphorescent emitting layer with use of emission caused by electron transfer from the triplet state directly to the ground state.

When the organic EL device includes the plurality of emitting layers, the plurality of emitting layers may be adjacent to each other, or provide a so-called tandem-type organic EL device in which a plurality of emitting units are layered through an intermediate layer.

For instance, a blocking layer may be provided in contact with an anode-side or a cathode-side of the emitting layer. The blocking layer is preferably provided in contact with the emitting layer to block at least ones of holes, electrons and excitons.

For instance, when the blocking layer is provided in contact with the cathode-side of the emitting layer, the blocking layer permits transport of electrons, but prevents holes from reaching a layer provided near the cathode (e.g., the electron transporting layer) beyond the blocking layer. When the organic EL device includes the electron transporting layer, the blocking layer is preferably interposed between the emitting layer and the electron transporting layer.

When the blocking layer is provided in contact with the anode-side of the emitting layer, the blocking layer permits transport of holes, but prevents electrons from reaching a layer provided near the anode (e.g., the hole transporting layer) beyond the blocking layer. When the organic EL device includes the hole transporting layer, the blocking layer is preferably interposed between the emitting layer and the hole transporting layer.

Further, the blocking layer may be provided in contact with the emitting layer to prevent an excitation energy from leaking from the emitting layer into a layer in the vicinity thereof. Excitons generated in the emitting layer are prevented from moving into a layer provided near the electrode (e.g., the electron transporting layer and the hole transporting layer) beyond the blocking layer.

The emitting layer and the blocking layer are preferably bonded to each other.

Further, the materials and treatments for practicing the invention may be altered to other arrangements and treatments as long as such other arrangements and treatments are compatible with the invention.

### Examples

Examples of the invention will be described below. However, the invention is not limited to Examples.

### Synthetic examples

### Synthetic example 1 (Compound 1)

### a) 2,6-di(9H-carbazol-9-yl)-3,5-difluorobenzonitrile (Intermediate 1.1)

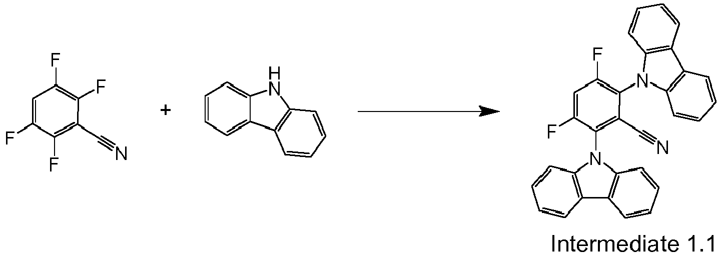

3.50 g (20.0 mmol) 2,3,5,6-tetrafluorobenzonitrile, 6.69 g (40 mmol) carbazol, 14.0 g (66.0 mmol) potassium phosphate tribasic in 40 ml NMP were stirred at 80 °C under nitrogen. After 18 h the reaction mixture was poured on water and the water phase was extracted with ethyl acetate. The organic phase was dried with magnesium sulfate and the solvent was removed in vacuum. Column chromatography on silica gel with toluene/ethyl acetate 70/30 gave the Intermediate 1.1. 3.20 g (34 %)
¹H NMR (400 MHz, CDCl₃): 8.21 (dt, 4H), 7.95 (dd, 1H), 7.53-7.59 (m, 4H), 7.40-7.46 (m, 6H), 7.33 (d, 2H).

### b) 3,5-bis(5H-benzo[d]benzo[4,5]imidazo[1,2-a]imidazol-5-yl)-2,6-di(9H-carbazol-9-yl)benzonitrile (Compound 1)

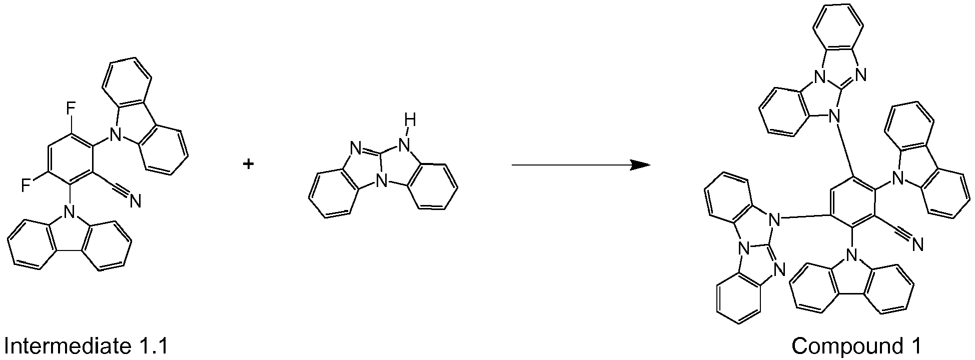

350 mg (0.750 mmol) 2,6-di(9H-carbazol-9-yl)-3,5-difluorobenzonitrile, 466 mg (2.25 mmol) 5H-benzo[d]benzo[4,5]imidazo[1,2-a]imidazole and 796 mg (3.75 mmol) potassium phosphate tribasic in 4 ml NMP were stirred under nitrogen at 80 °C. After 3 h the reaction mixture was poured on water and the water phase was extracted with dichloromethane. The organic phase was dried with magnesium sulfate and the solvent was removed in vacuum. Column chromatography on silica with toluene/ethyl acetate 95/5 gel gave the Compound 1. Yield 80 mg (13 %)
¹H NMR (400 MHz, DMSO d6): 9.28 (s, 1H), 8.05 (d, 1H), 7.85-8.01 (m, 9H), 7.70 (d, 1H), 7.61 (d, 1H), 7.45.7.55 (m, 5H), 7.40 (t, 1H), 7.02-7.32 (m, 12H), 6.89-7.96 (m, 2H).

### Photoluminescence Data

The PLQY was measured in the following manner

PLQY was measured in toluene solution at concentration of 10⁻⁴ M using FP-8300 JASCO Spectrofluorometer with 100mm φ integration sphere IFL-835.

Fluorescence and Phosphorescence spectra were measured using FP-8300 JASCO Spectrofluorometer using 10⁻⁶ M solution in toluene and methyl-THF respectively. ΔST was determined as difference between onsets of fluorescence and phosphorescence spectra.
τ _{d} is measured by transient photoluminescence (TR-PL) using Horiba DeltaFlex Modular Fluorescence Lifetime System.
The rate constant of reverse intersystem crossing (K_{RISC} /sec⁻¹) was calculated as described in the literature [1]
Before TR-PL and PLQY measurements, the solutions were deoxygenated with Argon to eliminated deactivation of triplets.
[1] - K. Masui et al. Org. Electron. 14, 2721-2726 (2013)

**Table 1:**

| Example | Solvent | PLQY | ΔST | τ_{d} |
|---|---|---|---|---|
| Compound 1 | toluene | 77% | 0.01 eV | 19 µs |
| Comparative Compound 1 | toluene | 76% | 0.19 eV | 43 µs |
| Comparative Compound 2 | toluene | no TADF effect | | |
| Comparative Compound 3 | toluene | no TADF effect | | |
| Comparative Compound 4 | toluene | no TADF effect | | |

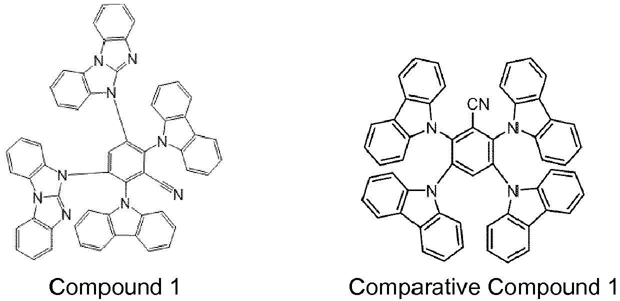

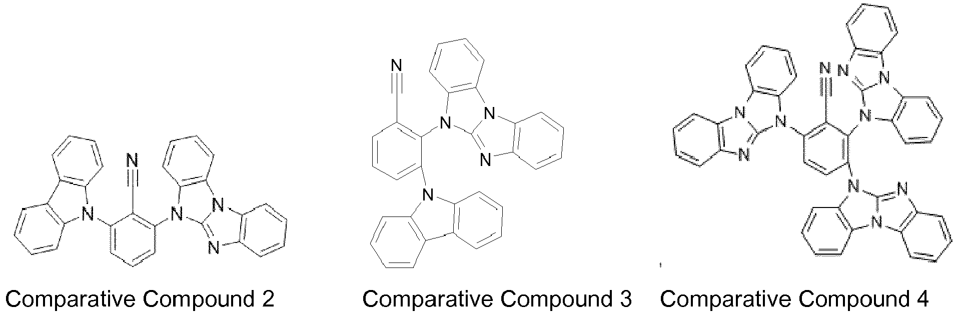

**Table 2**

| Example | Solvent | K_{RISC} /sec⁻¹ |
|---|---|---|
| Compound 1 | toluene | 2.01*10⁵ |
| Comparative Compound 1 | toluene | 8.76*10⁴ |

### Device Application Data (inventive compound as TADF emitter (dopant))

### Manufacture and Evaluation of Organic EL Device

The organic EL device was manufactured and evaluated as follows.

### Device Example 1

A glass substrate (size: 25 mm × 75 mm × 1.1 mm thick, manufactured by Geomatec Co., Ltd.) having an ITO transparent electrode (anode) was ultrasonic-cleaned in isopropyl alcohol for five minutes, and then UV/ozone-cleaned for 30 minutes. The thickness of the ITO film was set to be 130-nm.
After the glass substrate having the transparent electrode line was cleaned, the glass substrate was mounted on a substrate holder of a vacuum evaporation apparatus. Initially, a compound HI was vapor-deposited on a surface of the glass substrate where the transparent electrode line was provided in a manner to cover the transparent electrode, thereby forming a 5-nm-thick hole injecting layer.
Subsequently, the compound HT-1 was vapor-deposited on the hole injecting layer to form a 80-nm-thick first hole transporting layer on the HI film.
Next, the compound **HT-2** was vapor-deposited on the first hole transporting layer to form a 10-nm-thick second hole transporting layer.
Next, the compound **HT-3** was vapor-deposited on the second hole transporting layer to form a 5-nm-thick third hole transporting layer.

Further, on the third hole transporting layer, **compound 1** and the compound **BH** were codeposited to form a 25-nm-thick emitting layer.
The concentration of compound 1 in the emitting layer was set at 24 mass%.

Next, the compound **HB-1** was vapor-deposited on the emitting layer to form a 5-nm-thick blocking layer.
Subsequently, the compound **ET** was vapor-deposited on the blocking layer to form a 20-nm-thick electron transporting layer.
Next, lithium fluoride (LiF) was vapor-deposited on the electron transporting layer to form a 1-nm-thick electron injecting electrode (cathode).
A metal aluminum (Al) was then vapor-deposited on the electron injecting electrode to form an 80-nm-thick metal Al cathode.
The device arrangement of the organic EL device of Device example 1 is schematically shown as follows.
ITO(130) / HI(5) / HT-1(80) / HT-2(10) / HT-3(5) / BH : compound 1 (25, 24%) / HB-1(5) / ET(20) / LiF(1) / Al(80)
Numbers in parentheses represent a film thickness (unit: nm). The numbers in percentage in parentheses indicate a ratio (mass%) of the compound in the emitting layer.

### Comparative Device example 1

The organic EL device of comparative device example 1 was produced in the same manner as in Example 1 except that compound 1 in device example 1 was replaced by comparative compound 1.
The device arrangement of the organic EL device of comparative device example 1 is schematically shown as follows.
ITO(130) / HI(5) / HT-1(80) / HT-2(10) / HT-3(5) / BH : comparative compound (25, 24%) / HB-1(5) / ET(20) / LiF(1) / Al(80)

### Evaluation of Organic EL Devices

The manufactured organic EL devices of device example 1 and comparative device example 1 were evaluated as follows.

### Drive Voltage

Voltage was applied between the ITO transparent electrode and the metal Al cathode such that the current density was 10 mA/cm², and voltage (unit: V) was measured.

### CIE1931 Chromaticity

Voltage was applied on each of the organic EL devices such that the current density was 10 mA/cm², and CIE1931 chromaticity coordinates (x, y) were measured using a spectroradiometer CS-2000 (manufactured by Konica Minolta, Inc.).
Main Peak Wavelength λₚ(10 mA/cm²)

The *main peak wavelength* λₚ was calculated from the obtained spectral radiance spectra.

### External Quantum Efficiency EQE

Voltage was applied on each of the organic EL devices such that the current density was 0.10 mA/cm², or 10 mA/cm², and spectral radiance spectra were measured using the above spectroradiometer.

The external quantum efficiency EQE (unit: %) was calculated from the obtained spectral radiance spectra, assuming that the spectra were provided under a Lambertian radiation.

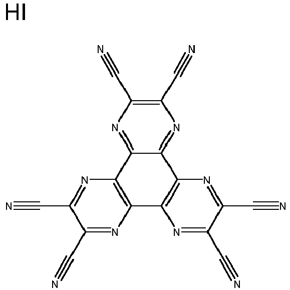

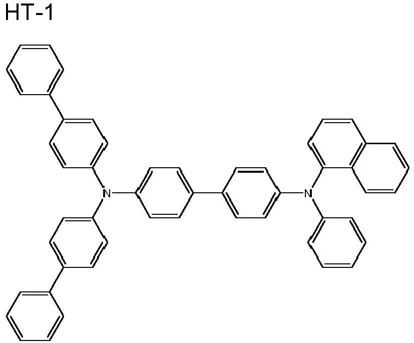

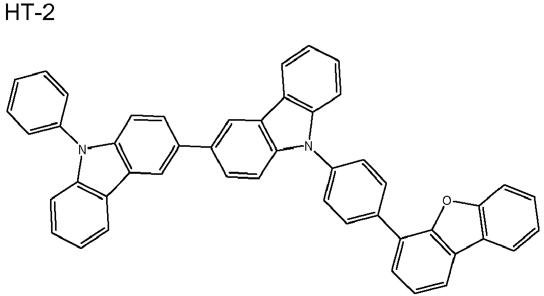

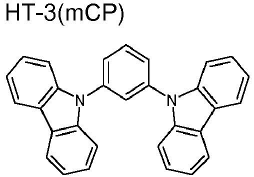

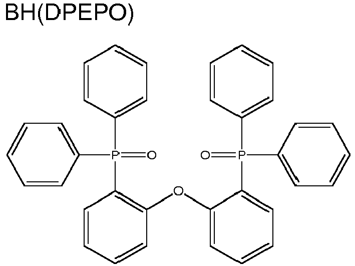

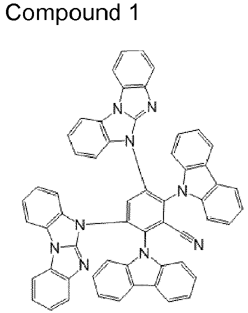

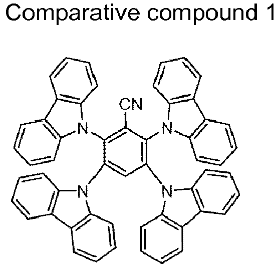

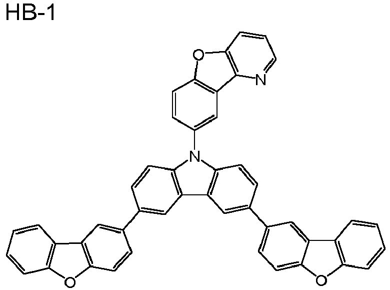

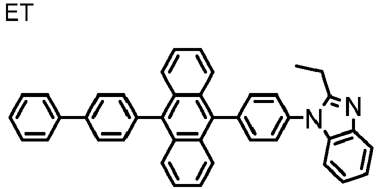

**Table 3**

| Example | x% | V @10mA | EQE @0.1mA | EQE @10mA | CIEx @10mA | CIEy @10mA | λₚ @10mA |
|---|---|---|---|---|---|---|---|
| Comparative compound 1 | 24 | 5.0 | 12.1 | 6.2 | 0.146 | 0.126 | 458 |
| Compound 1 | 24 | 5.2 | 14.4 | 9.1 | 0.155 | 0.174 | 462 |

## Claims

1. A compound of formula (I), 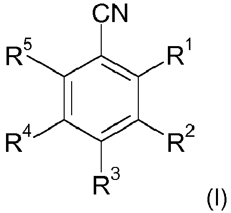 wherein
R¹, R², R³, R⁴ and R⁵ are R^{A}, D¹ or D²;
wherein
0, 1 or 2 of the residues R¹, R², R³, R⁴ and R⁵ are R^{A};
1, 2, 3 or 4 of the residues R¹, R², R³, R⁴ and R⁵ are D²; and
1, 2, 3 or 4 of the residues R¹, R², R³, R⁴ and R⁵ are D¹;
wherein the sum of the residues R^{A}, D² and D¹ is 5;
D² is a donor group except for a group of formula (II);
D¹ is a group of formula (II) 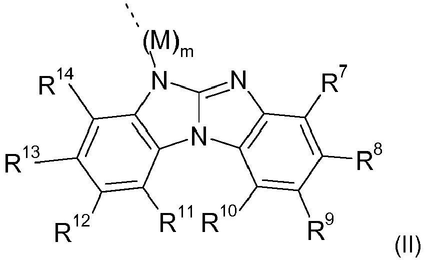 wherein
R⁷ to R¹⁴ are independently of each other H, a C₁-C₂₅alkyl group which is unsubstituted or substituted by at least one group E and/or interrupted by at least one group D, a C₆-C₄₀aryl group which is unsubstituted or substituted by at least one group E, a C₁-C₂₄heteroaryl group which is unsubstituted or substituted by at least one group E, a C₇-C₂₅aralkyl group which is unsubstituted or substituted by at least one group E, a C₅-C₁₂cycloalkyl group which is unsubstituted or substituted by at least one group E, -OR²¹, -SR²¹, -NR¹⁷R¹⁸, -COR²⁰, -COOR¹⁹, -CONR¹⁷R¹⁸, -SiR²²R²³R²⁴, -POR²⁵R²⁷, or halogen, or at least two of the remaining residues R⁷ to R¹⁴ if present at adjacent carbon atoms together form at least one C₆-C₁₈aryl ring or ring system which is unsubstituted or substituted by at least one group E or at least one C₁-C₂₄heteroaryl ring or ring system which is unsubstituted or substituted by at least one group E,
R^{A} is H, a C₁-C₂₅alkyl group which is unsubstituted or substituted by at least one group E and/or interrupted by at least one group D, a C₆-C₄₀aryl group which is unsubstituted or substituted by at least one group E, a C₁-C₂₄heteroaryl group which is unsubstituted or substituted by at least one group E, a C₇-C₂₅aralkyl group which is unsubstituted or substituted by at least one group E, a C₅-C₁₂cycloalkyl group which is unsubstituted or substituted by at least one group E, -OR²¹, -SR²¹, -NR¹⁷R¹⁸, -COR²⁰, -COOR¹⁹, -CONR¹⁷R¹⁸, -SiR²²R²³R²⁴, -POR²⁵R²⁷, or halogen, or
at least two of the residues R^{A} if present at adjacent carbon atoms together form at least one C₆-C₁₈aryl ring or ring system which is unsubstituted or substituted by at least one group E or at least one C₁-C₂₄heteroaryl ring or ring system which is unsubstituted or substituted by at least one group E,
wherein the dotted line is a bonding site to formula (I),
M is a C₆-C₄₀arylene group which is unsubstituted or substituted by at least one group E, a C₁-C₂₄heteroarylene group which is unsubstituted or substituted by at least one group E, a C₁-C₂₅alkylene group which is unsubstituted or substituted by at least one group E and/or interrupted by at least one group D, or a C₂-C₂₅alkenylene group which is unsubstituted or substituted by at least one group E and/or interrupted by at least one group D,
m is 0, 1 or 2,
D is -CO-, -COO-, -S-, -SO-, -SO₂-, -O-, -CR¹⁵=CR¹⁶-, -NR¹⁷-, -SiR²²R²³-, -POR²⁵-, -C≡C-,
E is -OR²¹, -SR²¹, -NR¹⁷R¹⁸, -COR²⁰, -COOR¹⁹, -CONR¹⁷R¹⁸, -CN, -SiR²²R²³R²⁴,-POR²⁵R²⁷, halogen
a C₆-C₄₀aryl group which is unsubstituted or is substituted by at least one group selected from a C₁-C₁₈alkyl group, a C₁-C₁₈alkoxy group, a C₆-C₁₈aryl group, which is in turn unsubstituted or substituted by at least one C₁-C₁₈alkyl group or at least one C₁-C₁₈alkoxy group, a C₅-C₁₂cycloalkyl group, which is in turn unsubstituted or substituted by at least one C₁-C₁₈alkyl group or at least one C₁-C₁₈alkoxy group, a C₁-C₁₈heteroaryl group, which is in turn unsubstituted or substituted by at least one C₁-C₁₈alkyl group or at least one C₁-C₁₈alkoxy group, -F, -CF₃, -CF₂CF₃, -CF₂CF₂CF₃, -CF(CF₃)₂, -(CF₂)₃CF₃ and -C(CF₃)₃,
a C₁-C₁₈ alkyl group, or
a C₁-C₂₄heteroaryl group which is unsubstituted or is substituted by at least one group selected from a C₁-C₁₈alkyl group, a C₁-C₁₈alkoxy group, a C₆-C₁₈aryl group, which is in turn unsubstituted or substituted by at least one C₁-C₁₈alkyl group or at least one C₁-C₁₈alkoxy group, a C₅-C₁₂cycloalkyl group, which is in turn unsubstituted or substituted by at least one C₁-C₁₈alkyl group or at least one C₁-C₁₈alkoxy group, a C₁-C₁₈heteroaryl group, which is in turn unsubstituted or substituted by at least one C₁-C₁₈alkyl group or at least one C₁-C₁₈alkoxy group, -F, -CF₃, -CF₂CF₃, -CF₂CF₂CF₃, -CF(CF₃)₂, -(CF₂)₃CF₃ and -C(CF₃)₃,
R¹⁵ and R¹⁶ are independently of each other H, a C₆-C₁₈aryl group which is unsubstituted or substituted by at least one C₁-C₁₈alkyl group or at least one C₁-C₁₈alkoxy group, a C₁-C₁₈alkyl group or a C₁-C₁₈alkoxy group,
R¹⁷ and R¹⁸ are independently of each other H , a C₆-C₁₈aryl group which is unsubstituted or substituted by at least one C₁-C₁₈alkyl group or at least one C₁-C₁₈alkoxy group, a C₁-C₁₈alkyl group or a C₁-C₁₈alkoxy group, or
R¹⁷ and R¹⁸ together form a five or six membered aliphatic, aromatic or heteroaromatic ring, which is unsubstituted or substituted by at least one group E,
R¹⁹ is H, a C₆-C₁₈aryl group which is unsubstituted or substituted by at least one C₁-C₁₈alkyl group or at least one C₁-C₁₈alkoxy group, a C₁-C₁₈alkyl group or a C₁-C₁₈alkoxy group,
R²⁰ is H or a C₆-C₁₈aryl group which is unsubstituted or substituted by at least one C₁-C₁₈alkyl group or at least one C₁-C₁₈alkoxy group, a C₁-C₁₈alkyl group or a C₁-C₁₈alkoxy group,
R²¹ is H, a C₆-C₁₈aryl group which is unsubstituted or substituted by at least one C₁-C₁₈alkyl group or at least one C₁-C₁₈alkoxy group or a C₁-C₁₈alkyl group,
R²², R²³ and R²⁴ are independently of each other H, a C₁-C₁₈alkyl group or a C₆-C₁₈aryl group which is unsubstituted or substituted by at least one C₁-C₁₈alkyl group, and
R²⁵ and R²⁷ are independently of each other H, a C₁-C₁₈alkyl group, a C₆-C₁₈aryl group which is unsubstituted or substituted by at least one C₁-C₁₈alkyl group, a C₇-C₂₅aralkyl which is unsubstituted or substituted by at least one group E, a C₅-C₁₂cycloalkyl group which is unsubstituted or substituted by at least one group E.

2. The compound according to claim 1, wherein the donor group D² is selected from the groups consisting of (i), (ii) and (iii)
(i) a group of formula (III), 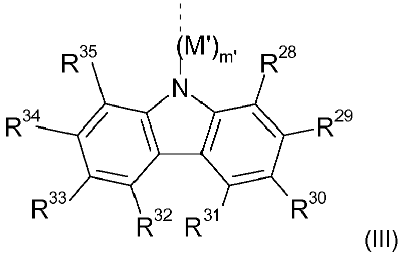 wherein
the dotted line is a bonding site to formula (I),
the residues R²⁸ to R³⁵ and R³⁶ to R³⁹ are independently of each other H, a C₁-C₂₅alkyl group which is unsubstituted or substituted by at least one group E and/or interrupted by at least one group D, a C₆-C₄₀aryl group which is unsubstituted or substituted by at least one group E, a C₁-C₂₄heteroaryl group which is unsubstituted or substituted by at least one group E, a C₇-C₂₅aralkyl group which is unsubstituted or substituted by at least one group E, a C₅-C₁₂cycloalkyl group which is unsubstituted or substituted by at least one group E, -OR²¹, -SR²¹, -NR¹⁷R¹⁸, -COR²⁰, -COOR¹⁹, -CONR¹⁷R¹⁸, -SiR²²R²³R²⁴,-POR²⁵R²⁷, or halogen, or
two adjacent residues R²⁸, R²⁹, R³⁰, R³¹, R³², R³³, R³⁴ and R³⁵ together may form a ring of formula (IV) 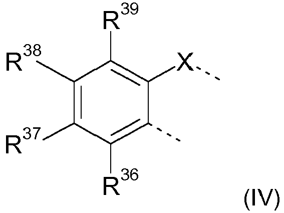 wherein
X is O, S, NR^{17'}, CR^{18'}R^{19'}, SiR^{18'}R^{19'} or PO(OR^{18'}); preferably O, S, NR^{17'} or CR^{18'}R^{19'}, wherein
the dotted lines are bonding sites to the group of formula (III), or
at least two of the residues R³⁶ to R³⁹ if present at adjacent carbon atoms together may form at least one C₆-C₁₈aryl ring or ring system, which is unsubstituted or substituted by at least one group E or at least one C₁-C₂₄heteroaryl ring or ring system, which is unsubstituted or substituted by at least one group E,
R^{17'} is a C₆-C₄₀aryl group which is unsubstituted or substituted by at least one group E or a C₁-C₂₄heteroaryl group which is unsubstituted or substituted by at least one group E,
R^{18'} and R^{19'} are each independently hydrogen, a C₆-C₄₀aryl group which is unsubstituted or substituted by at least one group E or a C₁-C₂₄heteroaryl group which is unsubstituted or substituted by at least one group E,
M' is a C₆-C₄₀arylene group which is unsubstituted or substituted by at least one group E, a C₁-C₂₄heteroarylene group which is unsubstituted or substituted by at least one group E, a C₁-C₂₅alkylene group which is unsubstituted or substituted by at least one group E and/or interrupted by at least one group D, or a C₂-C₂₅alkenylene group which is unsubstituted or substituted by at least one group E and/or interrupted by at least one group D,
m' is 0, 1 or 2, preferably 0 or 1, more preferably 0;
(ii) a group of formula (V),
---(M")_{m"}-NAr₁Ar₂ (V)
wherein
Ar₁ and Ar₂ are each independently a C₆-C₄₀aryl group which is unsubstituted or substituted by at least one group E or a C₁-C₂₄heteroaryl group which is unsubstituted or substituted by at least one group E; or
Ar₁ and Ar₂ may be connected together to form a ring,
M" is a C₆-C₄₀arylene group which is unsubstituted or substituted by at least one group E, a C₁-C₂₄heteroarylene group which is unsubstituted or substituted by at least one group E, a C₁-C₂₅alkylene group which is unsubstituted or substituted by at least one group E and/or interrupted by at least one group D, or a C₂-C₂₅alkenylene group which is unsubstituted or substituted by at least one group E and/or interrupted by at least one group D,
m" is 0, 1 or 2, preferably 0 or 1, more preferably 0,
wherein the dotted line is a bonding site to formula (I);
and
(iii) a group of formula (VI), 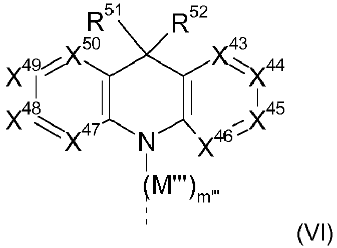 wherein
X⁴³ is CR⁴³ or N;
X⁴⁴ is CR⁴⁴ or N;
X⁴⁵ is CR⁴⁵ or N;
X⁴⁶ is CR⁴⁶ or N;
X⁴⁷ is CR⁴⁷ or N;
X⁴⁸ is CR⁴⁸ or N;
X⁴⁹ is CR⁴⁹ or N;
X⁵⁰ is CR⁵⁰ or N;
wherein
0, 1 or 2, preferably 0 or 1, more preferably 0 of the groups X⁴³, X⁴⁴, X⁴⁵, X⁴⁶, X⁴⁷, X⁴⁸, X⁴⁹ and X⁵⁰ are N;
R⁴³ to R⁵⁰ are independently of each other H, -CN, a C₁-C₂₅alkyl group which is unsubstituted or substituted by at least one group E and/or interrupted by at least one group D, a C₆-C₄₀aryl group which is unsubstituted or substituted by at least one group E, a C₁-C₂₄heteroaryl group which is unsubstituted or substituted by at least one group E, a C₇-C₂₅aralkyl group which is unsubstituted or substituted by at least one group E, a C₅-C₁₂cycloalkyl group which is unsubstituted or substituted by at least one group E, -OR²¹, -SR²¹, -NR¹⁷R¹⁸, -COR²⁰, -COOR¹⁹, -CONR¹⁷R¹⁸, -SiR²²R²³R²⁴, -POR²⁵R²⁷, or halogen, or at least two of the residues R⁴³ to R⁵⁰ if present at adjacent carbon atoms together may form at least one C₆-C₁₈aryl ring, which is unsubstituted or substituted by at least one group E or at least one C₁-C₂₄heteroaryl ring, which is unsubstituted or substituted by at least one group E
M'" is a C₆-C₄₀arylene group which is unsubstituted or substituted by at least one group E, a C₁-C₂₄heteroarylene group which is unsubstituted or substituted by at least one group E, a C₁-C₂₅alkylene group which is unsubstituted or substituted by at least one group E and/or interrupted by at least one group D, or a C₂-C₂₅alkenylene group which is unsubstituted or substituted by at least one group E and/or interrupted by at least one group D,
m'" is 0, 1 or 2, preferably 0 or 1, more preferably 0,
wherein the dotted line is a bonding site to formula (I);
R⁵¹ and R⁵² are each independently hydrogen, a C₁-C₂₅alkyl group which is unsubstituted or substituted by at least one group E and/or interrupted by at least one group D, a C₆-C₄₀aryl group which is unsubstituted or substituted by at least one group E or a C₁-C₂₄heteroaryl group which is unsubstituted or substituted by at least one group E,
or
R⁵¹ and R⁵² may togetherform a ring, which is unsubstituted or substituted by at least one group E.

3. The compound according to claim 2, wherein the donor group D² is a group of formula (III).

4. The compound according to any one of claims 1 to 3, wherein
1, 2, 3 or 4 of the residues R¹, R², R³, R⁴ and R⁵ are D²; and
1, 2, 3 or 4 of the residues R¹, R², R³, R⁴ and R⁵ are D¹;
wherein the sum of the residues D² and D¹ is 3, 4 or 5, preferably 3 or 4, most preferably 4.

5. The compound according to any one of claims 1 to 4, wherein
m in formula (II) is 0, m' in formula (III) is 0, m" in formula (V) is 0 and m"' in formula (VI) is 0.

6. The compound according to any one of claims 1 to 5, wherein R^{A} is H.

7. The compound according to any one of claims 1 to 6, wherein R³ is R^{A}.

8. The compound according to any one of claims 1 to 7, wherein R⁷ to R¹⁴ in formula (II) are H.

9. The compound according to any one of claims 2 to 8, wherein R²⁸ to R³⁵ in formula (III) are independently of each other H, a C₁-C₁₈alkyl group which is unsubstituted, a C₆-C₁₈aryl group which is unsubstituted or -OR²¹,
R²¹ is H, a C₆-C₁₈aryl group which is unsubstituted or substituted by at least one C₁-C₁₈alkyl group or at least one C₁-C₁₈alkoxy group or a C₁-C₁₈alkyl group.

10. The compound according to any one of claims 4 to 9, represented by formula (Ia) 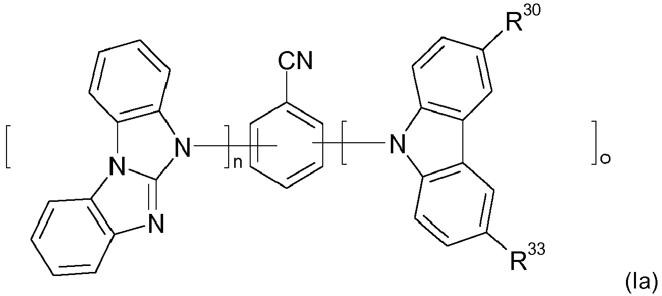 wherein
R³⁰ and R³³ are independently of each other H, a C₁-C₁₈alkyl group which is unsubstituted, a C₆-C₁₈aryl group which is unsubstituted or -OR²¹,
R²¹ is H, a C₆-C₁₈aryl group which is unsubstituted or substituted by at least one C₁-C₁₈alkyl group or at least one C₁-C₁₈alkoxy group or a C₁-C₁₈alkyl group,
n is 1, 2, 3 or 4, and
o is 1, 2, 3 or 4,
wherein the sum of n and o is 3, 4 or 5, preferably 4.

11. A process for the preparation of a compound according to formula (I) as defined in any one of claims 1 to 10, at least comprising the step of reacting an intermediate (IIa) or (IIIa), (Va) or (VIa) 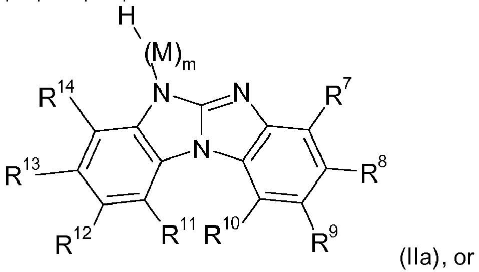 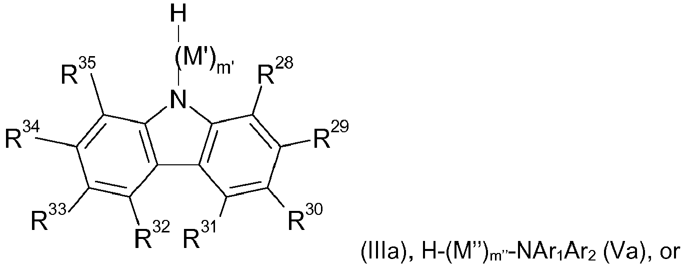 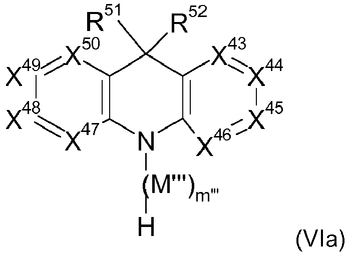 with a compound of formula 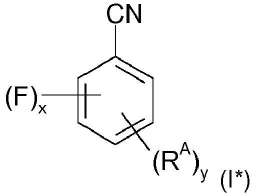 wherein
y is 0, 1 or 2, and
x is 2, 3, 4 or 5,
wherein the sum of the x and y is 5,
in the presence of a base, preferably K₃PO₄, K₂CO₃, Cs₂CO₃, NaOtBu or NaH,
wherein
R⁷ to R¹⁴ are independently of each other H, a C₁-C₂₅alkyl group which is unsubstituted or substituted by at least one group E and/or interrupted by at least one group D, a C₆-C₄₀aryl group which is unsubstituted or substituted by at least one group E, a C₁-C₂₄heteroaryl group which is unsubstituted or substituted by at least one group E, a C₇-C₂₅aralkyl group which is unsubstituted or substituted by at least one group E, a C₅-C₁₂cycloalkyl group which is unsubstituted or substituted by at least one group E, -OR²¹, -SR²¹, -NR¹⁷R¹⁸, -COR²⁰, -COOR¹⁹, -CONR¹⁷R¹⁸, -SiR²²R²³R²⁴, -POR²⁵R²⁷, or halogen, or at least two of the remaining residues R⁷ to R¹⁴ if present at adjacent carbon atoms together form at least one C₆-C₁₈aryl ring or ring system which is unsubstituted or substituted by at least one group E or at least one C₁-C₂₄heteroaryl ring or ring system which is unsubstituted or substituted by at least one group E,
R^{A} is H, a C₁-C₂₅alkyl group which is unsubstituted or substituted by at least one group E and/or interrupted by at least one group D, a C₆-C₄₀aryl group which is unsubstituted or substituted by at least one group E, a C₁-C₂₄heteroaryl group which is unsubstituted or substituted by at least one group E, a C₇-C₂₅aralkyl group which is unsubstituted or substituted by at least one group E, a C₅-C₁₂cycloalkyl group which is unsubstituted or substituted by at least one group E, -OR²¹, -SR²¹, -NR¹⁷R¹⁸, -COR²⁰, -COOR¹⁹, -CONR¹⁷R¹⁸, -SiR²²R²³R²⁴, -POR²⁵R²⁷, or halogen, or
at least two of the residues R^{A} if present at adjacent carbon atoms together form at least one C₆-C₁₈aryl ring or ring system which is unsubstituted or substituted by at least one group E or at least one C₁-C₂₄heteroaryl ring or ring system which is unsubstituted or substituted by at least one group E,
M is a C₆-C₄₀arylene group which is unsubstituted or substituted by at least one group E, a C₁-C₂₄heteroarylene group which is unsubstituted or substituted by at least one group E, a C₁-C₂₅alkylene group which is unsubstituted or substituted by at least one group E and/or interrupted by at least one group D, or a C₂-C₂₅alkenylene group which is unsubstituted or substituted by at least one group E and/or interrupted by at least one group D,
m is 0, 1 or 2, preferably 0 or 1, more preferably 0,
D is -CO-, -COO-, -S-, -SO-, -SO₂-, -O-, -CR¹⁵=CR¹⁶-, -NR¹⁷-, -SiR²²R²³-, -POR²⁵-, -C≡C-,
E is -OR²¹, -SR²¹, -NR¹⁷R¹⁸, -COR²⁰, -COOR¹⁹, -CONR¹⁷R¹⁸, -CN, -SiR²²R²³R²⁴, -POR²⁵R²⁷, halogen, preferably F,
a C₆-C₄₀aryl group which is unsubstituted or is substituted by at least one group selected from a C₁-C₁₈alkyl group, a C₁-C₁₈alkoxy group, a C₆-C₁₈aryl group, which is in turn unsubstituted or substituted by at least one C₁-C₁₈alkyl group or at least one C₁-C₁₈alkoxy group, a C₅-C₁₂cycloalkyl group, which is in turn unsubstituted or substituted by at least one C₁-C₁₈alkyl group or at least one C₁-C₁₈alkoxy group, a C₁-C₁₈heteroaryl group, which is in turn unsubstituted or substituted by at least one C₁-C₁₈alkyl group or at least one C₁-C₁₈alkoxy group, -F, -CF₃, -CF₂CF₃, -CF₂CF₂CF₃, -CF(CF₃)₂, -(CF₂)₃CF₃ and -C(CF₃)₃,
a C₁-C₁₈ alkyl group, or
a C₁-C₂₄heteroaryl group which is unsubstituted or is substituted by at least one group selected from a C₁-C₁₈alkyl group, a C₁-C₁₈alkoxy group, a C₆-C₁₈aryl group, which is in turn unsubstituted or substituted by at least one C₁-C₁₈alkyl group or at least one C₁-C₁₈alkoxy group, a C₅-C₁₂cycloalkyl group, which is in turn unsubstituted or substituted by at least one C₁-C₁₈ alkyl group or at least one C₁-C₁₈alkoxy group, a C₁-C₁₈heteroaryl group, which is in turn unsubstituted or substituted by at least one C₁-C₁₈alkyl group or at least one C₁-C₁₈alkoxy group, -F, -CF₃, -CF₂CF₃, -CF₂CF₂CF₃, -CF(CF₃)₂, -(CF₂)₃CF₃ and -C(CF₃)₃,
R¹⁵ and R¹⁶ are independently of each other H, a C₆-C₁₈aryl group which is unsubstituted or substituted by at least one C₁-C₁₈alkyl group or at least one C₁-C₁₈alkoxy group, a C₁-C₁₈alkyl group or a C₁-C₁₈alkoxy group,
R¹⁷ and R¹⁸ are independently of each other H , a C₆-C₁₈aryl group which is unsubstituted or substituted by at least one C₁-C₁₈alkyl group or at least one C₁-C₁₈alkoxy group, a C₁-C₁₈alkyl group or a C₁-C₁₈alkoxy group, or
R¹⁷ and R¹⁸ together form a five or six membered aliphatic, aromatic or heteroaromatic ring, which is unsubstituted or substituted by at least one group E,
R¹⁹ is H, a C₆-C₁₈aryl group which is unsubstituted or substituted by at least one C₁-C₁₈alkyl group or at least one C₁-C₁₈alkoxy group, a C₁-C₁₈alkyl group or a C₁-C₁₈alkoxy group,
R²⁰ is H or a C₆-C₁₈aryl group which is unsubstituted or substituted by at least one C₁-C₁₈alkyl group or at least one C₁-C₁₈alkoxy group, a C₁-C₁₈ alkyl group or a C₁-C₁₈alkoxy group,
R²¹ is H, a C₆-C₁₈aryl group which is unsubstituted or substituted by at least one C₁-C₁₈alkyl group or at least one C₁-C₁₈alkoxy group or a C₁-C₁₈alkyl group,
R²², R²³ and R²⁴ are independently of each other H, a C₁-C₁₈alkyl group or a C₆-C₁₈aryl group which is unsubstituted or substituted by at least one C₁-C₁₈alkyl group, and
R²⁵ and R²⁷ are independently of each other H, a C₁-C₁₈alkyl group, a C₆-C₁₈aryl group which is unsubstituted or substituted by at least one C₁-C₁₈alkyl group, a C₇-C₂₅aralkyl which is unsubstituted or substituted by at least one group E, a C₅-C₁₂cycloalkyl group which is unsubstituted or substituted by at least one group E,
at least two adjacent residues R²⁸, R²⁹, R³⁰, R³¹, R³², R³³, R³⁴ and R³⁵ together may form a ring of formula (IV) 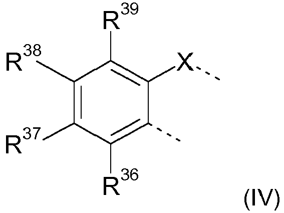 wherein
X is O, S, N_{R}^{17'}, CR^{18'}R^{19'}, SiR^{18'}R^{19'} or PO(OR^{18'}); preferably O, S, NR^{17'} or CR^{18'}R^{19'}, wherein
the dotted lines are bonding sites to the group of formula (IIIa), and
the residues R²⁸ to R³⁵ and R³⁶ to R³⁹ are independently of each other H, a C₁-C₂₅alkyl group which is unsubstituted or substituted by at least one group E and/or interrupted by at least one group D, a C₆-C₄₀aryl group which is unsubstituted or substituted by at least one group E, a C₁-C₂₄heteroaryl group which is unsubstituted or substituted by at least one group E, a C₇-C₂₅aralkyl group which is unsubstituted or substituted by at least one group E, a C₅-C₁₂cycloalkyl group which is unsubstituted or substituted by at least one group E, -OR²¹, -SR²¹, -NR¹⁷R¹⁸, -COR²⁰, -COOR¹⁹, -CONR¹⁷R¹⁸, -SiR²²R²³R²⁴, -POR²⁵R²⁷, or halogen, or
at least two of the remaining residues R³¹ to R³⁸ and R³⁹ to R⁴² if present at adjacent carbon atoms together may form at least one C₆-C₁₈aryl ring or ring system, which is unsubstituted or substituted by at least one group E or at least one C₁-C₂₄heteroaryl ring or ring system, which is unsubstituted or substituted by at least one group E,
R^{17'} is a C₆-C₄₀aryl group which is unsubstituted or substituted by at least one group E or a C₁-C₂₄heteroaryl group which is unsubstituted or substituted by at least one group E,
R^{18'} and R^{19'} are each independently hydrogen, a C₆-C₄₀aryl group which is unsubstituted or substituted by at least one group E or a C₁-C₂₄heteroaryl group which is unsubstituted or substituted by at least one group E,
M' is a C₆-C₄₀arylene group which is unsubstituted or substituted by at least one group E, a C₁-C₂₄heteroarylene group which is unsubstituted or substituted by at least one group E, a C₁-C₂₅alkylene group which is unsubstituted or substituted by at least one group E and/or interrupted by at least one group D, or a C₂-C₂₅alkenylene group which is unsubstituted or substituted by at least one group E and/or interrupted by at least one group D,
m' is 0, 1 or 2, preferably 0 or 1, more preferably 0;
Ar₁ and Ar₂ are each independently a C₆-C₄₀aryl group which is unsubstituted or substituted by at least one group E or a C₁-C₂₄heteroaryl group which is unsubstituted or substituted by at least one group E; or
Ar₁ and Ar₂ may be connected together to form a ring,
M" is a C₆-C₄₀arylene group which is unsubstituted or substituted by at least one group E, a C₁-C₂₄heteroarylene group which is unsubstituted or substituted by at least one group E, a C₁-C₂₅alkylene group which is unsubstituted or substituted by at least one group E and/or interrupted by at least one group D, or a C₂-C₂₅alkenylene group which is unsubstituted or substituted by at least one group E and/or interrupted by at least one group D,
m" is 0, 1 or 2, preferably 0 or 1, more preferably 0,
wherein the dotted line is a bonding site to formula (I);
X⁴³ is CR⁴³ or N;
X⁴⁴ is CR⁴⁴ or N;
X⁴⁵ is CR⁴⁵ or N;
X⁴⁶ is CR⁴⁶ or N;
X⁴⁷ is CR⁴⁷ or N;
X⁴⁸ is CR⁴⁸ or N;
X⁴⁹ is CR⁴⁹ or N;
X⁵⁰ is CR⁵⁰ or N;
wherein
0, 1 or 2, preferably 0 or 1, more preferably 0 of the groups X⁴³, X⁴⁴, X⁴⁵, X⁴⁶, X⁴⁷, X⁴⁸, X⁴⁹ and X⁵⁰ are N;
R⁴³ to R⁵⁰ are independently of each other H, -CN, a C₁-C₂₅alkyl group which is unsubstituted or substituted by at least one group E and/or interrupted by at least one group D, a C₆-C₄₀aryl group which is unsubstituted or substituted by at least one group E, a C₁-C₂₄heteroaryl group which is unsubstituted or substituted by at least one group E, a C₇-C₂₅aralkyl group which is unsubstituted or substituted by at least one group E, a C₅-C₁₂cycloalkyl group which is unsubstituted or substituted by at least one group E, -OR²¹, -SR²¹, -NR¹⁷R¹⁸, -COR²⁰, -COOR¹⁹, -CONR¹⁷R¹⁸, -SiR²²R²³R²⁴, -POR²⁵R²⁷, or halogen, or at least two of the residues R⁴³ to R⁵⁰ if present at adjacent carbon atoms together may form at least one C₆-C₁₈aryl ring, which is unsubstituted or substituted by at least one group E or at least one C₁-C₂₄heteroaryl ring, which is unsubstituted or substituted by at least one group E,
M'" is a C₆-C₄₀arylene group which is unsubstituted or substituted by at least one group E, a C₁-C₂₄heteroarylene group which is unsubstituted or substituted by at least one group E, a C₁-C₂₅alkylene group which is unsubstituted or substituted by at least one group E and/or interrupted by at least one group D, or a C₂-C₂₅alkenylene group which is unsubstituted or substituted by at least one group E and/or interrupted by at least one group D,
m'" is 0, 1 or 2, preferably 0 or 1, more preferably 0,
R⁵¹ and R⁵² are each independently hydrogen, a C₁-C₂₅alkyl group which is unsubstituted or substituted by at least one group E and/or interrupted by at least one group D, a C₆-C₄₀aryl group which is unsubstituted or substituted by at least one group E or a C₁-C₂₄heteroaryl group which is unsubstituted or substituted by at least one group E,
or
R⁵¹ and R⁵² may togetherform a ring, which is unsubstituted or substituted by at least one group E.

12. An electroluminescence device comprising a cathode, an anode, and a plurality of organic layers provided between the cathode and the anode, wherein at least one of the plurality of organic layers is an emitting layer, wherein at least one organic layer comprises at least one compound of formula (I) as defined in anyone of claims 1 to 10.

13. The electroluminescence device according to claim 12, wherein the emitting layer comprises the at least one compound of formula (I) as defined in any one of claims 1 to 10 as a delayed-fluorescent emitter and/or as a host material.

14. The organic electroluminescence device according to claim 12 or 13, further comprising a hole transporting layer between the anode and the emitting layer.

15. The organic electroluminescence device according to any one of claims 12 to 14, further comprising an electron transporting layer between the cathode and the emitting layer.

16. An electronic device comprising the organic electroluminescence device according to any one of claims 12 to 15.

17. An emitting layer comprising at least one compound of formula (I) as defined in any one of claims 1 to 10.

18. Use of the compound of formula (I) as defined in any one of claims 1 to 10 in an electronic device as a delayed-fluorescent emitter and/or as a host material.
